(19) Europäisches Patentamt
European Patent Office
Office européen des brevets

(11) **EP 1 727 833 B1**

(12) **EUROPÄISCHE PATENTSCHRIFT**

(45) Veröffentlichungstag und Bekanntmachung des Hinweises auf die Patenterteilung:
**18.09.2013 Patentblatt 2013/38**

(21) Anmeldenummer: **05716360.2**

(22) Anmeldetag: **24.03.2005**

(51) Int Cl.:
*A61K 45/06* (2006.01)      *C07K 14/705* (2006.01)
*A61K 38/00* (2006.01)      *C07K 14/525* (2006.01)
*C12N 15/62* (2006.01)      *C12N 15/63* (2006.01)
*A61K 38/19* (2006.01)      *C07K 16/40* (2006.01)

(86) Internationale Anmeldenummer:
**PCT/EP2005/003158**

(87) Internationale Veröffentlichungsnummer:
**WO 2005/103077 (03.11.2005 Gazette 2005/44)**

(54) **REKOMBINANTE POLYPEPTIDE DER MITGLIEDER DER TNF LIGANDENFAMILIE UND DEREN VERWENDUNG**

RECOMBINANT POLYPEPTIDES OF THE MEMBERS OF THE TNF LIGAND FAMILY AND USE THEREOF

POLYPEPTIDES RECOMBINES DES MEMBRES DE LA FAMILLE DES LIGANDS TNF ET LEUR UTILISATION

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **26.03.2004 DE 102004014983**

(43) Veröffentlichungstag der Anmeldung:
**06.12.2006 Patentblatt 2006/49**

(73) Patentinhaber: **Universität Stuttgart
70174 Stuttgart (DE)**

(72) Erfinder:
• **PFIZENMAIER, Klaus
75233 Tiefenbronn (DE)**
• **SCHEURICH, Peter
70565 Stuttgart (DE)**
• **GRUNWALD, Ingo
28359 Bremen (DE)**

• **KRIPPNER-HEIDENREICH, Anja
72074 Stuttgart (DE)**

(74) Vertreter: **Graf von Stosch, Andreas et al
Graf von Stosch
Patentanwaltsgesellschaft mbH
Prinzregentenstrasse 22
80538 München (DE)**

(56) Entgegenhaltungen:
WO-A-01/25277      WO-A-01/49866
WO-A-02/00893      WO-A-02/22680
WO-A-02/077018      WO-A-03/042244
WO-A-2004/099244      WO-A2-01/37873
WO-A2-01/43770      WO-A2-99/61085

Bemerkungen:
Die Akte enthält technische Angaben, die nach dem Eingang der Anmeldung eingereicht wurden und die nicht in dieser Patentschrift enthalten sind.

EP 1 727 833 B1

**Beschreibung**

Die vorliegende Erfindung betrifft Verfahren zur extrakorporalen Manipulation, Depletion und/oder Entfernung von in Körperflüssigkeiten enthaltenen Bestandteilen, z.B. mittels Apherese.

Bei den Mitgliedern der TNF- Ligandenfamilie handelt es sich um proinflammatorische Zytokine. Zytokine im allgemeinen und insbesondere die Mitglieder der TNF- Ligandenfamilie spielen eine wichtige Rolle bei der Stimulation und Koordination des angeborenen Immunsystems sowie der humoralen (Antikörper- vermittelten) Immunantwort, der Induktion von Apoptose, dem Aufbau von Knochen, der Ausbildung der Anlagen für Haarwuchs, Zahn- wuchs und Schweißdrüsenentwicklung, der Anlage von Lymphknoten und vielem mehr (Ag- garwal, B.B. (2003), Nat. Rev. Immunol. 3, 745- 756). Eine fehlerhafte Regulierung von Mit- gliedern der TNF- Ligandenfamilie kann dagegen zu zahlreichen pathologischen Zustände führen. Hierzu gehört bspw. der septische Schock, Autoimmunerkrankungen, wie Rheumatoide Arthritis, oder neurodegenerative Erkrankungen. Der Tumor- Nekose- Faktor (TNF) ist das namensgebende und wohl wichtigste Mitglied dieser großen Zytokinfamilie.

Ihre Wirkung entfalten die Mitglieder der TNF-Ligandenfamilie in ihrer biologisch aktiven Form als Homotrimere (Banner, D.W. et aL, (1993) Cell 73, 431-445). Trimere Strukturen und auch Aggregationen höherer Ordnung (z.B. Oligo- oder Multimere von Trimeren) von Proteinen sind in der Natur zahlreich anzutreffen. Beispiele sind das Cartilage Matrix Protein (CMP), ein Bindegewebsprotein (Beck et aL (1996), J Mol Biol 256, 909-923), Proteine aus der Kollagenfamilie, wie die Clq-Familie, zu der Clq, Kollagen α1 (X), α2 (VII), das Überwinterungsprotein, ACRP30, das innere Ohrstruktutprotein, Cellebrin und Multimerin gehören (Kishore und Reid, (1999), Immunopharmacol. 42, 15-21), und Proteine der Collectin-Familie, wie das Lung Surfactant Protein A (SP-A) und das Mannose-Bindungsprotein (MBP) (Epstein et al. (1996), Current Opinion in Immunology, Vol 8 Nr.1, 29-35).

Die Zusammenlagerung von Proteinen zu einem Trimer erfolgt an den Oberflächen dieser in Lösung trimexisierenden Proteine aufgrund von Wechselwirkungen, wie hydrophoben Wechselwirkungen, Wasserstoffbruckenbindungen, kovalenten Bindungen (z.B. Disulfidbrücken), und/oder Coulomb-Kräften, aber auch aufgrund von Strukturmotiven, d.h. chatakteristischen Aminosäuresequenzen, die eine Formation von intermolekularen Supersekundärstrukuten bewirken. Bei den Mitgliedern der TNF-Ligandenfamilie werden die drei Monomere in der homotrimeren Struktur nicht- kovalent über hydrophobe Bindungen zusammengehalten. In ihrer aktivierten Form aktivieren sie wiederum ihnen gegenüberstehende Mitglieder der TNF-Rezeptorfamilie, die als solche keine enzymatische Aktivität besitzen. Beispielsweise bindet TNF als ein Mitglied der TNF-Ligandenfamilie an die zwei Membranrezeptoren TNFR1 und TNFR2 und vermittelt die Trimerisierung bzw. die Aktivierung bereits trimervorliegender, aber Signal-inaktiver Rezeptoren. Durch die Komplexbildung der Rezeptoren wird eine Signalkaskade eingeleitet, mit der u.a. eine Assoziation zytoplasmatischer Adapterproteine einhergeht (Wajant, H. et al (2003), Cell Death, Differ, 10, 45-65). Die trimere Struktur von TNFR1 und TNFR2 stellt sich derart dar, daß die Rezeptoren jeweils im Zwischenraum zwischen zwei der drei TNF-Monomere des TNF-Homotrimers binden (Banner et aL (1993) *supra*). Hieraus wird deutlich, dass sowohl TNF als auch die anderen Mitglieder der TNF Ligandenfamilie nur in seiner/ihrer Struktur als Homotrimer biologisch aktiv ist/sind.

Aufgrund ihrer Funktion können die Mitglieder der TNF-Ligandenfamilie bzw. deren Membranrezeptoren mannigfaltig zur Behandlung zahlreicher Erkrankungen, wie Infektions- und Endzündungskrankheiten, Stoffwechselerkrankungen, Erkrankungen, die in einer fehlerhaften Regulierung der Apoptose begründet sind, neurodegenerative Erkrankungen und vielen weiteren Erkrankungen, eingesetzt werden. Eine besonders wichtige Rolle spielt ihr Einsatz bei der Behandlung von Krebserkrankungen, da es sich bei den Mitgliedern der TNF-Ligandenfamilie generell um antitumoral wirkende Substanzen handelt Insbesondere zu erwähnen sind in diesem Zusammenhang TNF selbst (Eggermont, A.M. and ten Hagen, T.L. (2003), Curr. Oncol. Rep. 5, 79-80), TRAIL (TNF releated apoptoses inducing ligand), auch Apo 2L genannt (Weley et al. (1995), Immunity 6: 673-682; Petti et aL (1996) J Biol Chem 271: 12687-12689) und FasL. *In vivo* Untersuchungen zeigten jedoch starke systemische Nebenwirkungen bei TNF und Agonisten des Fas Rezeptors und in vitro Untersuchungen deuten auch auf ähnliche toxische Wirkungen bei bestimmten TRAIL Präparaten hin (Jo et al. (2000) Nat Med 6: 564-567, Ichikawa et aL (2001) Nat Med 7: 954-960; Ogasawara et al. (1993) Nature 364: 806-809). Beispielsweise zeigten agonistische Antikörper gegen Fas, den Rezeptor von FasL, eine extrem hepatoxische Wirkung (Ogasawara et al. (1993) *supra*). Im Falle von Fas aktivierenden Liganden/Agonisten wurde deshalb eine klinische Anwendung aus Sicherheitsgründen bisher ausgeschlossen. Aufgrund der großen Bedeutung von TNF, TRAIL, FasL und anderer TNF-Ligandenfamilienmitgliedern auf diesem Gebiet und der mit ihrer Applikation in Form einer klinischen systemischen Verabreichung einhergehenden Nebenwirkungen wurden allerdings mehrere Ansätze verfolgt, diese Nebenwirkungen zu minimieren. (Eggermont, A.M. and ten Hagen, T.L. (2003), Curr. OncoL Rep. 5, 79-80).

So beschreibt beispielsweise WO 02/22680 Fusionsproteine, die eine gerichtete und gewebs- bzw. zellspezifische Wirkung von Zytokinen durch die Fusion des Zytokins mit einem Antigen-bindenden Antikörper ermöglichen. Auf diese Weise wird erreicht, dass die Zytokine auf Gewebe bzw. Zellen, die mit diesen Fusionsproteinen nicht in Kontakt kommen, keine Wirkung entfalten und dass Nebenwirkungen auf diese Gewebe oder Zellen verringert werden.

In der DE 102 47 755 wird ein Antikörper-unabhängiges System offenbart, welches ebenfalls eine gerichtete und gewebs- bzw. zellspezifische Wirkung der Zytokine ermöglicht. Es handelt sich hier um Fusionsproteine, bei denen die Aktivierung eines darin enthaltenen Proteinabschnitts mit biologischer Funktion über dessen Bindung an eine ZelloberflächenmolekülBindungsdomäne, die einen weiteren Proteinabschnitt des Fusionsproteins datstellt, erfolgt. Neben einer Verringerung von Nebenwirkungen auf Nicht-Zielgewebe kann dieses System vorteilhafterweise auch für Zelloberflächenmoleküle auf den Zielzellen, für die keine Antikörper bzw. Antikörper mit zu geringer Spezifität vorhanden sind, eingesetzt werden.

Neben den erwähnten Nebenwirkungen ist jedoch ebenfalls die Tatsache äußerst problematisch, daß die aktiven Homotrimere der Mitglieder der TNF-Ligandenfamilie in Verdünnungen, und das heißt auch bei physiologisch sinnvollen Konzentrationen, dissoziieren. Diese Dissoziation ist zwar grundsätzlich reversibel, jedoch verliert das Protein rasch seine Bioaktivität, da es denaturiert. Es wird angenommen, dass diese Denaturierung über die Stufe der instabilen Monomere erfolgt (Smith,R.A. and Baglioni, C. (1987), J. Biol. Chem. 262, 6951-6954; Narhi, LO. and Arakawa, T. (1987), Biochem. Biophys. Res. Commun 147, 740-746).

Aufgrund entsprechender Beobachtungen bei TRAIL, welches eine besondere Labilität aufweist, wurde versucht, eine Stabilität des Proteins durch Anfügen eines Leucin-Zippers als Trimerisierungsmodul zu überkommen (Cha, S.S. et al., (1999), Immunity. 11, 253-261). Im natürlichen Zustand wird TRAIL durch ein Zink-Ion stabilisiert, welches im Zentrum des trimeren Liganden sitzt, und welches durch Cystein-Reste koordiniert wird (Hymowitz, S.G. et al. (2000), Biochemistry 39, 633-640). Nachteilig kann hier jedoch sein, dass durch das Anfügen des Leucin-Zippers nicht nur erhöhte Stabilität erreicht wird, sondern auch andere Eigenschaften, z.B. Struktureigenschaften, wie Strukturveränderungen, Aktivitätsrate oder physiologische Eigenschaften, beeinträchtigt werden könnten.

In WO 01/37873 wird ein Verfahren zur Behandlung von Erkrankungen, die gekennzeichnet sind durch die Produktion von löslichen Cytokinrezeptormolekülen, z.B. löslichen Tumor- Nekrose- Faktor- Rezeptoren (sTNFR) , vorgestellt. Die Erkrankungen umfassen viele Krebstypen und bestimmte weitere Erkrankungen wie HIV- Infektionen. Dabei wird in einer bevorzugten Ausführungsform das Blut des Patienten durch eine Säule geführt, auf der Antikörper immobilisiert sind, die an die sTNFR Moleküle binden und diese entfernen, und anschließend wird dann das Blut zurück in den Patienten überführt. Der Vorgang kann alleine oder in Kombination mit anderen Therapieformen durchgeführt werden.

In WO 01/43770 wird ein Verfahren zur Verstärkung der Immunantwort in einem Säugetier vorgestellt, dass die Beseitigung eines chronischen Krankheitszustands erleichtert. Das Verfahren umfasst die Entfernung von Inhibitoren des immunsystems aus dem Kreislauf des Säugetiers und gestattet dadurch eine stärkere Immunantwort auf das Pathogen. Die Entfernung der Inhibitoren des Immunsystem wird erreicht, indem ein zellfreier Bestandteil des Bluts, oder eine Fraktion davon mit einem oder mehreren Bindungspartnern in Kontakt gebracht wird, die in der Lage sind, an die Inhibitoren zu binden und diese dadurch aus den biologischen Flüssigkeiten zu entfernen. Besonders nützlich bei der Ausführung des Verfahrens ist eine absorbierende Matrix, die sich aus einem inerten, biokompatiblen Substanz zusammensetzt, die kovalent mit einem Bindungspartner verbunden ist, z.B. einem Antikörper, der in der Lage ist, spezifisch an die gewünschten Inhibitoren zu binden.

In der internationalen Veröffentlichung WO 99/61085 wird ein Verfahren zur Behandlung von Krebs unter Verwendung von Ultra-Apherese offenbart, das verbessert wurde, um Verbindungen von weniger als 120 000 Dalton Molekulargewicht zu entfernen. Daran schließt sich die Verabreichung von Ersatzflüssigkeit an, um das Immunsystem des Patienten zu stimulieren, die festen Tumore zu attackieren. In einer bevorzugten Ausführungsform wird Ultra-Apherese am Patienten vorgenommen unter Verwendung eines Kapillarröhrchenultrafilters mit einer Porengröße von 0,02 - 0,05 mm und mit einem Molekulargewichtsausschluss von 120000 Dalton. Die bevorzugte Austauschflüssigkeit ist mit Ultra-Apherese behandeltes Plasma.

Es besteht daher ein Bedarf, die Stabilität aktiver Zytokine, insbesondere Mitglieder der TNF-Ligandenfamilie, zu erhöhen, ohne dass deren natürliche Eigenschaften durch eine Änderung ihrer Struktur nachteilig beeinflusst werden, und ohne daß sie starke zytotoxische oder andere Nebenwirkungen bei therapeutischen Anwendungen zeigen.

Der vorliegenden Erfindung liegt die Erkenntnis zugrunde, daß natürlich vorkommende lösliche Zytokinmitglieder der TNF-Ligandenfamilie ihre volle Bioaktivität lediglich als Homotrimere entfalten, aber auf der anderen Seite dazu neigen, über Dissoziation in ihre Mo-nomere zu denaturieren.

Es gilt daher diese Dissoziation der Homotrimere in Monomere zu unterbinden. Dies wurde erreicht, indem mindestens drei Monomere eines Mitglieds der TNF-Ligandenfamilie, insbesondere TNF, über ihre C- und N-Termini mittels kurzer Peptid-Linker kovalent miteinander verbunden wurden zu einem "single chain" -Molekül (nachfolgend "sc"), insbesondere zu einem scTNF. Demnach besteht das gesamte Molekül (mindestens drei Monomere eines Mitglieds der TNF-Ligandenfamilie mit den zwei Peptid-Linkern) aus einem einzigen Proteinstrang, so dass eine Dissoziation in die Monomere nicht mehr erfolgen kann. Es wurde festgestellt, dass derartige Moleküle im Vergleich zu ihren entsprechenden löslichen Wildtyp-Mitgliedern der TNF-Ligandenfamilie einen sehr geringen Verlust in ihrer Bioaktivität ausweisen. Es wurde im Gegenteil nicht nur nachgewiesen, dass die Polypeptide dieselben (qualitativen) Aktivitäten aufweisen wie ihr entsprechendes lösliches Wildtyp Mitglied der TNF-Ligandenfamilie, sondern auch, dass sie aufgrund ihrer erheblich höheren Stabilität auch zu einem Zeitpunkt noch Bioaktivitäten aufweisen, zu dem das lösliche Wildtyp Mitglied der TNF-Ligandenfamilie seine Aktivität bereits verloren hat, d.h. dissoziiert bzw. denaturiert ist (hierzu wird auf die nachfolgend beschriebenen verschiedene Stabilitätstests verwiesen, welche in den Beispielen sowie in den Figuren beschrieben werden).

Unter einem "löslichen Wildtyp-Mitglied der TNF-Ligandenfamilie" ist ein löslicher extrazellulärer Abschnitt eines membranständigen Mitglieds der TNF-Ligandenfamilie zu verstehen. Nachfolgend werden synonym zu dem Begriff "löslicher/n/s Wildtyp" die Ausdrücke "Wirdtyp", "wt", "löslich" und "s" (für "soluble") verwendet. Insbesondere kann es sich um lösliches Wildtyp TNF (als ein Mitglied der TNF-Ligandenfamilie) handeln, für das entsprechend nachfolgend die synonymen Begriffe "Wildtyp TNF", "wtTNF", lösliches TNF und "sTNF" verwendet werden.

Eine Komponente A im Sinne der Erfindung ist ein TNF Monomer oder ein funktionelles Fragment oder eine funktionelle Variante hiervon. Unter einem "Monomer" ist die kleinste Protein- oder Polypeptideinheit zu verstehen, die von einem oligomeren Protein ohne Trennung kovalenter Bindungen separiert werden kann.

Ein Polypeptid oder eine Komponente A oder ein Fragment oder eine Variante hiervon sind im Sinne der Erfindung funktionell, sofern es/sie seine/ihre biologische Aktivität bzw. Funktion aufweist, insbesondere seine/ihre Bindungseigenschaft an einen Interaktionspartner, z.B. einen membranständigen Rezeptor, und auch seine/ihre Trimerisierungseigenschaft. Bei den funktionellen Fragmenten und den funktionellen Varianten der Erfindung können diese biologischen Funktionen zwar verändert sein, z.B. hinsichtlich ihrer Spezifität oder Selektivität, die grundsätzliche biologische Funktion wird jedoch beibehalten.

Im Stand der Technik sind zahlreiche Verfahren zur Messung der biologischen Aktivität eines Proteins, Polypeptids bzw. Moleküls bekannt, bspw. Protein-Assays, die markierte Substrate verwenden, Substratanalysen durch chromatographische Verfahren, wie HPLC oder Dünnschicht-Chromatographie, spektrophotometrische Verfahren etc. (siehe z.B. Maniatis et aL (2001) Molecular Cloning: A Laboratory Manual, Cold Spring Harbour Laboratory Press, Cold Spring Harbour, NY).

Unter einem Fragment im Sinne der Erfindung ist sowohl ein Fragment eines Monomers eines Mitglieds der TNF-Ligandenfamilie als auch ein Fragment eines Polypeptids bzw. Proteins der vorliegenden Erfindung zu verstehen. Es kann sich hierbei um N-terminal, C-terminal oder intrasequentiell verkürzte Aminosäuresequenzen des Monomers, Polypeptids bzw. Proteins handeln. Insbesondere bei intrasequentiellen Verkürzungen des Polypeptids bzw. Proteins kann es sich um Verkürzungen der Sequenz eines oder mehrerer der drei Monomere handeln, die wiederum N-terminal, C-terminal oder intrasequentiell auftreten können.

In einer besonders bevorzugten Ausführungsform der Erfindung stellt das Fragment eines Monomers dessen extrazelluläre Domäne dar, die der gesamten extrazellulären Domäne des löslichen Wildtyp Mitglieds der TNF-Ligandenfamilie oder einem Abschnitt hiervon entspricht. Insbesondere stellt das Fragment eines Monomers dessen extrazelluläre Domäne dar, die entweder dem löslichen Wildtyp TNF (Aminosäuren 77-233) oder der gesamten extrazellulären Domäne (Aminosäuren 53 - 233) entspricht.

Die Herstellung solcher Fragmente ist im Stand der Technik gut bekannt und kann von einem Fachmann unter Anwendung von Standardverfahren durchgeführt werden (siehe z.B. Maniatis et aL (2001), Molecular cloning: Laboratory Manual, Cold Spring Harbour Laboratory Press). Im allgemeinen kann die Herstellung von Fragmenten der Monomere, Polypeptide bzw. Proteine durch Modifizieren der DNA-Sequenz, die das native Monomer, Polypeptid bzw. Protein codiert, gefolgt von einer Transformation dieser DNA-Sequenz in einen geeigneten Wirt und Expression dieser modifizierten DNA-Sequenz, unter der Voraussetzung, daß die Modifikation der DNA die funktionellen Aktivitäten des Monomere, Polypeptids bzw. Proteins nicht zerstört durchgeführt werden.

Die Identifizierung eines Fragments kann entweder über die Überprüfung seiner Funktionalität durch Messung seiner biologischen Aktivität erfolgen, wie oben beschrieben, oder auch anhand einer Sequenzierung des Fragments und einem nachfolgenden Vergleich der erhaltenen Sequenz mit der nativen Sequenz. Die Sequenzierung kann anhand von Standardverfahren, die im Stand der Technik zahlreich und gut bekannt sind, erfolgen.

Als Varianten von biologisch aktiven Monomeren, Polypeptiden bzw. Proteinen oder Fragmenten hiervon oder einer Komponente A werden insbesondere solche Monomere, Polypeptide bzw. Proteine oder Fragmente hiervon bezeichnet, welche Sequenzunterschiede zu den entsprechenden nativen Sequenzen aufweisen. Bei diesen Sequenzabweichungen kann es sich um eine oder mehrere Insertion (en) , Deletion (en) und/ oder Substitution (en) von Aminosäuren handeln, wobei eine Sequenzhomologie von mindestens 60%, bevorzugt 70%, stärker bevorzugt 80%, ebenfalls stärker bevorzugt 85%, noch stärker bevorzugt 90% und am meisten bevorzugt 97% vorliegt.

Um die prozentuale Identität zweier Nukleinsäure- oder Aminosäuresequenzen zu bestimmen, können die Sequenzen abgeglichen werden, um nachfolgend miteinander verglichen zu werden. Hierfür können z.B. Lücken in die Sequenz der ersten Aminosäure- bzw. Nukleinsäuresequenz eingeführt werden und die Aminosäuren bzw. Nukleotide an der entsprechenden Position der zweiten Aminosäure- bzw. Nukleinsäuresequenz verglichen werden. Wenn eine Position in der ersten Aminosäuresequenz mit der gleichen Aminosäure bzw. dem gleichen Nukleotid besetzt ist, wie es an einer Position in der zweiten Sequenz der Fall ist, dann sind beide Sequenzen an dieser Position identisch. Die prozentuale Identität zwischen zwei Sequenzen ist eine Funktion der Anzahl identischer Positionen geteilt durch die Sequenzen.

Die Bestimmung der prozentualen Identität zweier Sequenzen kann anhand eines mathematischen Algorithmus durchgeführt werden. Ein bevorzugtes, jedoch nicht beschränkendes, Beispiel eines mathematischen Algorithmus, der für den Vergleich zweier Sequenzen herangezogen werden kaan, ist der Algorithmus von Karlin et al. (1993), PNAS USA, 90:5873-5877. Ein solcher Algorithmus ist in dem NBLAST-Programm integriert, mit dem Sequenzen identifiziert werden können, die eine gewünschte Identität zu den Sequenzen der vorliegenden Erfindung besitzen. Um einen Lücken-Abgleich (auch "gapped alignment"), wie oben beschrieben, zu erhalten, kann das "Gapped BLAST"-Programm verwendet werden, wie in Altschul et al. (1997), Nucleic Acids Res, 25:3389-3402 beschrieben.

Biologisch aktive, also funktionelle, Varianten von Monomeren, Polypeptiden bzw. Proteinen oder Fragmenten hiervon im Sinne der Erfindung, können vorzugsweise selektive Rezeptorbindungseigenschaften aufweisen, wobei die Variante z.B. hinsichtlich ihrer spezifischen Bioaktivität oder anderer Eigenschaften, insbesondere ihrer Stabilität, optimiert sein kann.

Unter den Begriff Varianten fallen insbesondere solche Aminosäuresequenzen, die gegenüber den physiologischen Sequenzen konservative Substitution aufweisen. Als konservative Substitutionen werden solche Substitutionen bezeichnet, bei denen Aminosäuren gegeneinander ausgetauscht werden, die aus der gleichen Klasse stammen. Insbesondere gibt es Aminosäuren mit aliphatischen Seitenketten, positiv oder negativ geladenen Seitenketten, aromatischen Gruppen in der Seitenketten oder Aminosäuren, deren Seitenketten Wasserstoffbrücken eingehen können, bspw. Seitenketten, die eine Hydroxyfunktion besitzen. Das bedeutet, daß bspw. eine Aminosäure mit einer polaren Seitenkette durch eine andere Aminosäure mit einer gleichfalls polaren Seitenkette ersetzt wird oder beispielsweise eine durch eine hydrophobe Seitenkette gekennzeichnete Aminosäure durch eine andere Aminosäure mit gleichfalls hydrophober Seitenkette substituiert wird (z.B. Serin (Threonin) durch Threonin (Serin) bzw. Leucin (Isoleucin) durch Isoleucin (Leucin) ) . Insertionen und Substitutionen sind insbesondere an solchen Sequenzpositionen möglich, die keine Veränderung der dreidimensionalen Struktur hervorrufen oder den Bindungsbereich betreffen. Eine Veränderung einer dreidimensionalen Struktur durch Insertion (en) oder Deletion (en) ist bspw. mit Hilfe von CD- Spektren (Zirkulardichroismus- Spektren) leicht überprüfbar (Urry, 1985, Absorption, circular Dichroism and ORD of Polypeptides, in: Modem Physical Methods in Biochemistry, Neuberger et aL (Hrgb.) , Elsevier, Amsterdam) .

Geeignete Verfahren zur Herstellung von Varianten, z.B. von Monomeren, Polypeptiden bzw. Proteinen oder Fragmenten hiervon mit Aminosäuresequenzen, die gegenüber der (den) nativen Sequenzen Substitutionen aufweisen, werden bspw. in den Druckschriften US 4,737,462, US 4,588,585, US 4,959,314, US 5,116,943, US 4,879,111 und US 5,017,691 offenbart Die Herstellung von Varianten im allgemeinen wird insbesondere auch von Maniatis et al, (2001), Molecular Cloning: A Laboratory Manual, Cold Spring Harbor Laboratory Press) beschrieben. Es können hierbei Codons weggelassen, ergänzt oder ausgetauscht werden. Varianten können insbesondere auch solche Proteine oder Polypeptide sein, die stabilisiert sind, um der physiologischen Degradation zu entgehen, bspw. durch Stabilisierung des Proteinrückgrats durch Substitution der amidartigen Bindung, bspw. auch durch den Einsatz von ß-Aminosäuren.

Varianten können ebenfalls hergestellt werden, indem in die Nukleinsäuren, welche für die Varianten codieren, Veränderungen eingeführt werden, wie bspw. Insertionen, Deletionen und/ oder Substitutionen einer oder mehrerer Nukleotide. Im Stand der Technik sind zahlreiche Verfahren für derartige Veränderungen von Nukleinsäuresequenzen bekannt. Eine der meist verwendeten Technik ist die Oligonukleotid- gerichtete Ortsspezifische Mutagenese (siehe Comack B., Current Protocols in Molecular Biology, 8.01- 8.5.9, Ausubel F. et aL, Aufl. 1991) . Bei dieser Technik wird ein Oligonukleotid synthetisiert, dessen Sequenz eine bestimmte Mutation aufweist. Dieses Oligonukleotid wird dann mit einem Template hybridisiert, das die Wildtyp- Nukleinsäuresequenz enthält. Bevorzugt wird bei dieser Technik ein einzelsträngiges Template verwendet. Nach dem Annealing von Oligonukleotid und Template, wird eine DNA- abhängige DNA- Polymerase eingesetzt, um den zweiten Strang des Oligonukleotids, der komplementär zu dem Template- DNA- Strang ist, zu synthetisieren. Als Ergebnis wird ein Heteroduplex- Molekül erhalten, welches eine Fehlpaarung enthält, die durch die oben erwähnte Mutation in dem Oligonukleotid entsteht. Die Oligonukleotidsequenz wird in ein geeignetes Plasmid eingeführt, dieses wird in eine Wirtszelle eingeführt und in dieser Wirtszelle wird die Oligonukleotid- DNA repliziert. Mit dieser Technik erhält man Nukleinsäuresequenzen mit gezielten Veränderungen (Mutationen) , welche für die Herstellung von Varianten gemäß der Erfindung verwendet werden können.

Die von dem Polypeptid umfassten Komponenten A können identisch oder verschieden sein. Beispielsweise kann ein Polypeptid 4, 5, 6 oder mehr Komponenten A enthalten, die ein Tetramer, Pentamer, Hexamer, etc. bilden. Ebenfalls besonders bevorzugt enthält ein Polypeptid ein ganzzahliges Vielfaches eines wie zuvor beschriebenen Trimers der Komponenten A, z.B. zwei, drei, vier, oder mehr hintereinander angeordnete Trimere. Die in Polypeptiden enthaltenen Komponenten A können durch Linker voneinander getrennt sein (siehe unten). Sind dabei, wie oben beschrieben, zwei, drei, vier, oder mehr Trimere der Komponenten A hintereinander angeordnet, so können die Linker, die die verschiedenen Trimere miteinander verbinden, gegebenenfalls länger sein, als die Linker, die die Komponenten A in einem einzelnen Trimer miteinander verbinden.

Die Komponenten A können aus dem gleichen oder verschiedenen Organismen stammen. Es kann sich hierbei um Vertebraten, insbesondere um Säugetiere, beispielsweise der Maus, der Ratte, dem Schwein und vor allem dem Menschen, handeln.

Gemäß der Erfindung handelt es sich bei den Komponenten A des Polypeptids jeweils um ein Monomer oder ein funktionelles Fragment oder eine funktionelle Variante von TNF (Tumor Nekrose Faktor; Genbank Zugriffsnt. NM_ 000594).

In einer besondets bevorzugten Ausführungsform handelt es sich bei der wie oben definierten Komponente A des Polypeptids um ein TNF Monomer das resistent gegen das Prozessierungsenzym TACE ist. TACE ist ein Mitglied der ADAM Protease Familie und stellt das physiologische TNF-spezifische Prozessierungsenzym des natürlicherweise in der Zellmembran befindlichen TNF dar. TNF wird üblicherweise von der Zellmembran abgespalten und in die Umgebung freigesetzt. Einer erfindungsgemäßen, gegen TACE resistenten, Komponente A, fehlt bevorzugt die Spaltstelle Ala-Val (z.B. AS 76-77 bei scTNF gemäß SWISSPROT Nomenklatur für humanes TNF, No. PO1375). Die Spaltstelle kann dabei erfindungsgemäß durch Deletion einer oder beider relevanten Aminosäuren Ala oder Val entfernt werden. Alternativ oder zusätzlich kann erfindungsgemäß die Erkennungssequenz für TACE (z.B. AS 77-88 bei scTNF gemäß SWISSPROT Nomenklatur für humanes TNF, No. PO1375) ganz oder teilweise deletiert werden. Dies erfolgt bevorzugt durch Deletion von zwei, drei, vier oder mehr Aminosäuren, bspw. aller Aminosäuren der TACE Erkennungssequenz. Für scTNF liegt beispielsweise die TACE resistente Sequenz im Bereich der Aminosäuren 89-233 (AS 89-233) des scTNF gemäß SWISSPROT Nomenklatur für humanes TNF, No. PO1375. Eine solche Deletion in Komponente A verhindert eine potentielle Spaltung der Komponente A, z.B. scTNF, durch TACE nahe der Verknüpfungsstellen der Komponenten A im Bereich der Linker (Komponente B, siehe unten), und erhöht damit die *in vivo* Stabilität der Polypeptide. Gegebenenfalls kann die durch die Deletion entstehende Verkürzung der Sequenz durch eine entsprechende Verlägerung der Linker kompensiert werden. Die beispielhaft für scTNF beschriebene Deletion der Sequenz der Komponente A kann auf jede der oben genannten Komponenten A angewendet werden. Sollte die TACE-Sequenz nicht oder nur teilweise entfernt werden, so wird bevorzugt darauf geachtet, daß bei Verwendung zusätzlicher Komponenten B oder C (siehe unten) im erfindungsgemäßen Polypeptid die TACE-Erkennungssequenz und die TACE Spaltstelle nicht wiederhergestellt werden.

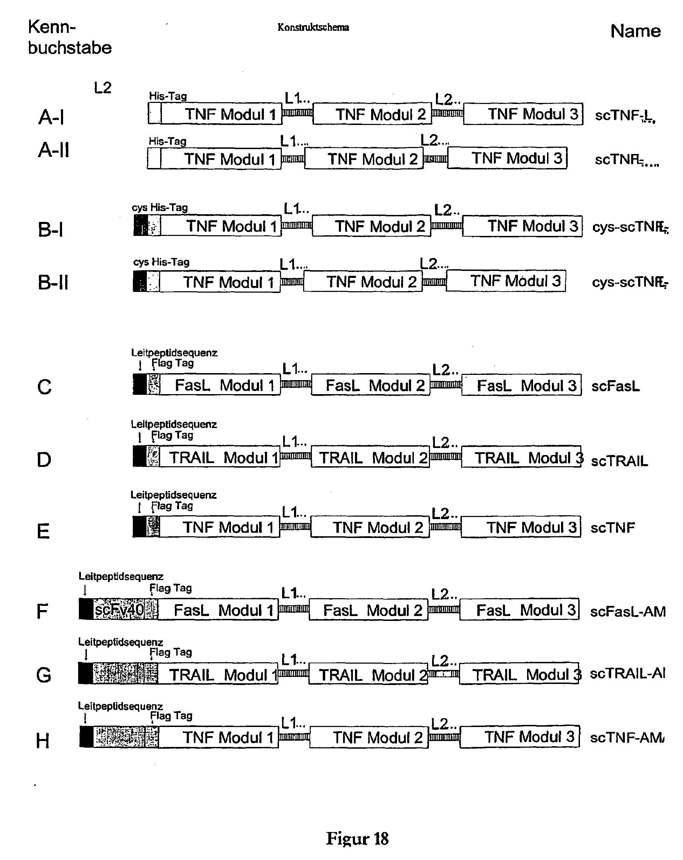

In einer weiteren besonders bevorzugten Ausführungsform sind die wie oben definierten Komponenten A des Polypeptids der Erfindung so modifiziert, daß die erfindungsgemäßen Polypeptide kovalent an Oberflächen koppeln können. Diese Kopplung erfolgt bevorzugt an planaren Oberflächen oder an sphärischen Partikeln, wie z.B. an magnetische Partikel (Magnetbeads) , oder an Nanopartikel/ Mikropartikel, vorzugsweise als Zell- mimetisches, therapeutisches Reagenz mit dem membrangebundenem TNF ähnlichen Eigenschaften. Zur Kopplung wird eine Kopplungsgruppe, bevorzugt die SH- Gruppe eines Cysteinrestes, in den N- terminalen Bereich mindestens einer Komponente A eines Polypeptids eingeführt. (Weitere erfindungsgemäß bevorzugte Kopplungsgruppen werden weiter unten beschrieben) . Dies kann besonders bevorzugt durch Einführen eines Cysteinrestes in Form einer Addition und/ oder Substitution an der gewünschten Position erfolgen. Die Kopplungsgruppe kann sich an jeder Position des N- terminalen Bereichs der Komponente A befinden, bevorzugt nahe am N- Terminus. Besonders bevorzugt befindet sich die Kopplungsgruppe in einem Bereich der ersten 1- 15, und noch stärker bevorzugt in einem Bereich der ersten 1- 10 N- terminalen Aminosäuren. Beispielsweise kann die Modifikation bei einem prokaryotisch exprimierten scTNF nach einem initialen Methionin (Position 2) der Aminosäuresequenz oder bei einem eukaryotisch exprimierten scTNF direkt nach der abgespaltenen Leader-Sequenz eingeführt werden, die also die N- terminalen Aminosäuren der Komponente A darstellen. Alternativ kann bei einem erfindungsgemäß verwendeten Tag, bspw. einem HisTag, einem Flag- Tag (siehe z.B. Figur 18) erfindungsgemäß eine Kopplungsgruppe eingeführt werden, z.B. resultierend in einem CysHis- Tag mit einem Cystein an Aminosäureposition 9. Eine weitere Alternative beinhaltet das Einführen einer Kopplungsgruppe in eine bzw. beide Komponenten B (Linker) des Polypeptids.

Bevorzugt wird in einem Polypeptid nur die am N-Terminus gelegene Komponente A bzw. der weiter N-terminal gelegene Tag mit einer wie oben beschriebenen Kopplungsgruppe versehen/modifiziert. In einer weiteren Ausführungsform werden im Falle eines Polypeptids, welches 3 Komponenten A enthält, beispielsweise 2 oder 3 der Komponenten A mit jeweils einer Kopplungsgruppe modifiziert, sodass das Gesamtmolekül (das erfindungsgemäße Polypeptid) über zwei bzw. 3 kovalente Bindungen an eine dafür geeignete Matrix gekoppelt werden kann. Die Kopplung des Polypeptids über eine oder mehrere Kopplungsgruppen aller Komponenten A eines Polypeptids beeinträchtigt bevorzugt nicht die Funktion der einzelnen Komponenten A von TNF, sondern ermöglicht vielmehr eine Verbesserung der Immobilisation von scTNF, an Oberflächen und/oder Partikel. Wie zuvor ausgeführt, enthält jedes der erfindungsgemäßen Polypeptide bevorzugt die für die biologische Funktion notwendigen Untereinheiten (Komponenten A) in einer funktionell relevanten Anordnung. Durch die Kopplung eines Polypeptids an eine Oberfläche/einen Partikel ist es erfindungsgemäß möglich Oberflächen bzw. Partikel mit z.B. gekoppelten TNF-Homo- oder Heterotrimeren bzw. -multimeren herzustellen. Auf diesen Oberflächen/Partikeln sind die gekoppelten TNF-Homo- oder Heterotrimere bzw. -multimere stabil und bioaktiv immobilisiert sind. Durch das single-chain Prinzip ist der funktionell relevante trimere Zustand der Komponenten A durch die Komponenten B stabilisiert, wodurch sichergestellt ist, dass nach kovalenter Kopplung an eine Oberfläche/den Partikel eine Dissoziation einzelner Komponenten A ausgeschlossen und damit Verlust der biologischen Aktivität verhindert wird.

Eine bevorzugte Eigenschaft solcher an Oberflächen oder Partikel gekoppelten Polypeptide ist ihre biomimetische Aktivität, die der natürlicher, membranständiger Liganden der TNF Familie entspricht. Beispielsweise erlangt ein immobilisiertes scTNF eine hohe Affinität für TNFR2 und damit hohe Signalfähigkeit.

Polypeptide, die wie oben beschriebene, mit einer Kopplungsgtuppe modifizierte, Komponenten A enthalten und an eine Oberfläche und/ oder Partikel gekoppelt werden/ sind, können erfindungsgemäß zur Detektion wie auch zur Manipulation, Depletion und/ oder Entfernung von Bindungspartnern von TNF auch damit assoziierten Verbindungen eingesetzt werden. Von vorrangigem Interesse sind dabei Verfahren zur extrakorporalen Manipulation, Depletion und/ oder Entfernung von in Körperflussigkeiten enthaltenen Bestandteilen, z.B. mittels Apherese.

Die nachfolgenden Ausführungen zu den Gegenständen der Erfindung beziehen sich auf das TNF-Ligandenfamilienmitglied TNF.

Die Polypeptide der vorliegenden Erfindung umfassen neben den beschriebenen Komponenten A mindestens zwei Komponenten B, wobei die Komponente B die Eigenschaft eines Peptid-Linkers aufweist. Als Peptid-Linker im Sinne der Erfindung ist jede in einem biologischen System exprimierbare Peptidsequenz denkbar. Peptidlinker stellen sich bei den Polypeptidkonstrukten bspw. als eine flexible Verbindung dar, die vorzugsweise jedoch die intrinsischen Trimerisierungseigenschaften von TNF nicht negativ beeinflusst. Vorzugsweise werden Linker mit solchen Eigenschaften, insbesondere Flexibilität und Lange, gewählt, welche die spontan gebildeten Homotrimeren des jeweiligen Liganden (derivats) zu stabilisieren vermögen.

In einer bevorzugten Ausführungsform der Erfindung handelt es sich bei der Komponente B demnach um Peptidlinker bestehend aus 2 bis 30, bevorzugt zwischen 4 und 16 und besonders bevorzugt zwischen 4 und 12 Aminosäuren, welche bevorzugt repetitive Glycin- Serin- Strukturen, ganz bevorzugt Gly- Gly- Gly- Ser- Module (GGGS-Module) in repetitiver Anordnung enthalten.

Bevorzugt handelt es sich bei den Peptid-Linkern der Erfindung also um Glycin (G) reiche Peptid-Linker, d.h. dass die Aminosäuresequenz eines Peptid-Linkers einen hohen Glycin-Anteil, vorzugsweise von 60 bis 80%, besonders bevorzugt von 75%, aufweist.

In besonders bevorzugten Ausführungsformen der Erfindung umfasst der Peptid-Linker (Komponente B) die Aminosäuresequenz GGGSGGGSGGGS, auch (GGGS), oder $L_{short}$ genannt, oder die Aminosäuresequenz GGGS-GGGSGGGSGGS, auch $(GGGS)_4$ oder $L_{long}$ genannt. Die Peptid-Linker werden erfindungsgemäß u.a. als L1 und L2 bezeichnet, so dass sich auch Bezeichnungen wie $L1_{short}$, $L2_{short}$, $L1_{long}$ und/oder $L2_{long}$ ergeben. Eine Verwendung zweier unterschiedlicher Linker zur Stabilisierung eines trimeren Moleküls ist möglich.

Bevorzugt verknüpfen die Peptid-Linker gemäß der Erfindung, also die Komponenten B, jeweils zwei der mindestens drei Komponenten A miteinander. Diese Verknüpfung erfolgt kovalent über den C-Terminus einer Komponente A und dem N-Terminus einer weiteren Komponente A. Das Polypeptid stellt ein Einzelketten-Molekül (auch single chain- oder sc-Molekül genannt) dar. Das bedeutet, daß sämtliche Komponenten A und Komponenten B, die das Polypeptid umfasst, auf einem einzigen Polypeptid- bzw. Proteinstrang liegen.

In einer bevorzugten Ausführungsform der Erfindung bilden die Komponenten A und die Komponenten B eine trimere Proteinstruktur aus. Bevorzugt handelt es sich hierbei um eine homotrimere Proteinstruktur, jedoch sind auch heterotrimere Proteinstrukturen von der Erfindung umfasst.

Die Ausbildung dieser trimeren Proteinstruktur wird bevorzugt durch die Komponente B bewirkt und/oder durch sie verstärkt Da die zur Trimerisierung führende Komponente B bzw. die eine Trimerisierung verstärkende Komponente B des Polypeptids im wesentlichen keine höheren Aggregate bilden sollte, sollte die Komponente B typischerweise keinen Cysteinrest aufweisen, der eine intermolekulare Disulfidbrücke ausbilden kann. Vorzugsweise wird die Komponente B in einem Polypeptid daher keinen Cysteinrest oder nur solche Cysteinreste, die eine intramolekulare Disulfidbrücke, also im Polypeptid selbst, besitzen, um zu vermeiden, daß unter oxidierenden Bedingungen eine kovalente Verknüpfung mit dem mindestens einen Cysteinrest eines Fusionsproteins eines anderen Trimers auftreten kann.

Die Sequenzen von nativen Polypeptiden oder Fragmenten dieser nativen Polypeptide, die als Peptid-Linker erfindungsgemäß eingesetzt werden, können auch in Form biologisch aktiver funktioneller Varianten derselben im Sinne dieser Erfindung und nach obiger Definition vorliegen.

Bei den erfindungsgemäßen Komponenten B kann es sich um identische oder verschiedene natürlich vorkommende Peptid-Sequenzen handeln. Sie können aus dem gleichen oder verschiedenen Organismen stammen. Bei den Organismen kann es sich um Vertrebraten, insbesondere um Säugetiere, beispielsweise um die Maus, die Ratte, das Schwein und vor allem um den Menschen handeln.

Das Polypeptid kann neben den Komponenten A und B zusätzliche Sequenzabschnitte aufweisen. Bevorzugt sind in diesem Zusammenhang sog. Tag-Sequenzen, beispielsweise mindestens ein Flag-Tag, also die Aminosäurenfolge DYKDDDDK, und/oder beispielsweise mindestens ein His-Tag (enthaltend mehrere konsekutive Histidine, bspw. mindestens 5) und/oder weitere Tag- oder anigenische Sequenzen. Ein besonders bevorzugtter zusätzlicher Sequenzabschnitt ist eine Leitpeptidsequenz. Vorzugsweise stellt diese Leitpeptidsequenz ein Signal zur Sekretion des Polypeptids bzw. Proteins in eukaryontischen Zellen dar. Diese Leitpeptidsequenz ist vorzugsweise N-terminal angeordnet.

In einer weiteren Ausführungsform der Erfindung umfasst ein Polypeptid eine weitere Komponente C, wobei diese charakterisiert ist durch spezifische Wechselwirkung mit einem komplementären Zelloberflächenmolekül ("Antigen"). Das Wirkprinzip der Polypeptidkonstrukte ist insbesondere für all diejenigen Mitglieder der TNF-Ligandenfamilie zutreffend, die für bestimmte Rezeptoren ausschließlich oder in besonders gutem Maße als Membranmolekül wirksam sind. Hierzu gehören neben TRAIL (TNFSF10), FasL (INFSF6) und TNF (INFSF2) bspw. auch die Immunmodulatoren CD40L (INFSF5) und CD30L (INFSF8). Komponente C hat damit "targeting" Funkti-on. Dementsprechend ist die Komponente C ein Antikörperfragment, vorzugsweise ein Einzelketten Antikörperfragment, sog. scfv, oder ein Antikörperdetivat das ein spezifisches Zielmolekül auf einer Zelloberfläche erkennt In einer weiteren Ausführungsform ist die Komponente C ein nicht den Antikörpern zugehötiges Protein bzw. Peptid, welches aber analog zu einer Antikörper-Antigen-Wechselwirkung bzw. einer Rezeptor-Ligand-Wechselwirkung ebenfalls ein spezifisches Zielmolekül auf einer Zelloberfläche selektiv erkennt. In einer besonders bevorzugten Ausführungsform umfasst die vorliegende Erfindung ein wie oben beschriebenes Polypeptid als Komponente A mindestens ein scTNF, sowie als Komponente C ein wie oben beschriebenes Antikörperfragment, z.B. scFv. Dadurch entstehen kleinere Polypeptide mit jeweils einer "tatgeting-Domäne". Solche kleineren Polypeptide sind vorteilhafterweise weniger anfällig für z.B. Aggregation.

Bevorzugt handelt es sich bei der Komponente C um ein Antigen-bindendes Antikörperfragment oder ein Antigen-bindendes Antikörperderivat eines Säugetiers, insbesondere murinen oder humanen Ursprungs, oder es handelt sich um ein humanisiertes Antikörperfragment oder ein humanisiertes Antikörperdetivat, z.B. mit Säugetierursprung. Im Falle der Derivatisierung und/oder Humanisierung besteht die Komponente C typischerweise aus einem nach dem Stand der Technik hergestellten single chain Fv-Derivat murinen, durch CDR grafting humanisierte Komponente C oder es handelt sich um eine Komponente C vollständig humanen Ursprungs die entsprechend zu einem scFv-Derivat umgewandelt wurde.

In einer weiteren bevorzugten Ausführungsform handelt es sich bei der Komponente C um einen Protein- oder Peptid-Liganden, der als Monomer eine spezifische Bindung an einem Membmnrezeptor eingeht

In einer weiteren bevorzugten Ausführungsform handelt es sich bei der Komponente C um ein Protein oder Peptid mit Spezifität für Zelloberflächenmoleküle, das insbesondere ein Zytokinrezeptor, ein Wachstumsfaktorrezeptor, ein Integrin oder Zelladhäsionsmolekül ist Besonders bevorzugt handelt es sich um einen Zytokinrezeptor, der aus der Gruppe der TNFR-Genfamilie ausgewählt ist

Die Komponente C eines Polypeptids weist vorzugsweise Spezifität für ein im Tumorgewebe selektiv bzw. dominant exprimiertes Antigen auf. Ein solches Tumorantigen kann generell auf den malignen Zellen selbst exprimiert sein oder auch im nicht malignen Anteil des Tumors, den Stromazellen oder dem Tumorendothel exprimiert sein. Derartige Antigene nicht-maligner Gewebeanteile eines soliden Tumors (Karzinom) sind auf der einen Seite genetisch invariant, auf der anderen Seite bei unterschiedlichsten Tumorentitäten vorkommend und damit univetselle Tumormarker Beispiele für solche Liganden für Tumorassoziierung, gegen die eine Komponente C des Polypeptids gerichtet sein kann, sind der VEGFR bzw. der VEGFR/VEGF Komplex sowie das Integrin $a_v \beta_3$ und die Vibronektinisoform $\beta$ Fn als weitgehend selektive Zielstruktuten des Tumorendothels und das Fibroblast Activation Protein (als selektiver Marker des Tumorstromas). Alle vorgenannten Beispiele können mit spezifischen scFv wirksam erfasst werden, weswegen sich derartige scFv ("single chain Fv") besonders als Komponente C eines Polypeptids eignen. Auch Galectin kommt hierbei als Tumormarker, gegen den die Komponente C gerichtet ist, in Betracht

Demnach werden Autikörperfragmente in verschiedenen Antikörperformaten, bspw. scfv, insbesondere scFv40, als Komponente C besonders bevorzugt.

In einer anderen Ausführungsform der vorliegenden Erfindung erkennt das Polypeptid über seine Komponente C als spezifisches Zielmolekül einen Zellmembran-ständigen Rezeptor eines Mitglieds der TNF-Ligandenfamilie, bevorzugt unterschiedlich von dem TNF-Ligandenfamilien-Mitglied der Komponente A. Beispiele, die keine abschließende Aufzählung darstellen, solcher möglicher Liganden sind TNFSF1 (LTalpha), TNFSF2 (TNF), TNFSF3 (LTbefa), TNFSF4 (OX40L), TNFSF5 (CD40L), TNFSF6 (FasL), TNFSF7 (CD27L), TNFSF8 (CD30L), TNFSF9 (4-1BBL), TNFSF10 (TRAIL), TNFSF11 (RANKL), TNFSF12 (TWEAKL), TNFSF13 (APRIL), TNFSF143B (BLYS), TNFSF14 (LIGHT), TNFSF15 (VEGI), TNFSF16 (CD30L), und TNFSF18 (AITRL), sowie EDA, die oder deren funktionelle Fragmente oder funktionelle Varianten der nativen Sequenz oder der Fragmente gleichfalls als Komponente A in einem Polypeptidkonstrukt dienen können. Insbesondere werden diesbezüglich alle Membran-ständigen Typ II-Proteine (C-Terminus extrazellulär), deren funktionelle Fragmente oder funktionelle Varianten, die eine trimere Organisation ihrer Untereinheiten als Voraussetzung für die biologische Aktivität bedingen, mit offenbart.

Verfahren zur Herstellung eines erfindungsgemäß zu verwendenden Polypeptids umfassen typischerweise die folgenden Schritte (a) Kultivieren einer Wirtszelle unter geeigneten Bedingungen, (b) Expression einer entsprechenden Nukleinsäurekonstrukts unter geeigneten Bedingungen, und (c) Isolieren des Polypeptids aus den Wirtszellen und/oder dem Kulturüberstand.

Kultivieren einer Wirtszelle bedeutet, der Wirtszelle ein Wachstum in einem geeigneten Kulturmedium, bei einem geeigneten pH und geeigneten Temperaturen zu ermöglichen. Solche Wachstumsbedingungen sind von den jeweils verwendeten Wirtszellen abhängig und dem Fachmann gut bekannt Hinweise zum Kultivieren von Zellen finden sich auch in Maniatis et al. (2001), *supra*.

Die Expression des Polypeptids kann hierbei typischerweise nach dem Stand der Technik in geeigneten Expressionssystemen erfolgen, vorzugsweise als sezerniertes Produkt stabiler Transfektanten, z.B. CHO- Zellen oder anderer tierischer Zellen, wie Cos7 oder SF9 (Insektenzellen) , oder weiterer eukaryontischer Zellsysteme, beispielsweise *Pichia pastoris*. Vorzugsweise weisen die exprimierten Polypeptide jeweilige zur Sekretion in dem Zollsystem geeignete Leadersequenzen auf. Daher werden die zur Expression eingesetzten Vektoren auch codierende Abschnitte enthalten, die für eine funktionelle Leadersequenz codieren, z.B. wie in Brocks et aL (Immuaotechnology 3: 173- 184, 1997) für Säuger und Insektenzellen beschrieben oder unter Verwendung von beispielsweise pPIC- Zalpha Vektoren (Invitrogen, Karlsruhe, Deutschland) zur Expression und Sekretion in der Hefe *Pichia pastoris*.

Das Isolieren des Polypeptids aus der Wirtszelle kann durch Standardverfahren, wie Chromatographie-Verfahren, Präzipitationsverfahren usw., die zur Aufreinigung von Polypeptiden und Proteinen geeignet sind, erfolgen (s.a. Maniatis et aL (2001), *supra*. Gegenstand der vorliegenden Erfindung sind Verfahren zur extrakorporalen (*ex vivo*) Manipulation, Depletion und/oder Entfernung von in Körperflüssigkeiten enthaltenen Bestandteilen, wie z.B. Bindungspartnem einer wie oben definierten Komponente A oder daran bindende oder damit assoziierte Zellen. Solche extrakorporalen Verfahren umfassen bevorzugt Verfahren wie z.B. Apherese, insbesondere die grundlegenden Formen der Apherese, die Plasmapherese und die Cytapherese.

Die Plasmapherese beinhaltet erfindungsgemäß die extrakorporale Manipulation, Depletion und/ oder Entfernung bestimmter löslicher oder suspendierter Bestandteile im Plasmaanteil des Blutes, sowie die Rückführung des so behandelten Blutes in den Patienten. Dazu wird einem Patienten bevorzugt mittels einer Pheresemaschine peripheres Blut entnommen, dem Blut werden optional gerinnungshemmende Mittel zugesetzt und das Blut wird in seine Hauptkomponenten- feste (rote Blutkörperchen, weisse Blutkörperchen und Blutplättchen) und flüssige Anteile (Plasma)- aufgetrennt. Nach Auftrennung in diese Hauptbestandteile können die in der so erhaltenen Plasmafraktion enthaltenen löslichen oder suspendierten Bestandteile des Blutes in einem weiteren Verfahrensschritt manipuliert, depletiert und/ oder entfernt werden, beispielsweise unter Verwendung von erfindungsgemäß beschriebenen Polypetiden. Anschließend kann das so behandelte Blutplasma zusammen mit den vorher abgetrennten festen Blutbestandteilen zusammengegeben und dem Patienten reinjiziert werden. Der durch die Plasmapherese bedingte Volumensverlust kann im erfindungsgemäßen Verfahren weiterhin durch isotonische Kochsalzlösung ersetzt werden. Plasmapherese wird erfindungsgemäß bevorzugt unter Verwendung von Verfahren wie therapeutischem Plasmaaustausch (TPE) , Immunabsorption (IA) , Fällung (HELP) , differentieller Membranfiltration und anderen Mitteln durchgeführt Beispielhaft seien hier Plasmafiltrationssäulen genannt (siehe z.B. US 4, 619, 639, Asahi Medical Company, hier durch Referenz mit einbezogen) . Ebenfalls können Membranfiltrationssysteme (MDF) unter Verwendung von verschiedenen Partikeln und Oberflächen, bspw. Filtern wie, PlasmaFlo®OP- 05 (W) L und RheoFilter®AR2000 Blutfilter, eingesetzt werden (hergestellt durch Asahi Medical Company, Ltd. of Japan) . Alternativ können in erfindungsgemäßen Plasmaphereseverfahren alle geeigneten Oberflächen und Partikel verwendet wenden.

Bei der Cytapherese werden erfindungsgemäß im Unterschied zur zuvor beschriebenen Plasmapherese extrakorporal im Blut zirkulierende und/ oder an Knochenmark gebundenen zelluläre Bestandteile (rote Blutkörperchen, weiße Blutkörperchen, Stammzellen oder Thrombozyten) oder spezifische Subpopulationen dieser Zellen manipuliert, depletiert und/ oder entfemt, um einen klinischen Effekt zu erzielen. Auch hier wird bevorzugt einem Patienten mittels einer Pheresemaschine peripheres Blut entnommen. Dem Blut werden danach optional gerinnungshemmende Mittel zugesetzt und das Blut wird in seine Hauptkomponenten aufgetrennt. Anschließend können in der die Zellbestandteile enthaltenden Fraktion des Blutes diese Zellbestandteile in einem weiteren Verfahrensschritt manipuliert, depletiert und/ oder entfernt werden, bevorzugt ebenfalls unter Verwendung von erfindungsgemäß beschriebenen Polypeptiden. Die so behandelte Fraktion kann abschließend dem Patienten wieder reinjiziert werden. Bevorzugt erfolgt bei der erfindungsgemäßen Cytapherese die Auftrennung des Blutes in verschiedene Fraktionen sowie gegebenenfalls die Abtrennung bestimmter Zellbestandteile des Blutes mittels Verfahren wie Zentdfugation, differentieller Membranfiltradon, oder anderen Mitteln. Es können weiterhin Membranfiltrationssysteme (MDF) unter Verwendung von verschiedenen Partikeln und Oberflächen, bspw. Filtern wie, PlasmaFlo®OP- (W) L und RheoFilter®AR2000 Blutfilter, eingesetzt werden (hergestellt durch Asahi Medical Company, Ltd. of Japan) .

In einer dritten erfindungsgemäßen Alternative können die oben beschriebenen Verfahren der Plasmapherese und der Cytapherese miteinander kombiniert werden, beispielsweise um sowohl lösliche oder suspendierter Bestandteile im Plasmaanteil des Blutes wie auch im Blut zirkulierende und/oder an Knochenmark gebundenen zelluläre Bestandteile (rote Blutkörperchen, weiße Blutkörperchen, Stammzellen oder Thrombozyten) oder spezifische Subpopulationen dieser Zellen zu manipulieren, depletieren und/oder entfernen, um einen klinischen Effekt zu erzielen. Eine solche Kombination aus Plasmapherese und Cytapherese wird unter Verwendung der beschriebenen Polypeptide hergestellt.

Die vorliegende Erfindung betrifft ein (Apherese- ) Verfahren gemäß Anspruch 1.

In einer besonderen Ausführungsform des erfindungsgemäßen Aphereseverfahrens kann vor der gegebenenfalls stattfindenden Auftrennung des Blutes optional eine Blutentnahme an einem Patienten durchgeführt werden. Weiterhin kann optional das mit dem erfindungsgemäßen Verfahren behandelte Blut oder die so behandelte Blutfraktion dem Patienten wieder reinjiziert werden. Dazu ist es gegebenenfalls erforderlich, die zuvor aufgetrennten und unter Umständen verschieden behandelten Blutfraktionen mit anderen nicht behandelten festen und/oder flüssigen Bestandteilen des Blutes wiederzuvereinigen. Durch das erfindungsgemäße Verfahren entstandene Volumenverluste können durch Zugabe geeigneter Flüssigkeiten, z.B. durch Zugabe einer isotonischen Kochsalzlösung, ausgeglichen werden.

Der im erfindungsgemäßen Aphereseverfahren enthaltene Schritt zur Bindung der löslichen, suspendierten oder zellularen Bestandteile des Blutes an eine mit einem erfindungsgemäßen Polypeptid gekoppelten Oberfläche oder Partikel kann je nach Erfordernis einmalig oder mehrmalig durchgeführt werden, um eine gewünschte Selektivität zu erzielen

In dem erfindungsgemäßen Aphereseverfahren werden solche Oberflächen und/oder Partikel eingesetzt, die mit einem edindungsgemäßen, wie oben definierten Polypeptid, kovalent gekoppelt wurden. Dazu wird bevorzugt das Polypeptid über die im Polypeptid enthaltene(n) Kopplungsgruppe(n) A auf die Oberfläche und/oder den Partikel kovalent gekoppelt.

Eine Kopplung der erfindungsgemäß verwendeten Polypeptide über die einzelnen Komponenten A findet bevorzugt wie in DE 101 44 252 A1 beschrieben statt Die Kopplung der edindungsgemäß verwendeten Polypeptide erfolgt dabei bevorzugt an Oberfläche oder Partikeln (Träger) über eine Bindung zwischen ersten auf der Trägeroberfläche vorhandenen funktionellen Gruppen und im Polypeptid über die in Komponenten A enthaltenen Kopplungsgruppen. Diese Kopplungsgruppen sind bevorzugt komplementär zu den funktionellen Gruppen des Trägers und können mit diesen eine affine, vorzugsweise kovalente Bindungen eingehen. Vorzugsweise ist die funktionelle Gruppe der Komponente A innerhalb der Komponente A so positioniert, dass sie außerhalb der für die biologische Aktivität verantwortlichen Domänen des Polypeptids in einem geeigneten Abstand angeordnet ist Auf diese Weise ist es erfindungsgemäß möglich, TNF gerichtet und unter Beibehaltung seiner biologischen Aktivitäten auf dem Träger zu immobilisieren. Nach Immobilisierung ist das Polypeptid bevorzugt so auf der Trägeroberfläche fixiert, dass die dreidimensionale Struktur der für die biologische Aktivität erforderlichen Domäne(n) gegenüber nicht-immobilisiertem Polypeptid nicht verändert ist, und dass die Domäne(n) bei Kontakt mit zellulären Reaktionspartnern für diese frei zugänglich ist/sind. In bevorzugter Ausführungsform der Erfindung ist die funktionelle Gruppe der Trägeroberfläche ausgewählt aus der Gruppe bestehend aus Aminogruppe, Carboxylgruppe, Epoxygruppe, Maleinimidogruppe, Alkylketongruppe, Aldehydgruppe, Hydrazingruppe, Hydrazidgruppe, Thiolgruppe und Thioestergruppe.

Erfindungsgemäss werden die Kopplungsgruppen der zu immobilisierenden Komponenten A des Polypeptids ausgewählt aus einer Gruppe, die die gleichen Spezies wie für die funktionelle Gruppe der Trägeroberfläche enthält. Eine im erfindungsgemäßen Aphereseverfahren verwendbare Oberfläche/Partikel (= Träger) weist also auf seiner Oberfläche eine funktionelle Gruppe auf, die kovalent mit einer Kopplungsgruppe des zu immobilisierenden Polypeptids verknüpft ist, wobei die Kopplungsgruppe des Polypeptids eine andere Gruppe als die funktionelle Protein des Trägers ist. Die beiden mit-einander in Bindung tretenden Kopplungsgruppen und funktionellen Gruppen müssen dabei komplementär zueinander sein, das heißt in der Lage sein, eine kovalente Bindung mit- einander einzugehen. Wird erfindungsgemäß als funktionelle Gruppe des Trägers beispielsweise eine Aminogruppe verwendet, ist die Kopplungsgruppe der Komponente A des erfindungsgemäßen Polypeptids eine Carboxygruppe. Wird erfindungsgemäß umgekehrt eine Carboxygruppe als funktionelle Gruppe des Trägers verwendet, ist erfindungsgemäß die Kopplungsgruppe der Komponente A des Polypeptids eine Aminogruppe. Wird erfindungsgemäß als funktionelle Gruppe des Trägers eine Thiolgruppe gewählt, ist erfindungsgemäß die komplementäre Kopplungsgruppe der Komponente A des Polypeptids eine Maleinimidogruppe. Wird umgekehrt eine Maleinimidogruppe als funktionelle Gruppe des Trägers ausgewählt, ist erfindungsgemäss die komplementäre Kopplungsgruppe der Komponente A des Polypeptids eine Thiolgruppe. Wird erfindungsgemäß als funktionelle Gruppe des Trägers Alkylketongruppe, insbesondere Methylketon-oder Aldehydgruppe verwendet, ist die funktionelle komplementäre Kopplungsgruppe der Komponente A des Polypeptids eine Hydrazin- oder Hydrazidgruppe. Wird erfindungsgemäß umgekehrt eine Hydrazin-oder Hydrazidgruppe als funktionelle Gruppe des Trägers verwendet, ist erfindungsgemäß die funktionelle komplementäre Kopplungsgruppe der Komponente A des Polypeptids eine Alkylketon-, insbesondere Methylketon- oder Aldehydgruppe. Erfindungsgemäß besonders bevorzugt handelt es sich bei der funktionellen Gruppe auf der Trägeroberfläche um eine Maleinimido-Gruppe und bei der Kopplungsgruppe der Komponente A des Polypeptids um eine Thiol-Gruppe.

Die Immobilisierung erfolgt dabei bevorzugt gerichtet. Im Zusammenhang mit der vorliegenden Erfindung bedeutet der Begriff "gerichtet immobilisiert" oder "gerichtete Immobilisierung", dass ein ein scTNF, an definiaten Positionen innerhalb des scTNF dergestalt an einem Träger immobilisiert ist, dass die dreidimensionale Struktur der für die biologische Aktivität erforderlichen Domäne(n) gegenüber dem nicht- immobilisierten Zustand nicht verändert ist und dass diese TNF-Domäne (n), beispielsweise Bindungstaschen für zelluläre Reaktionspartner, bei Kontakt mit zellulären Reaktionspartnern für diese frei zugänglich ist/sind. "Gerichtet immobilisiert" bedeutet auch, dass die Kopplung des Polypeptids auf der Trägeroberfläche so erfolgt, dass das immobilisierte Protein bei einer späteren Verwendung in einer zellulären beziehungsweise zellähnlichen Umgebung durch Protein-degradierende Enzyme nicht oder nur sehr langsam degradiert werden kann. Das heißt, das immobilisierte TNF-Molekül ist auf der Trägeroberfläche so ausgerichtet, das es so wenig wie möglich Angriffspunkte für Proteasen bietet. Zusätzlich kann das Polypeptid vor der Kopplung durch molekularbiologische Verfahren gegen Proteasen resistent gemacht werden, wie z.B. oben für TNF-Mitglieder bei der Protease TACE beschrieben.

Das Polypeptid behält via seiner gekoppelten Komponente(n) A seine biologische Funktion und die Fähigkeit, mit anderen Verbindungen/Stoffen eine Wechselwirkung einzugehen. Enthält ein Polypeptid beispielsweise drei Komponenten A (Trimer), so werden typischerweise jede, zwei oder bevorzugt nur eine der drei Komponenten A auf der Oberfläche/dem Partikel kovalent gebunden.

Zur Kopplung geeignete Oberflächen und/oder Partikel können alle geeigneten Oberflächen und/oder Partikel umfassen, an die sich die Polypeptide koppeln lassen. Bevorzugt sind insbesondere Kulturplatten oder sogenannte Mikrobeads, z.B. Dynabeads (Dynal Biotech GMBH), nano-beads, Nanopartikel, oder Festphasen wie beispielsweise Nylonwolle, Sepharose, Sephadex, etc.. Erfindungsgemäß ist weiterhin vorgesehen, dass es sich bei den erfindungsgemäss verwendeten Trägersystemen um kompakte oder hohle Nanopartikel mit Grössen zwischen 25 und 1000 nm handelt. Diese bestehen entweder aus organischen oder anorganischen Partikelmaterialien. Die Art des Materials kann erfindungsgemäss entsprechend der späteren Anwendung variiert werden, wobei der Träger der Nanopartikel vorzugsweise aus biologisch verträglichen und/oder biologisch abbaubaren Materialien besteht Im Zusammenhang mit der vorliegenden Erfindung werden unter "Nanopartikeln" weiterhin Bindematrizen verstanden, die einen Träger mit einer Oberfläche umfassen, an dem chemisch reaktive funktionelle Gruppen angeordnet sind, die mit komplementären funktionellen Gruppen von zu bindenden Molekülen, insbesondere Polypeptide, affine Bindungen, also kovalente und/oder nicht-kovalente Bindungen, eingehen und auf diese Weise die Polypeptide an ihren Oberflächen stabil fixieren können. Die Träger der Nanopartikel bestehen aus chemisch inerten anorganischen oder organischen Materialien und besitzen eine Grösse von weniger als 1$\mu$m, vorzugsweise von 25 bis 500 nm.

Eine besondere Ausführungsform des erfindungsgemäßen Aphereseverfahrens beinhaltet eine biofunktionalisierte Oberfläche (planar oder als Partikel) mit dem Polypeptid scTNF, welches in der zuvor beschriebenen Art und Weise kovalent und gerichtet auf der Oberfläche immobilisiert wurde. Ein derart biofunktionalisierte Oberfläche, enthaltend beispielsweise mehrere immobilisierte scTNF Moleküle oder Varianten davon, ermöglicht eine hohe Affinität für die jeweiligen komplementären Bindungspartner, beispielsweise TNFR1 und TNFR2. Die Rezeptoren TNFR1 und insbesondere TNFR2 werden in stark erhöhten Konzentrationen als prozessierte, lösliche Moleküle im Blut von Tumorpatienten und bei anderen Erkrankungen gefunden. Die Entfernung von TNFR aus dem Blut von Tumorpatienten führt nachweislich zu klinisch dokumentierten Tumorregressionen, was auf eine Rekonstitution der körpereigenen Abwehr zurückgeführt wird (siehe bspw. Lentz M R, (1999) Therapeutics Apheresis, Vol. 3, No. 1). Ein Aphereseverfahren mit scTNF ermöglicht, dass bevorzugt beide TNF Rezeptoren gleichzeitig und effizient aus Proben entfernt werden können. Ein weiterer Vorteil dieses Verfahrens ist die oben beschriebene Tatsache, dass eine Dissoziation einzelner Komponenten A des Polypeptids nicht möglich ist, d.h. weder ein Aktivitätsverlust der funktionalisierten Oberfläche noch eine Kontamination des behandelten Blutes mit abdissoziierten Stoffen zu erwarten ist.

Das erfindungsgemäße Aphereseverfahren kann als kontinuierliches Verfahren oder als diskontinuierliches Verfahren durchgeführt werden. Als kontinuierliches Verfahren wird das erfindungsgemäße Verfahren bevorzugt direkt anschließend an die Blutentnahme bzw. die Fraktionierung des entnommenen Blutes und vor der Rückführung des Blutes in den Patienten ohne zwischenzeitliche Lagerung des Blutes oder der erhaltenen Blutfraktionen durchgeführt In einem diskontinuierlichen Verfahren kann dagegen das entnomme Blut bzw. die Fraktionen des entnommenen Blutes nach jedem Schritt für einen bestimmten Zeitraum gelagert werden.

Die Temperatur in erfindungsgemäßen Aphereseverfahren liegt bevorzugt bei 0-40°C. Bei einen kontinuielichen Verfahren liegt die Temperatur bevorzugt bei bei 25 bis 40°C, besonders bevorzugt in einem Bereich zwischen 36 und 38°C. Bei einen diskontinuierlichen Verfahren kann die Tamperatur zwischen 0-40°C liegen.

Das wie oben beschriebene erfindungsgemäße Aphereseverfahren wird bevorzugt zur Diagnose, Therapie und/oder Prophylaxe bei mit TNF assoziierten Erkrankungen eingesetzt, insbesondere bei Tumorerkrankungen, insbesondere soliden oder lymphatischen Tumoren, insbesondere soliden und lymphatischen Tumoren. Die Wiederherstellung der Homöostase des Immunsystems mit seinen humoralen und zellulären Komponenten wird als Schlüssel der Wirksamkeit von Aphereseverfahren angesehen. In einer weiteren Anwendung kann das erfindungsgemäße Apheresesystem deshalb auch zur Wiederherstellung der Immunhomöostase bei nicht malignen Erkrankungen eingesetzt werden. Dazu gehören inflammatorische Erkrankungen, arthritische und theumatischen Erkrankungen, oder Erkrankungen des Immunsystems, sowie zur Behandlung Infektionskrankheiten, Stoffwechselerkrankungen, Entzündungszuständen, hyperproliferatorische Erkrankungene Autoimmunerkrankungen, insbesondere rheumatoider/arthritischer Erkrankungen, toxischer epidermal Nekrolyse (TEN), Multiple Sklerose, Hashimoto Thyroiditis, GvHD, virale Hepatitis (HBV, HCV), Alkohol-induzierter Hepatitis, Abstoßungsreaktionen bei Lebertransplantation, Erkrankungen, die auf hyperapoptotischen Reaktionen beruhen, degenerativen Erkrankungen, insbesondere neurodegenerativen Erkrankungen.

Bei Tumorerkrankungen sind insbesondere mit umfasst Kolonkarzinome, Melanome, Nierenkarzinome, Lymphome Akute myeloide Leukämie (AML) , Akute Lymphoide Leukämie (ALL) , chronische myeloide Leukämie (CML) , chronische lymphozytische Leukämie (CLL) , Gastrointestinale Tumore, Lungenkarzinome, Gliome, Schilddrüsentumore, Mammakarzinome, Prostatatumore, Hepatome, diverse virus- induzierte Tumoren wie z.B. Papillomvirusinduzierte Karzinome (z.B. Cervixkarzinom) , Adenokarzinome, Herpesviren- induzierte Tumore (z.B. Burkitt's Lymphom, EBV-induziertes B Zelllymphom) , Hepatitis B- induzierte Tumore (Hepatozellkarzinome) , HTLV- 1 und HTLV- 2 induzierte Lymphome, Akustikusneurinom, Gebärmuttehalskrebs, Lungenkrebs, Rachenkrebs, Analkarzinom, Glioblastom, Lymphome, Rektumkarzinom, Astrozytom, Hirntumore, Magenkrebs, Retinoblastom, Basaliom, Himmetastasen, Medulloblastome, Scheidenkrebs, Bauchspeicheldrüsenkrebs, Hodenkrebs, Melanom, Schilddrüsenkarzinom, Blasenkrebs, Hodgkin- Syndrom, Meningeome, Schneeberger Krankheit, Bronchialkarzinom, Hypophysentumor, Mycosis fungoides, Speiseröhrenkrebs, Brustkrebs, Karzinoide, Neurinom, Spinaliom, Burkitt- Lymphom, Kehlkopfkrebs, Nierenkrebs, Thymom, Corpuskarzinom, Knochenkrebs, Non- Hodgkin- Lymphome, Urethrakrebs, CUP- Syndrom, Kopf- Hals- Tumore, Oligodendrogliom, Vulvakrebs, Darmkrebs, Kolonkarzinom, Ösphaguskarzinom, Warzenbeteiligung, Dünndarmtumore, Kraniopharyngeome, Ovarial- Karzinom, Wächteiltumore, Eierstockkrebs, Leberkrebs, Pankreaskarzinom, Zervixkarzinom, Endometriumkarzinom, Lebermetastasen, Peniskrebs, Zungenkrebs, Gallenblasenkrebs, Leukämie, Plasmozytom, Gebärmutterkrebs, Lidtumor, Prostatakrebs, etc..

Arthritische Erkrankungen umfassen insbesondere Monarthritis, Oligoarthritis, Polyarthritis, akute Arthritis (v.a. septische, kristallinduzierte, reaktive Arthritis und akute Arkoidose) , subakute Arthritis, chronische Arthritis, (v.a rheumatoide Arthritis, Arthritis bei seronegativer Spondylarthritis) , infektiöse Arthritis, para- oder postinfektiöse Arthritis, rheumatoide Arthritis, juvenile chronische Arthritis, Arthritis bei entzündlichen Bindegewebeerrkrankungen und Vaskulitiden, allergische Arthritis, Arthritis in Verbindung mit Stoffwechselerkrankungen und ernährungsbedingten Störungen, Arthritis bei endokrinen Störungen, Arthritis bei granulomarosen Erkrankungen, Arthritis bei Erkrankungen des hämatopoietischen Systems, Arthritis bei Gelenkblutungen infolge von Störungen der Blutgerinnung, neoplastische Arthritis, paraneoplastische Arthritis, (post- ) traumatische Arthritis, Arthritis bei Erkrankungen des Gelenkkaorpels, Arthritis bei Neuropathien, Arthritis bei pustulösen abszedierenden, nekrotisierenden oder mit Gewebeneutrophilie einhergehenden Dermatosen, Arthritis bei sonstigen extraartikulären Grunderkrankungen, sowie Arthritis allergica, Chlamydien- induzierte Arthritis, Arthritis dysenterica, Arthritis gonnorrhoica, Arthritis mutilans, Arthritis psoriatica, Arthritis sicca, Arthritis syphilitica, Arthritis tuberculosa, Arthritis urica, etc..

Zusammenfassend ist festzustellen, daß hierin ein Polypeptid offenbart wird, das erhöhte Stabilität aufweist, dadurch dass das gesamte Molekül aus einem Protein- bzw. Polypeptidstrang besteht ( scTNF), so daß die Monomere (Komponenten A) nicht mehr dissoziieren können. scTNF zeigt in der Art seiner Bioaktivität keine qualitativen Unterschiede zu dem normalen löslichen sTNF (auch Wildtyp TNF oder wtTNF), ist jedoch weitaus stabiler und zeigt daher höhere Bioaktivität, ist also bei niedrigeren Konzentrationen wirksam. Wie normal lösliches TNF (wtTNF), vermag scTNF in sensitiven Zellen Apoptose auszulösen, den Transkriptionsfaktor NF-$\kappa$B zu aktivieren, den Rezeptor TNFR1, nicht aber TNFR2, zu aktivieren sowie in Gegenwart bestimmter Antikörper (z.B. 80M2) auch den Rezeptor TNFR2 zu aktivieren. Im Falle einer Verwendung der Polypeptide mit einer Kopplungsgruppe zur Herstellung biofunktionalisierter Oberflächen und Verwendung derselben bspw. zur Apherese ist kein Ausbluten des Polypeptids oder eine Rückführung von schädlichen Liganden in das System zu beobachten. Weiterhin stellt scTNF ein ideales Ausgangsmaterial zur Herstellung neuer, bi-funktioneller Moleküle dar. Zum einen kann scTNF bzw. funktionelle Fragmente hiervon kovalent an Zelloberflächen binden. Das hat den Vorteil, dass hier zur stabilen Bindung des Moleküls nur eine einzige kovalente Bindung hergestellt werden muss. Bei einem normalen löslichen Wildtyp Mitglied der TNF-Ligandenfamilie, beispielsweise löslichem sTNF, müssen alle drei Monomere des einzelnen Homotrimers kovalent an die Oberfläche geknüpft werden, um eine stabile Kontruktion zu erhalten, da sonst die nicht-kovalent gebundenen Monomere abdissoziieren. In dieser Ausführungsform ist eine Anwendung in der Apherese mit scTNF als biofunktionalem Wirkstoff zur Entfernung von TNF Rezeptoren aus Körperflüssigkeiten besonders vorteilhaft.

Ebenfalls ist es durch die vorliegende Erfindung möglich, aufgrund der kovalenten Verknüpfung der Monomere (Komponenten A) des Polypeptids gezielt Mutationen in nur eines oder auch zwei oder mehrere der mindestens drei Monomere (Komponenten A) stabil einzufügen. So sind z.B. Punktmutationen bekannt, welche eine Rezeptorselektivität erzwingen, d.h. zum Beispiel im Fall von TNF erfolgt eine Bindung nur noch an einen der beiden TNF-Rezeptoren (TNFR1 und TNFR2). Erfindungsgemäß ist es möglich, erstmals eine z.B. TNF-Mutante herstellen, die beispielsweise gezielt ein Molekül TNFR1 und zwei Moleküle TNFR2 gleichzeitig zu binden vermag, d.h. es entstünden heteromere Rezeptorkomplexe. Auf diese Weise ist es erstmals möglich, heteromere, stabile, single chain Mitglieder TNF-Ligandenfamilie herzustellen, die gezielt nur einen von mehreren möglichen Rezeptor binden und aktivieren.

Die vorliegende Erfindung wird durch die nachstehenden Figuren näher erläutert:

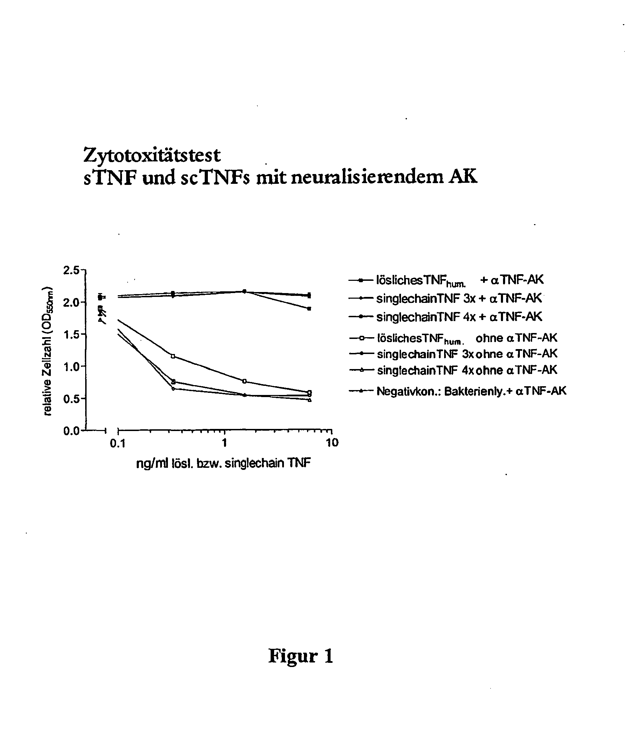

**Figuren 1 - 3** zeigen Ergebnisse verschiedener biologischer Zytotoxitätstests, in denen die Eigenschaften, insbesondere die Spezifität, unterschiedlicher scTNF Varianten mit denen von klassischem löslichem Wildtyp-TNF, d.h. der löslichen extrazellulären Domäne des humane TNF (NCBI gi:25952111, 17,09 kDa als Monomer der löslichen Form, abgekürzt mit TNFhum oder sTNF, AA 79-181) untersucht verglichen werden.

Für diese Versuche wurden sowohl transfizierte Mausfibroblasten als auch humane Kym1-Zellen verwendet Dargestellt werden beispielhafte Ergebnisse der Analysen, wobei scTNF Varianten getestet wurden, die unterschiedlich lange Peptid-Linker zwischen den einzelnen TNF Modulen (d.h. TNF Monomeren) aufweisen. Die Peptid-Linker bestehen jeweils aus der dreifachen bzw. der vierfachen Aminosequenzwiederholung GGGS (oder auch GlyGlyGly-Ser) (bezeichnet als "singlechainTNF3x" bzw. "singlechainTNF4x" oder als "scTNF3x" bzw. "scTNF4x").

**Figur 1** zeigt das Ergebnis eines typischen Zytotoxizitästests von TNF. Als Zielzellen wurden Mausfibroblasten (MF) aus TNFR1/TNFR2 doppel- knockout- Mäusen verwendet, die stabil TNFR1- Rezeptorchimären (INFR1- Fas) exprimieren (MF TNFR1- Fas- Zellen) . Solche Zellen exprimierten ein Hybridmolekül, das extrazellulär aus dem entsprechenden Teil des TNFR1 besteht, und somit TNF bindet, und intrazellulär die stark apoptotisch wirkende intrazelluläre Domäne des Fas- Rezeptors aufweist. Diese MF TNFR1- Fas Zellen können durch TNFR1- spezifische Stimuli aktiviert werden, vermittelten jedoch ein Fas- spezifisches Todessignal. Die überlebende Zellzahl wurde nach 24 Stunden durch Anfärben mit einem Farbstoff durch ihre Absorption quantifiziert. Als Negativkontrolle wurde ein Kontroll- Bakterienlysat eingesetzt, wobei es sich um ein nicht- scTNF exprimierendes Bakterienlysat handelte, das identisch zu den rekombinanten scTNF- Proteinen prozessiert wurde Im Ergebnis ist festzustellen, dass sowohl das herkömmliche humane lösliche TNF (TNFhum) als auch die rekombinanten scTNF- Varianten zum Zelltod führten. Es ist ein vergleichbarer dosisabhängiger zytotoxischer Effekt von TNFhum bzw. scTNF zu erkennen (siehe die unteren drei Kurven der Grafik.) , wobei die spezifische Aktivität des scTNF mindestens äquivalent, wenn nicht  sogar höher, als die des Wildtyp- TNF ist. Wie erwartet, zeigt die Negativkontrolle keinen toxischen Effekt.

In einem weiteren Versuchsansatz wurde die Wirkung neutralisierender TNF- spezifischer Antikörper auf die Zytotoxität von Wildtyp TNF (TNFhum) bzw. scTNF getestet Es wurden frisch angesetzte Verdünnungen von löslichem humanen TNF (TNFhum) bzw. den oben beschriebenen scTNF Varianten mit 3fach (3x) bzw. 4fach (3x) Glycin- Serin Peptid- Linker (scTNF3x: GGGS- GGGS- GGGS / scTNF4x: GGGS- GGGS- GGGS- GGGS) eingesetzt. Es wurden Konzentradonsreihen mit 1: 3 Verdünnungen in An- und Abwesenheit neutralisierender TNF- spezifischer Antikörper ($\alpha$TNF- AK) [1$\mu$g/mL) eingesetzt. Als Negativkontrolle wurde das oben beschriebenen Kontroll- Bakterienlysat eingesetzt Als Ergebnis ist zu erkennen, dass der zytotoxische Effekt von löslichem humanem Wildtyp- TNF bzw. scTNF durch neutralisierende TNF- spezifische Antikörper aufgehoben werden kann (siehe die oberen drei Kurven der Grafik.) und zwar in einem weiten Konzentrationsbereich (0, 03- 10 ng/mL) . Unterschiede zwischen der Reaktion von scTNF mit 3- fach und scTNF mit 4- fach Peptid- Linker wurden nicht festgestellt werden.

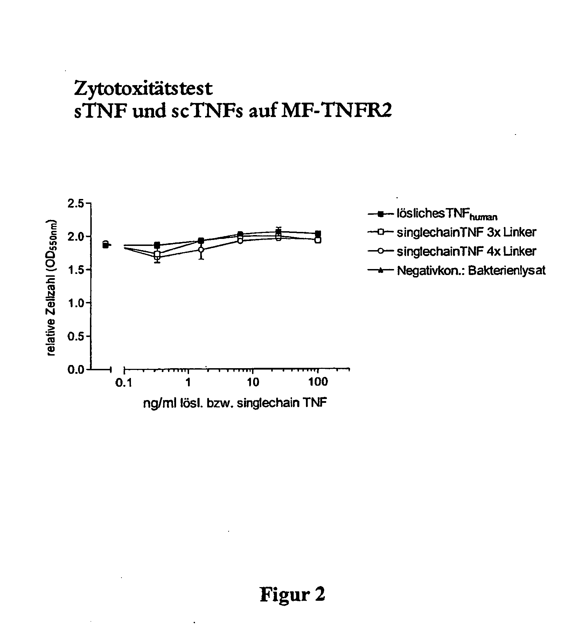

**Figur 2** zeigt das Ergebnis eines Zytotoxizitäts- Test, in dem die Wirkung von sTNF und scTNF- Varianten auf TNFR2- Rezeptorchimären (TNFR2- Fas) exprimierende Zellen untersucht wurde. In diesem Versuchsansatz wurden Mausfibroblasten eingesetzt, die stabil Rezeptorchimären, bestehend aus TNFR2 und Fas (TNFR2- Fas, wobei der extrazelluläre Teil von TNFR2, der intrazelluläre Teil vom Fas- Rezeptor stammt) , exprimieren (MF TNFR2- Fas Zellen) . MF TNFR2- Fas Zellen (wie auch der Wildtyp- TNFR2) können lediglich durch einen adäquaten TNFR2- Stimulus aktiviert werden, beispielsweise durch membranständiges TNF, nicht jedoch durch lösliches TNF. Ergebnis: Wie erwarten, zeigten sowohl das lösliche humane TNF (TNFhuman) als auch die scTNF Varianten mit 3fach und 4fach Peptid- Linker (scTNF3x, scTNF4x) keinen toxischen Effekt auf die MF TNFR2- Fas Zellen. Mit anderen Worten, sTNF und die scTNF- Varianten vermögen gleichermaßen TNFR2- Chimären (TNFR2- Fas) exprimierende Zellen nicht zu aktivieren, d.h. sie rufen in diesen Zellen keinen Zelltod hervor. Das als Negativkontrolle eingesetzte Kontroll- Bakterienlysat (wie oben beschrieben) zeigte erwartungsgemäß ebenfalls keinen toxischen Effekt.

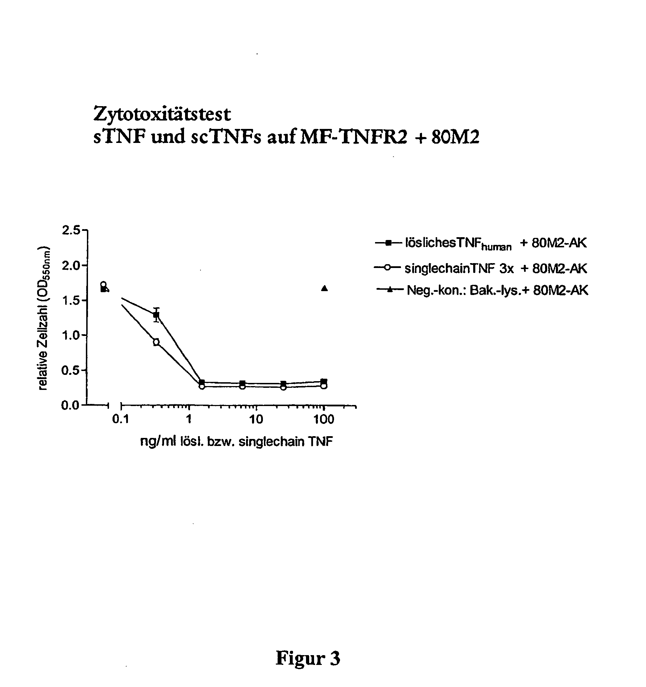

**Figur 3** zeigt das Ergebnis eines Zytotoxizitäts- Test, in dem die Wirkung von löslichem humanem Wildtyp-TNF und einer scTNF- Variante in Kombination mit einem speziellen TNFR2- spezifischem Antikörper, 80M2, auf TNFR2- Chimären (FNFR2- Fas) exprimierende Zellen untersucht wurde. Der Versuchsansatz entspricht dem aus Figur 2 mit dem Unterschied, dass das lösliche Wildtyp- TNF bzw. das scTNF mit 3fach Peptid- Linker (scTNF3x) zusammen mit dem Antikörper 80M2 zugegeben wurden. Der Antikörper 80M2 ist dafür bekannt, dass er normalem löslichem TNF die besondere Signalkapazität von membrangebundenem TNF verleiht. Das Ergebnis zeigt, das sowohl lösliches Wildtyp-TNF als auch scTNF in Kombination mit dem Antikörper 80M2 über die TNFR2- Fas Rezeptorchimäre einen starken zytotoxischen Effekt erzielen. Grund hierfür ist, dass lösliches TNF in Kombination mit bestimmten TNFR2- spezifischen Antikörpern, wie z.B. 80M2, die Aktivität von membrangebundenem TNF entfalten kann. MF TNFR2- Fas Zellen sterben nach Inkubation mit löslichem TNF bzw. dem scTNF in Anwesenheit eines solchen Ligand- Rezeptor Komplex- stabilisierenden Antikörpers (80M2- AK) . D.h., in Gegenwart dieses Antikörpers rufen lösliches TNF sowie scTNF gleichermassen einen toxischen Effekt in den MF TNFR2/FAS- Zellen hervor, wie dies sonst nur durch membranständiges TNF erfolgen kann. Das als Negativkontrolle eingesetzte Kontroll- Bakterienlysat (wie oben in Fig. 1 beschrieben) zeigte erwartungsgemäß keinen toxischen Effekt.

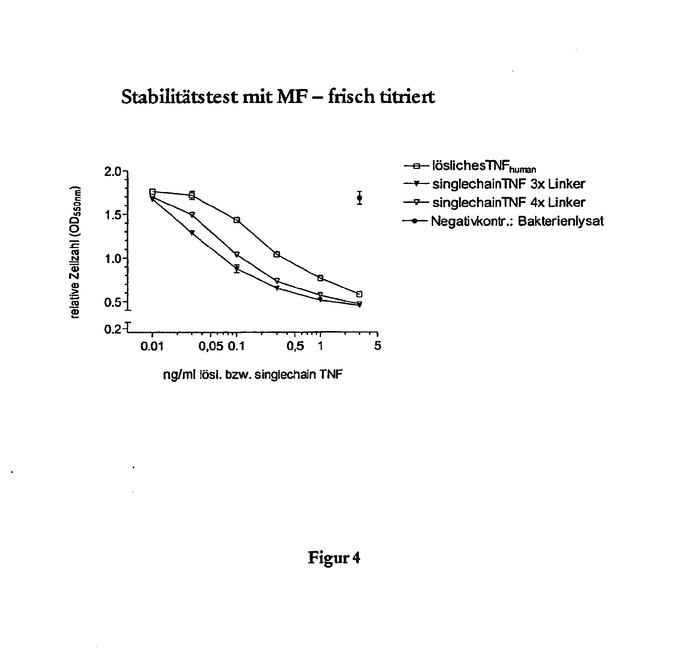

**Figuren 4 - 10** zeigen Ergebnisse verschiedener Stabilitätstests, die nachweisen, dass die erfindungsgemäßen scTNF Varianten aufgrund ihrer Struktur über eine erheblich bessere Stabilität verfügen als lösliches Wildtyp-TNF.

Es wurde sTNF bzw. die scTNF Varianten in serumhaltigem Kulturmedium bei Konzentrationen von 3,0 bis 0,01 ng/mL bei 37°C und 5% $CO_2$ für unterschiedliche Zeiten inkubiert. Im Anschluss wurde die Funktionalität der sTNF bzw. scTNF Varianten (scTNF3x, scTNF4x) in Zytotoxizitäts-Tests, basierend auf MF TNFR1-Fas Zellen und Kym1 Zellen, überprüft. Für diesen Zytotoxizitätstest wurden 1:3 Verdünnungen verwendet, ausgehend von einer 3,0 ng/ml TNF-Probe. Als Negativkontrolle wurde das in den oben beschriebenen Zytotoxitätstests verwendete Kontroll-Bakterienlysat eingesetzt (nicht-scTNF exprimierendes Bakterienlysat, das identisch zu den rekombinanten scTNF-Proteinen prozessiert wurde).

**Figur 4** zeigt die Bioaktivität der eingesetzten Moleküle VOR den Stabilitätstests auf Mausfibroblasten MF TNFR1-Fas Zellen. Es wurden lösliches humanes Wildtyp-TNF (TNFhuman) und scTNF Verdünnungen frisch angesetzt und auf den Zellen ausverdünnt. Sowohl das lösliche Kontroll-TNF als auch die scTNF Varianten führten nach Inkubation mit den MF zu deren Zelltod Das Kontroll-Bakterienlysat zeigte erwartungsgemäß keinen toxischen Effekt. Die $ED_{50}$-Werte (halbmaximale Wirkkonzentration) lagen bei ca. 0,2 ng/ml für das lösliche humane Wildtyp-TNF und bei 0,1 ng/ml für die zwei eingesetzten unterschiedlichen scTNF Varianten. Das als Negativkontrolle eingesetzte Kontroll-Bakterienlysat zeigte erwartungsgemäß keinen toxischen Effekt.

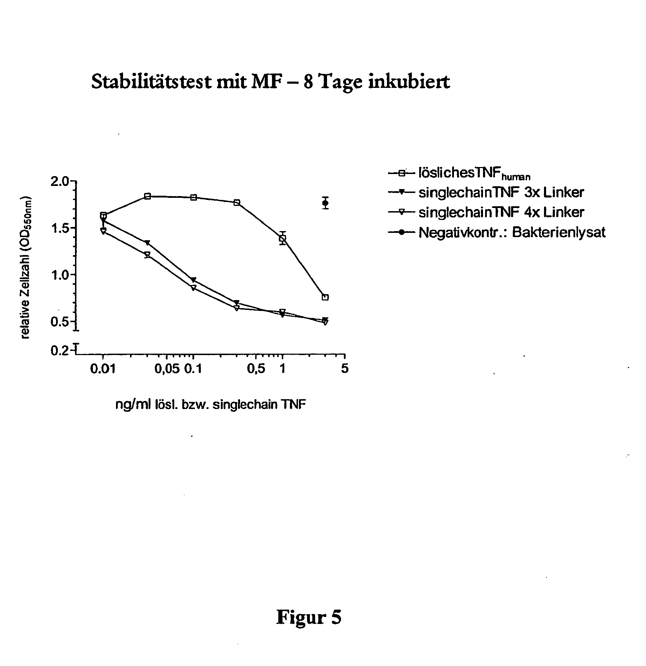

**Figur 5** zeigt das Ergebnis eines Stabilitätstest mit Mausfibroblasten MF TNFR1-Fas Zellen. Die in Figur 5 gezeigten Wildtyp-TNF und scTNF Verdünnungen waren hierbei für 8 Tage im Zellinkubator vor Durchführung des Tests inkubiert worden, um die Stabilität der Proben beurteilen zu können. Es konnte eine deutliche Abnahme der Bioaktivität des löslichen Wildtyp-TNF (TNFhuman) nach 8 Tagen bei 37°C gemessen werden, während die Aktivität beider scTNF Varianten (scTNF3x, scTNF4x; Erklärung siehe oben) unverändert blieb. Für das Wildtyp-TNF ergab sich ein $ED_{50}$-Wert von etwa 2 ng/ml (frisch verwendet 0,2 ng/ml, vgl. Figur 4). Die scTNF Varianten blieben mit $ED_{50}$ -Werten von ca. 0,1 ng/ml genauso aktiv wie die frisch eingesetzten Proben (vgl. Figur 4). Das als Negativkontrolle eingesetzte Kontroll-Bakterienlysat zeigte erwartungsgemäß keinen toxischen Effekt.

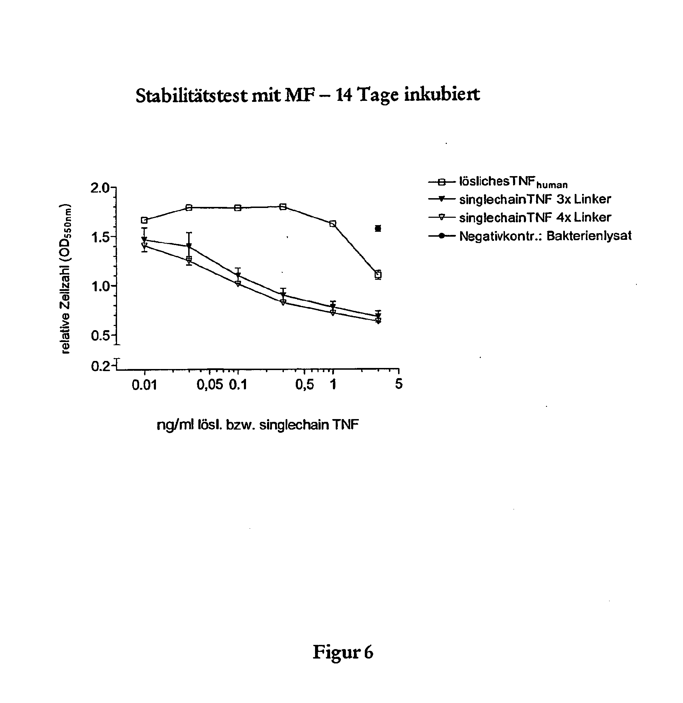

**Figur 6** zeigt das Ergebnis eines Stabilitätstest mit Mausfibroblasten MF TNFR1-Fas Zellen. Die Wildtyp-TNF und scTNF Lösungen waren wie in Figur 5 erklärt jetzt für 14 Tage im Zellinkubator inkubiert worden. Es konnte eine deutliche weitere Abnahme der Bioaktivität des Wildtyp-TNF nach 14 Tagen bei 37°C gemessen werden, während die Aktivität der scTNF Varianten annähernd gleich blieb. Für sTNF ergab sich ein $ED_{50}$ -Wert von etwa 2,8 ng/ml (frisch verwendet 0,2 ng/mL, vgL Figur 4), die scTNF Varianten blieben mit 0,13 ng/ml fast genauso aktiv wie die frisch angesetzten Proben (0,1 ng/ml, vgL Figur 4). Das als Negativkontrolle eingesetzte Kontroll-Bakterienlysat zeigte erwartungsgemäß keinen toxischen Effekt.

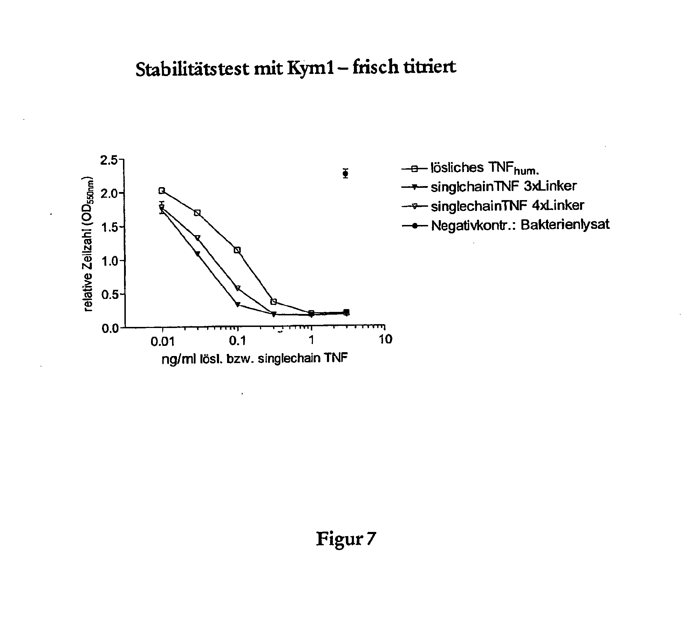

**Figur 7** zeigt das Ergebnis eines Stabilitätstest mit der humanen Zelllinie Kym1. Kym1 Zellen exprimieren normale humane Wildtyp TNF Rezeptoren und reagieren ebenfalls zytotoxisch auf TNF. Der Versuch wurde analog zu den Stabilitätstests mit Mausfibroblasten gemäß Figuren 4 - 6 durchgeführt: Es wurden lösliches Wildtyp-TNF bzw. scTNF Varianten (scTNF3x, scTNF4x) in serumhaltigem Medium bei Konzentrationen von 3,0 bis 0,01 ng/mL bei 37°C und 5% $CO_2$ für unterschiedliche Zeiten inkubiert. Nachfolgend wurde die Funktionalität des löslichen sTNF bzw. der scTNF Varianten in Zytotxizitäts-Tests auf Kym1-Zellen überprüft. Ausgehend von einer 3,0 ng/mL TNF-Probe wurden 1:3 Verdünnungen für den Test verwendet. Als Negativkontrolle wurde das oben beschriebene Kontroll-Bakterienlysat eingesetzt. Es wurden Wildtyp-TNF und scTNF Verdünnungen frisch angesetzt und eingesetzt. Das Ergebnis zeigt, dass sowohl Wildtyp-TNF als auch die scTNF Varianten starke zytotoxische Aktivität besaßen. Die $ED_{50}$-Werte liegen bei etwa 0,1 ng/ml für das Wildtyp-TNF und bei ca. 0,06 ng/mL für die scTNF Varianten. Das als Negativkontrolle eingesetzte Kontroll-Bakterienlysat zeigte erwartungsgemäß keinen toxischen Effekt

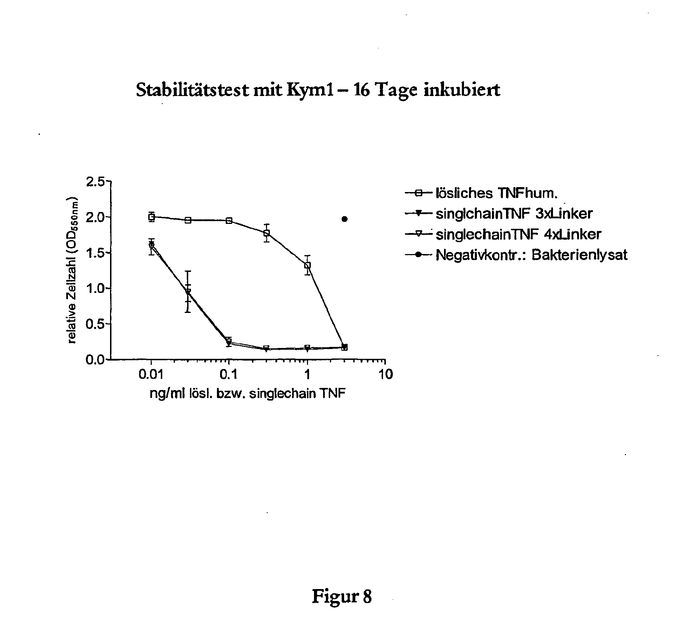

**Figur 8** zeigt das Ergebnis eines Stabilitätstest mit der humanen Zelllinie Kym1. Die Wildtyp-TNF und scTNF Lösungen wurden hierbei für 16 Tage im Zellinkubator inkubiert. Anschließend wurde der Kym1-Zellen Zytotoxizitäts-Test durchgeführt. Es konnte eine deutliche Abnahme der Aktivität des sTNF nach 16 Tagen bei 37°C gemessen werden, wohingegen die Toxizität, d.h. die Bioaktivität, der scTNF Varianten annähernd gleich blieb. Für Wildtyp-TNF ergab sich ein $ED_{50}$ -Wert von ca. 1,2 ng/mL Die scTNF Varianten blieben mit 0,06 ng/ml genauso aktiv wie die frisch angesetzten Proben. Das als Negativkontrolle eingesetzte Kontroll-Bakterienlysat zeigte erwartungsgemäß keinen toxischen Effekt

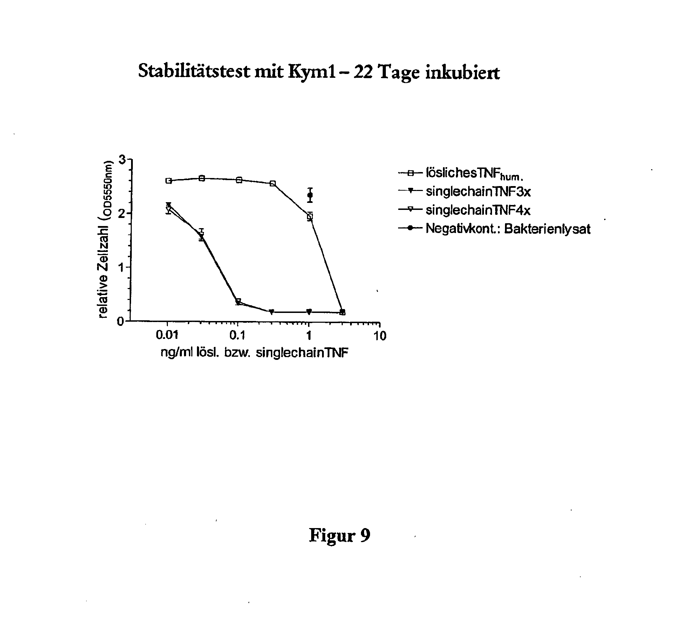

**Figur 9** zeigt das Ergebnis eines Stabilitätstest mit der humanen Zelllinie Kym1. sTNF und scTNF Lösungen wurden hierbei für 22 Tage im Zellinkubator inkubiert. Es konnte eine deutliche weitere Abnahme der Aktivität des sTNF nach 22 Tagen bei 37°C gemessen werden Die scTNF Varianten waren nach wie vor stabil. Das als Negativkontrolle eingesetzte Kontroll-Bakterienlysat zeigte erwartungsgemäß keinen toxischen Effekt.

Die nachfolgende **Tabelle 1** zeigt die Ergebnisdaten der Stabilitätstests gemäß Figuren 7 und 9 im Vergleich:

**Tabelle 1:** Vergleich der $ED_{50}$-Werte aus Figuren 7 + 9

|  | $ED_{50}$ frisch titriert (Figur 7) | $ED_{50}$ nach 22 Tagen (Figur 9) | Aktivitätsverlust |
|---|---|---|---|
| sTNF | 0,1 ng/mL | 1,50 ng/mL | >90% |
| scTNF | 0,06 ng/mL | 0,07 ng/mL | Kaum nachweisbar |

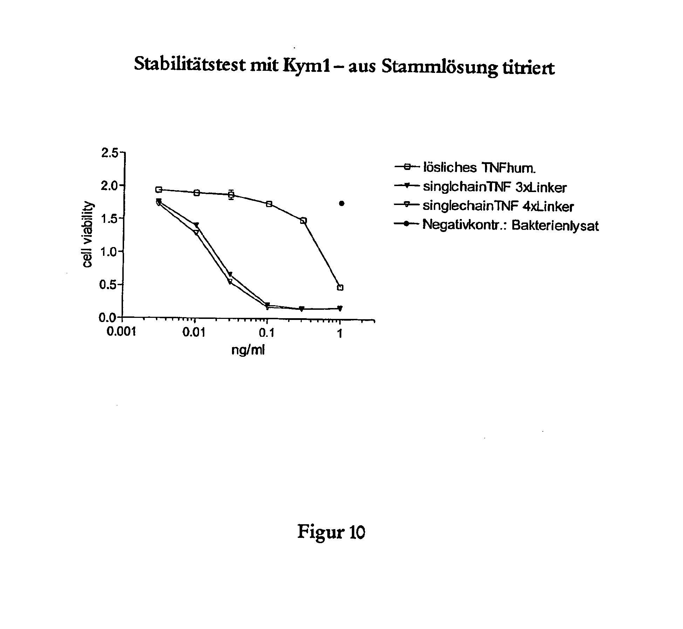

**Figur 10** zeigt das Ergebnis eines Stabilitätstest mit der humanen Zelllinie Kym1. Es wurden Verdünnungen aus 3 ng/ml nach 16 Tagen bei 37°C austitriert. Im Gegensatz zu den vorherigen Figuren 4 - 9 wurde in diesem Versuch eine Titrationskurve ausgehend von einer 16 Tage gelagerten 3 ng/mL TNF-Verdünnung angefertigt, während bei den vorherigen gezeigten Titrationskurven die jeweiligen Verdünnungen hergestellt und dann für die angegebene Zeit gelagert wurden. Es bleibt festzustellen, dass ein Vergleich der Daten aus Figur 8 (analoger Versuch mit 16 Tagen Inkubationszeit) und Figur 10 keine wesentlichen Unterschiede ergibt. Das als Negativkontrolle eingesetzte Kontroll-Bakterienlysat zeigte erwartungsgemäß keinen toxischen Effekt.

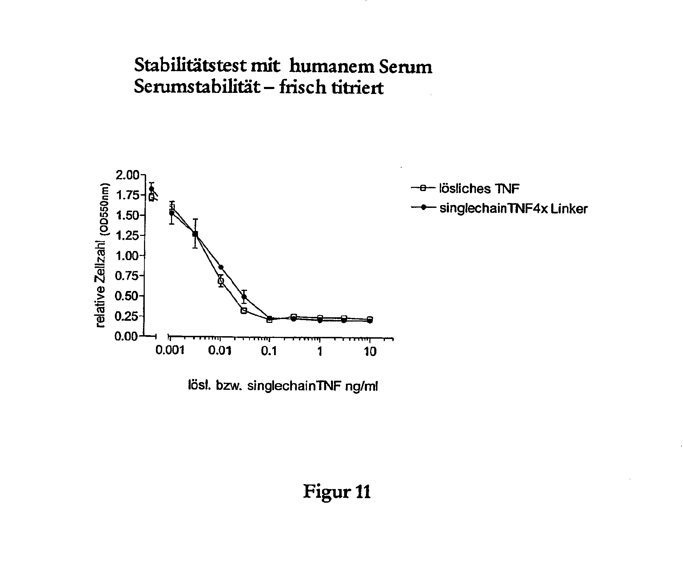

**Figur 11** zeigt das Ergebnis eines Stabilitätstests in humanem Serum. Um die Stabilität von Wildtyp-TNF und scTNF in humanem Serum zu testen, wurde in einem ersten Versuchsansatz das lösliche Wildtyp-TNF und die scTNF4x Variante in 100% Serum verdünnt und frisch austritiert. Als Ergebnis wurde für Wildtyp-TNF ein $ED_{50}$-Wert von 0,004 ng/ml gemessen und für die scTNF Variante ein $ED_{50}$-Wert von 0,007ng/ml.

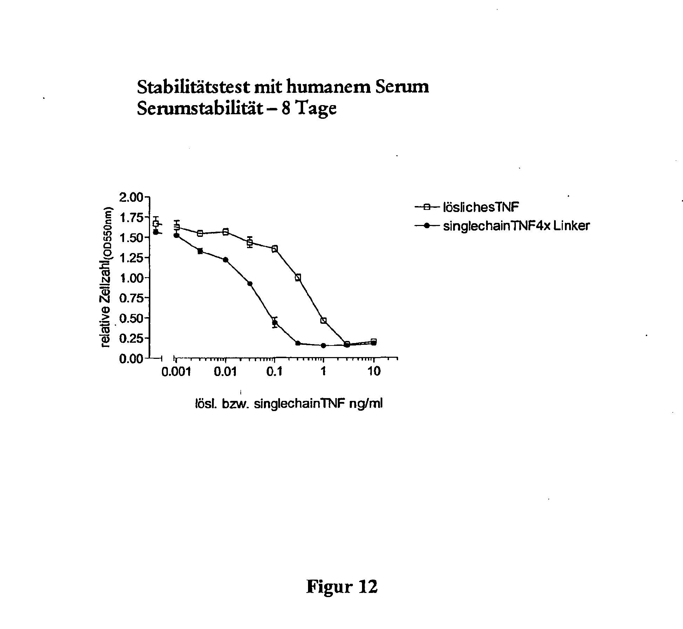

**Figur 12** zeigt das Ergebnis eines Stabilitätstests in humanem Serum. Um die Stabilität von Wildtyp-TNF und scTNF in humanem Serum zu testen, wurde in einem weiteren Versuchsansatz Wildtyp-TNF und die scTNF4x Variante in 100%igen frischem nicht-hitzeinaktivierten Serum für 8 Tage bei 37°C gelagert und anschließend auf Kym1 Zellen austitriert. Als Ergebnis wurde für Wildtyp-TNF ein $ED_{50}$-Wert von 0,40 ng/ml gemessen und für die scTNF Variante ein $ED_{50}$-Wert von 0,03 ng/ml. Die Auswirkungen der 8-tägigen Lagerung in 100%igem Serum stellten sich wie folgt dar: im Vergleich zum frisch austitrierten scTNF zeigte das 8 Tage gelagerte scTNF einen Aktivitätsverlust um den Faktor von ca. 4,3, wohingegen sich für das lösliche sTNF ein dramatischer Aktivitätsverlust um den Faktor 100 während der Lagerung in 100% Serum zeigte. Das erfindungsgemäße scTNF zeigte im Vergleich zum sTNF demnach eine erheblich höhere Bioaktivität nach 8-tägiger Inkubation in Serum und erwies sich damit als sehr stabil in humanem Serum bei physiologischen Temperaturen.

In der nachfolgenden **Tabelle 2** sind die Daten der Ergebnisse aus den Figuren 11 und 12 noch einmal zur Verdeutlichung aufgeführt.

**Tabelle 2:** Vergleich der $ED_{50}$-Werte aus Figuren 11 + 12

|  | $ED_{50}$ frisch titriert (Figur 11) | $ED_{50}$ nach 8 Tagen (Figur 12) | Aktivitätsverlust |
|---|---|---|---|
| LöslichesTNF | 0,004 ng/mL | 0,40 ng/mL | 100fach |
| Single chain TNF | 0,007 ng/mL | 0,03 ng/mL | 4,3fach |

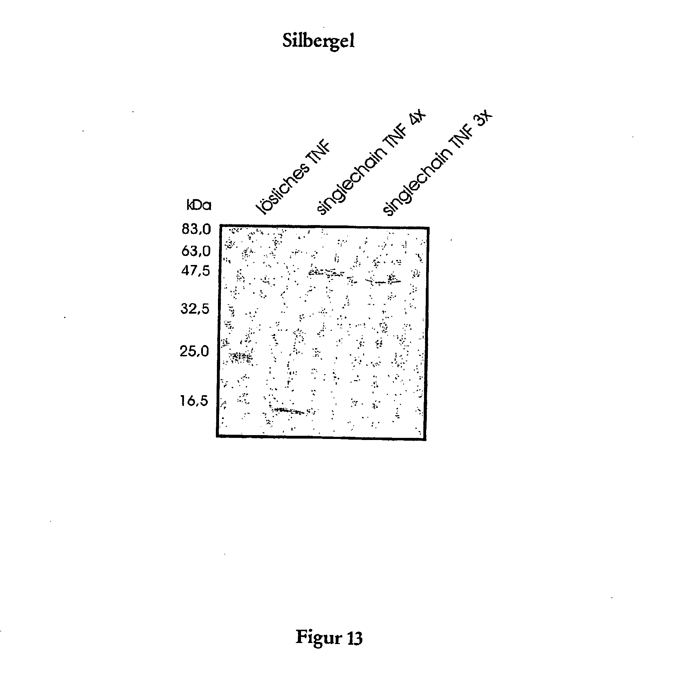

**Figur 13** zeigt das Ergebnis einer Analyse durch Polyacrylamid-Gelelektrophorese unter reduzierenden und denaturierenden Bedingungen. Dargestellt ist ein Silbergel von aufgereinigtem Wildtyp-TNF und den aufgereinigten scTNF Varianten scTNF3x und scTNF4x. Die Proben wurden jeweils mit β-Mercaptoethanol (final 5%) bei 95°C für 5 min inkubiert. Auf das Silbergel wurden ca. 500 ng sTNF und scTNF4x und ca. 150 ng scTNF3x pro Spur aufgetragen. Das Ergebnis des Silbergels der in einem 15%igem SDS-PAGE aufgetrennten scTNF Varianten zeigt, dass beide scTNF-Varianten auch unter reduzierenden Bedingungen ein Molekulargewicht von ca. 50 kDa aufwiesen, das mit ihrer Struktur übereinstimmt. Festzustellen ist auch, dass die unterschiedlichen Mengen der aufgetragenen Proteine scTNF3x und scTNF4x keinen Einfluss auf das Ergebnis hatten. Dies belegt die Stabilität der erfindungsgemäßen Proteine bzw. Polypeptide unter reduzierenden und denaturierenden Bedingungen. Das Ergebnis für sTNF hingegen zeigt, dass das Protein in seine Monomere von ca. 17 kDa zerfällt.

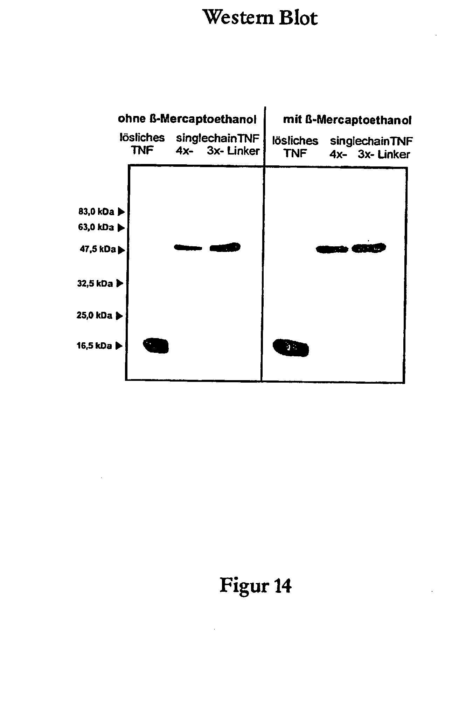

**Figur 14** zeigt das Ergebnis eines Stabilitätstest unter reduzierenden und denaturierenden Bedingungen. In diesem Versuchsansatz wurde ein Western Blot der in einem 15%igem SDS-PAGE aufgetrennten Proben von Wildtyp-TNF und den scTNF Varianten scTNF3x und scTNF4x durchgeführt. Es wurden zwei parallele Ansätze durchgeführt, bei denen die Proben in einem Ansatz jeweils mit β-Mercaptoethanol (final 5%) bei 95°C für 5 min inkubiert wurden, während in dem anderen Ansatz keine β-Mercaptoethanol Inkubation erfolgte. Die Detektion nach dem Lauf der Elektrophorese erfolgte mit einem AntiTNF-Antikörper. Als Ergebnis ist zu erkennen, dass der Antikörper das Protein beider scTNF Varianten in deutlichen Banden bei ca. 50 kDa spezifisch detektierte. Für Wildtyp-TNF wurden das Protein bei ca. 17 kDa spezifisch detektierte. Dieses belegt wiederum die Stabilität der erfindungsgemäßen scTNF3x und scTNF4x Varianten unter reduzierenden und denaturierenden Bedingungen und deckt sich ebenfalls erwartungsgemäß mit den Ergebnissen aus Figur 13.

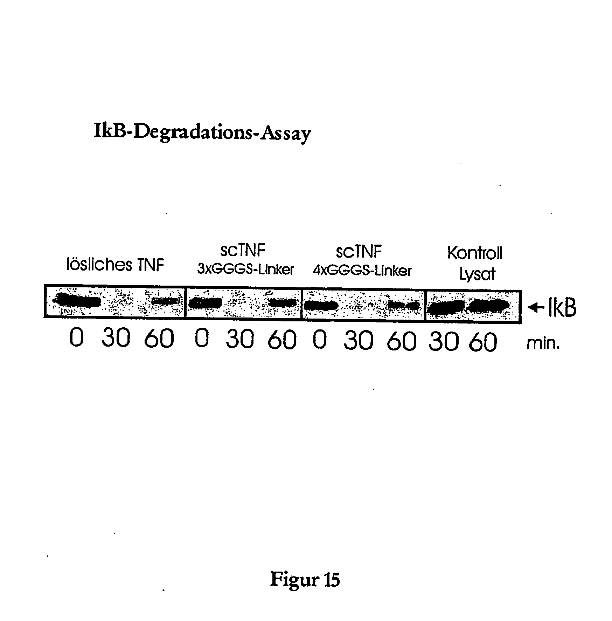

**Figur 15** zeigt das Ergebnis eines IkappaB-Degradations-Assays. IkappaB (I-κB) ist ein Inhibitor des Transkriptionsfaktors Nukleärer Faktor kappa B (NF-κB), der bekanntermaßen durch TNF der Degradierung zugeführt wird, wodurch NF-κB aktiviert wird. Für den Nachweis der Degradation von I-κB wurden Zell-Lysate 0, 30 und 60 Minuten nach Stimulierung mit jeweils 10 ng/ml Wildtyp-TNF, scTNF3x und scTNF4x hergestellt und nachfolgend im Western-Blot mit I-κB-spezifischen Antikörpern analysiert. Das Ergebnis zeigt, dass die transiente Degradation von I-κB sowohl von Wildtyp-TNF als auch von beiden scTNF Varianten induziert wurde, wobei das Reaktionsverhalten der scTNF Varianten dem des Wildtyp-TNF entsprach. Das als Negativkontrolle eingesetzte Kontroll-Bakterienlysat (wie oben beschrieben ein nicht-scTNF exprimierendes Bakterienlysat, das identisch zu den rekombinanten scTNF-Proteinen prozessiert wurde) zeigte erwartungsgemäß keinen Effekt auf die I-κβ-Degradation.

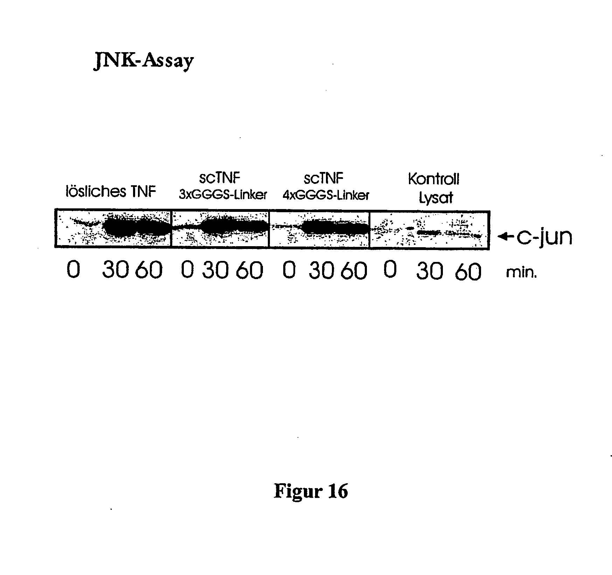

**Figur 16** zeigt das Ergebnis eines JNK-Assays. JNK (c-jun N-terminale Kinase) ist eine Stress-induzierte Kinase, welche durch TNF bekanntermaßen sehr stark aktiviert wird. Nach Stimulierung von Kym-1 Zellen für 0, 30 bzw. 60 Minuten mit jeweils 10 ng/ml Wildtyp-TNF, scTNF3x und scTNF4x wurden Zell-Lysate hergestellt und die JNK-Aktivität über Immunopräzipitation der JNK mit JNK-spezifischen Antikörpern und nachfolgendem Kinase-Assay mit GST c-Jun als Substrat überprüft. Das als Negativkontrolle eingesetzte Kontroll-Bakterienlysat (wie oben beschrieben) zeigte erwartungsgemäß keine Aktivierung der JNK-Kinase. Das Ergebnis zeigt, dass eine Aktivierung von JNK sowohl durch beide scTNF Variante als auch Wildtyp-TNF erfolgte, wobei das Reaktionsverhalten der scTNF Varianten dem des Wildtyp-TNF entsprach.

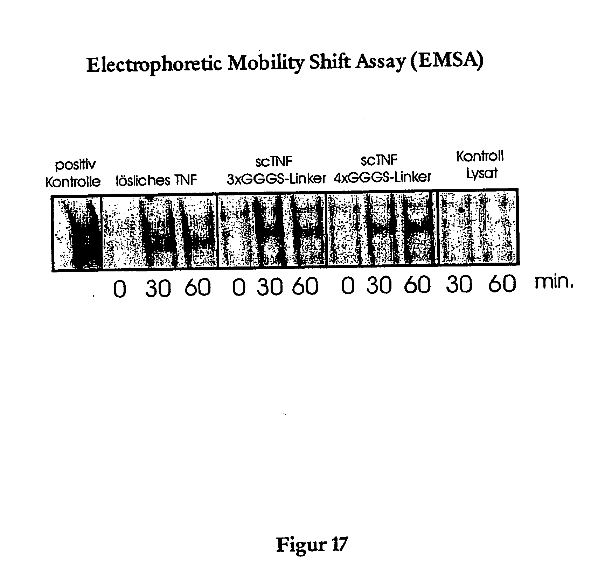

**Figur 17** zeigt das Ergebnis eines Electrophoretic Mobility Shift Assays (EMSA) . Einen weiteren typischen Nachweis für die Aktivität von TNF stellt die nach erfolgter Induktion der I-κB- Degradation ablaufende Translokation des Transkriptionsfaktors NF-κB in den Zellkern dar. Dazu wurden Zellkernpräparationen von nicht- stimulierten KYM-1 Kontrollzellen (Null Minuten Stimulierung) bzw. von stimulierten Zellen (30 und 60 Minuten Stimulierung) hergestellt Die Stimulierung der Zellen erfolgte jeweils mit Wildtyp- TNF, scTNF3x und scTNF4x. Der in den Zellkern transloziierte Transkriptionsfaktor NF-κB wurde mit Hilfe von NF-κB- spezifischen, radioaktiv markierten Oligonukleotiden nachgewiesen. Das Ergebnis zeigt, dass NF-κB in den Zellkern transloziert wurde. Dabei entspricht das Reaktionsverhalten der scTNF Varianten dem des Wildtyp- TNF.

**Figur 18** zeigt ein beispielhaftes Konstruktschema der erfindungsgemäßen Polypeptide, dargestellt anhand von TNF (als ein Mitglied der TNF-Ligandenfamilie). Die Bezeichunungen haben die folgenden Bedeutungen:

die Konstrukte AI/AII und BI/II weisen eine optimierte Codonusage für die Expression der Proteine in *E.coli* auf, und stellen Moleküle dar, die mit jeweils GlyGlyGlySer-Dreifachlinker bzw. Vierfachlinker verknüpft sind Alle Moleküle tragen N-terminal einen Histidin-tag zur leichteren Anreinigung, die Moleküle BI/BII tragen zusätzlich eine kurze Aminosäurenkette mit einem Cysteinrest zur gerichteten kovalenten Verknüpfung.

die Konstrukte C bis H sind geeignet für die Expression der Proteine in eukaryontischen Zellen, sie tragen N-terminal entsprechende Leitpeptidsequenzen.

sc = ist die Abkürzung für single chain,

cys = bezeichnet einen N-terminalen Peptidlinker mit internem Cystein für eine kovalente Kopplung,

$L1_{long}$ bzw. $L2_{long}$ = bezeichnet die Linker 1 bzw. 2, jeweils mit der Aminosäuresequenz (GGGS- GGGS- GGGS- GGGS) bzw. $(GGGS)_4$,

$L1_{short}$ bzw. $L2_{short}$ = bezeichnet die Linker 1 bzw. 2 jeweils mit der Aminosäuresequenz (GGGS- GGGS- GGGS) bzw. $(GGGS)_3$,

Leitpeptidsequenz = ist die Aminosäuresequenz für die Sekretion des Proteins aus eukaryontischen (Wirts- ) Zellen.

scFv40 = steht für die Sequenz des Einzelketten (scfv)- Antikörperfragmentes 40, das spezifisch für das Tumorstroma- Antigen FAP ist,

AMAIZe = ist die Abkürzung für "Antibody-Mediated Apoptosis Inducing Zytokine" und

His/Flag-Tag = steht für die Peptidsequenz zur Affinitätsreinigung der exprimierten Proteine

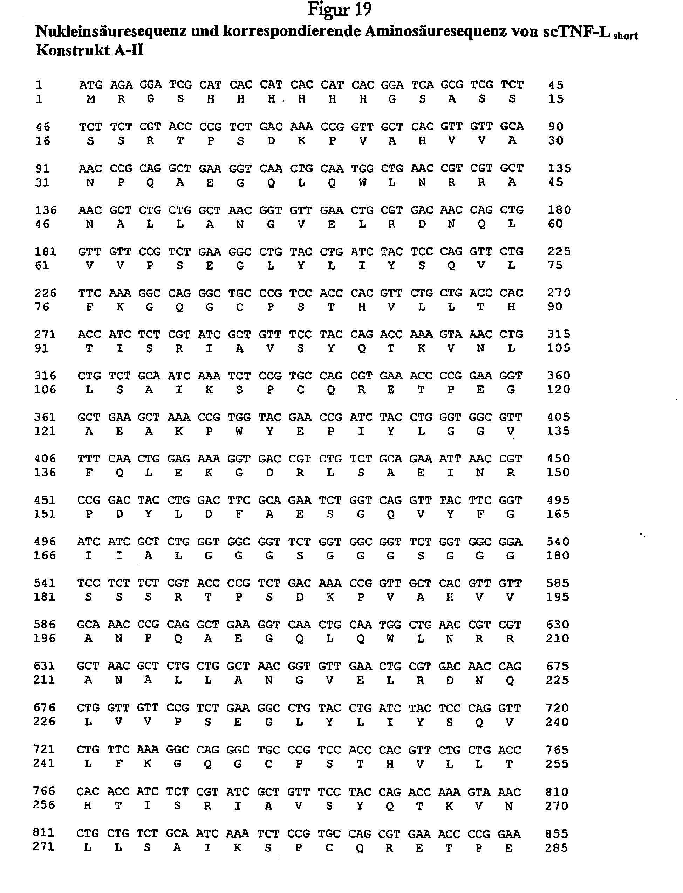

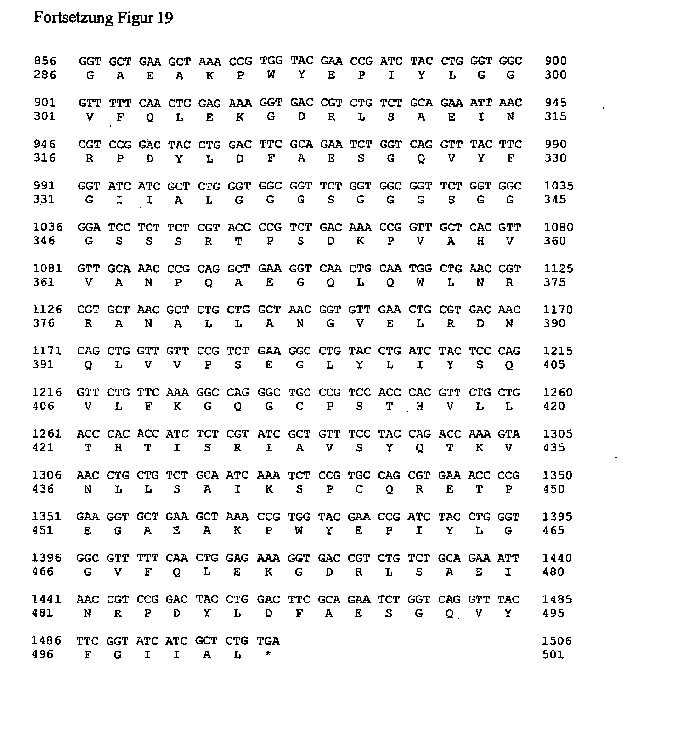

**Figur 19** zeigt die Nukleinsäuresequenz und die korrespondierende Aminosäuresequenz von scTNF-$L_{short}$ (Konstrukt A-II)

Merkmale Konstrukt A-II

- His-Tag Peptidsequenz zur Affinitätsreinigung des gebildeten Proteins: Aminosäuren (nachfolgend in Figuren 19 bis 26 mit "AA" abgekürzt) AA 5-10, Nukleotid (nachfolgend in Figuren 19 bis 26 mit "NT" abgekürzt) NT 13-30

- Sequenz des humanen TNF Modul1 (extrazelluläre Domäne, AA 79-181, des natürlichen, humanen TNF Moleküls, Sequenz mit optimierter E.coli Codonverwendung): AA 11-169, NT 31-507

- Sequenz des $(GGGS)_3$- Linker1: AA 170- 181, NT 508- 543

- Sequenz des humanen TNF Modul2 (extrazelluläre Domäne, AA 79-181, des natürlichen, humanen TNF Moleküls, Sequenz mit optimierter E.coli Codonverwendung): AA 182-335, NT 544-1005

- Sequenz des (GGGS)$_3$- Linker2: AA 336- 347, NT 1006- 1041

- Sequenz des humanen TNF Modul3 (extrazelluläre Domäne, AA 79-181, des natürlichen, humanen TNF Moleküls, Sequenz mit optimierter E.coli Codonverwendung): AA 348-501, NT 1042-1503

- Stopcodon: NT 1504-1506

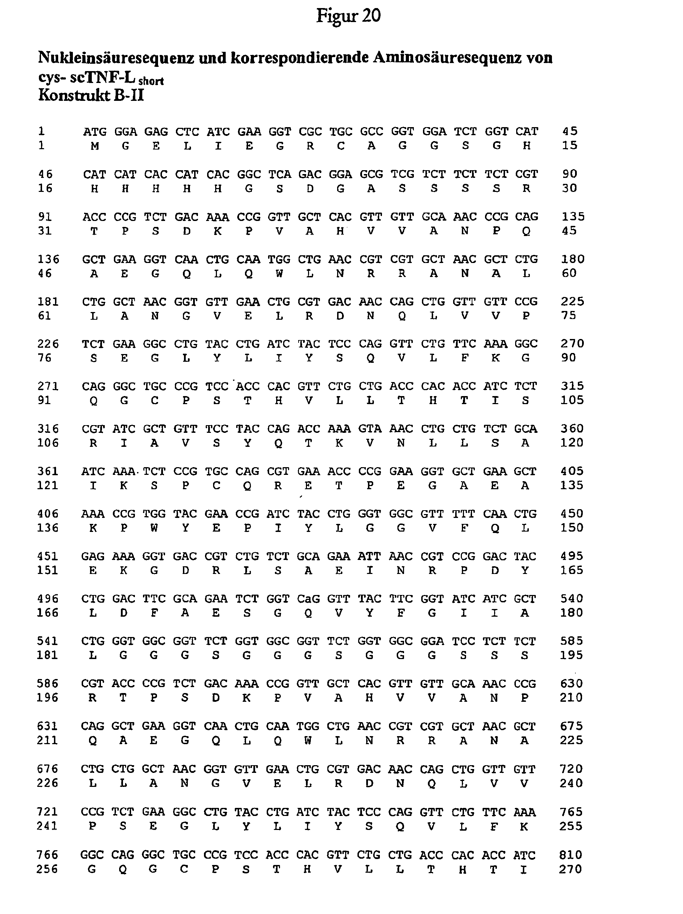

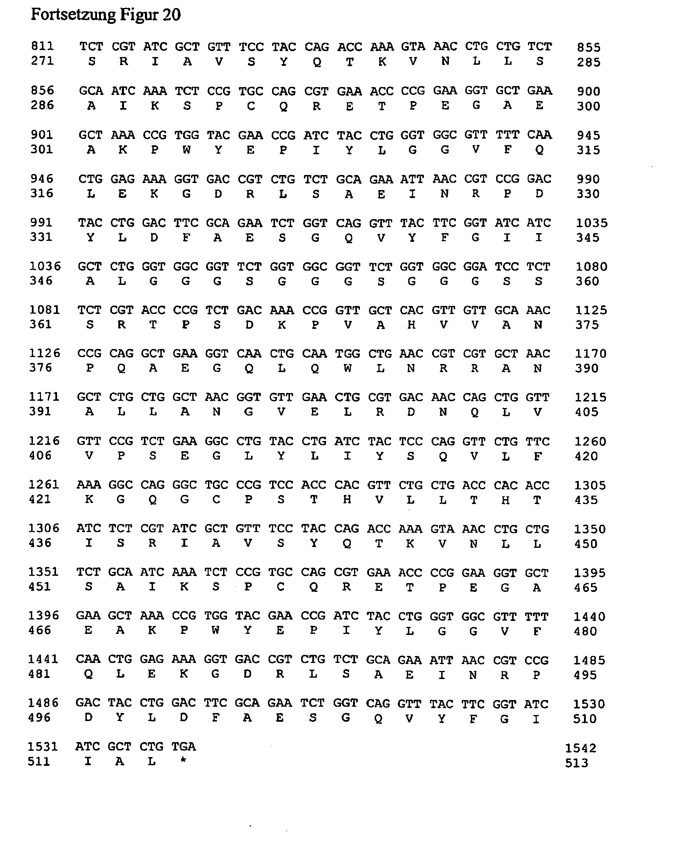

**Figur 20** zeigt die Nukleinsäuresequenz und die korrespondierende Aminosäuresequenz von cys- scTNF-L$_{short}$ (Konstrukt B-II)

Merkmale Konstrukt B-II

- Aminosäure Cystein für kovalente Kopplung: AA 9, NT 25-27

- His-Tag Peptidsequenz zur Affinitätsreinigung des gebildeten Proteins: AA 15-20, NT 43-60

- Sequenz des humanen TNF Modul (extrazelluläre Domäne des natürlichen, humanen TNF Moleküls, Sequenz mit optimierter E.coli Codonverwendung): AA 21-181, NT 61-543

- Sequenz des (GGGS)$_3$- Linker1: AA 182- 193, NT 544- 579

- Sequenz des humanen TNF Modul2 (extrazelluläre Domäne des natürlichen, humanen TNF Moleküls, Sequenz mit optimierter E.coli Codonverwendung): AA 194-347, NT 580-1041

- Sequenz des (GGGS)$_3$- Linker2: AA 348- 359, NT 1042- 1077

- Sequenz des humanen TNF Modul3 (extrazelluläre Domäne des natürlichen, humanen TNF Moleküls, Sequenz mit optimierter E.coli Codonverwendung): AA 360-513, NT 1078-1539

- Stopcodon: NT 1540-1542

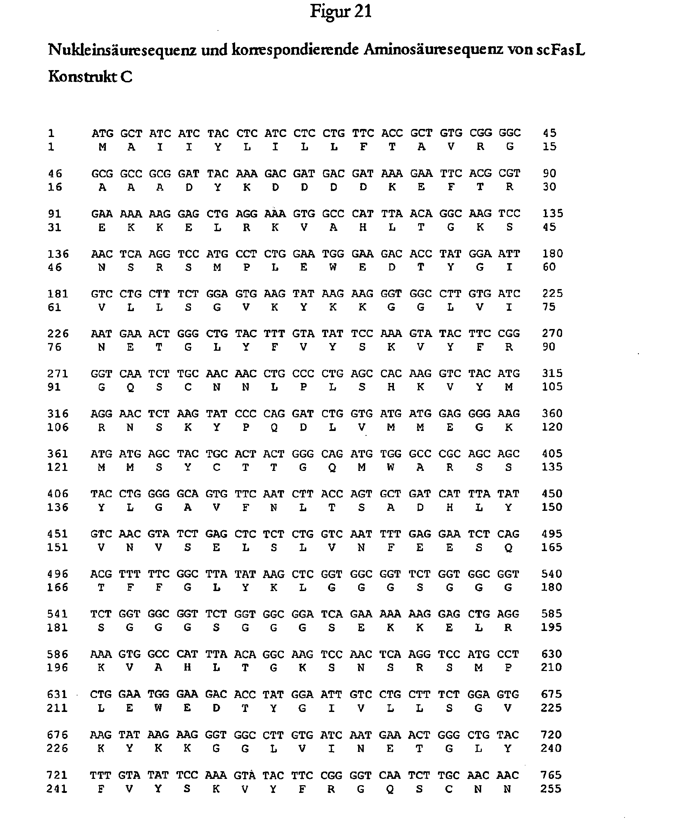

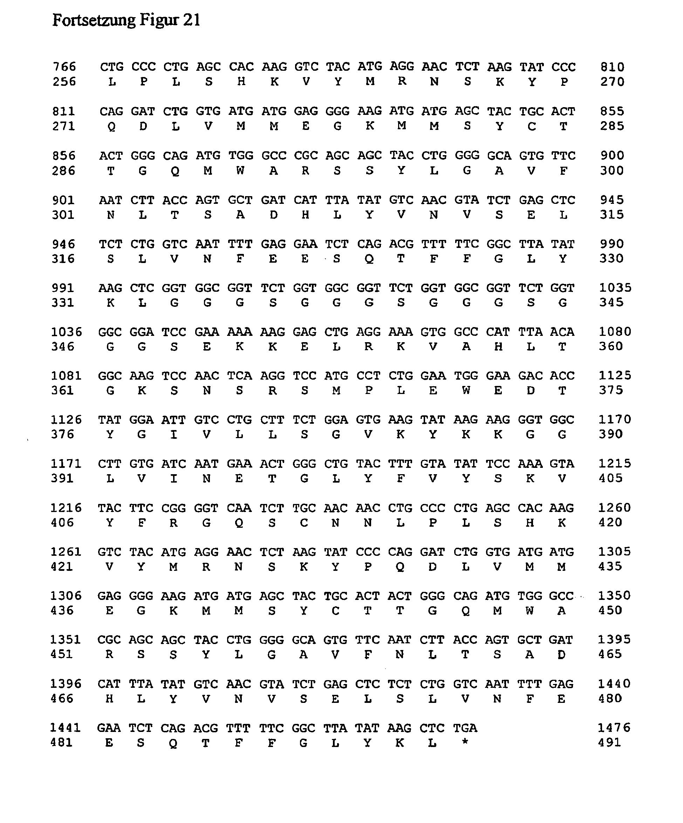

**Figur 21** zeigt die Nukleinsäuresequenz und die korrespondierende Aminosäuresequenz von scFasL (Konstrukt C)

Merkmale Konstrukt C

- Leitpeptidsequenz für Sekretion des Proteins in eukaryontischen Zellen: AA1-15, NT 1-45

- Flag Tag Peptidsequenz zur Affinitätsreinigung des gebildeten Proteins: AA 19-26, NT 55-78

- Sequenz des humanen FasL Modul1 (extrazelluläre Domäne AA 139-281 des natürlichen, humanen FasL Moleküls): AA 30-173, NT, 90-519

- Sequenz des (GGGS)$_4$- Linker1: AA 174- 189, NT 520- 567

- Sequenz des humanen FasL Modul2 (extrazelluläre Domäne AA 139-281 des natürlichen, humanen FasL Moleküls): AA 190- 332, NT 568-996

- Sequenz des (GGGS)$_4$- Linker2: AA 333- 348, NT 997- 1044

- Sequenz des humanen FasL Modul3 (extrazelluläre Domäne AA 139-281 des natürlichen, humanen FasL Moleküls): AA 349-491, NT 1045-1473

- Stopcodon: NT 1474-1476

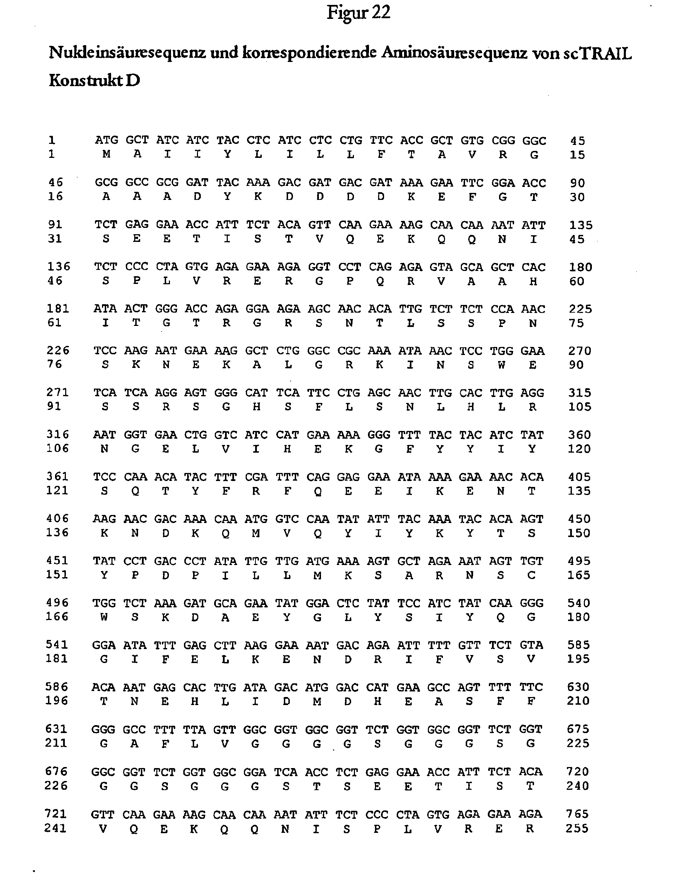

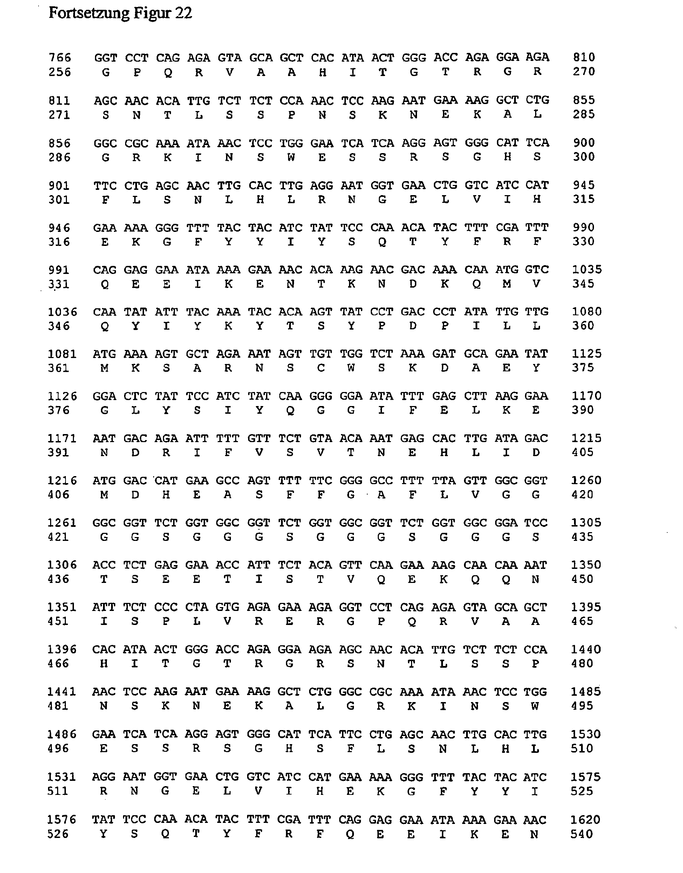

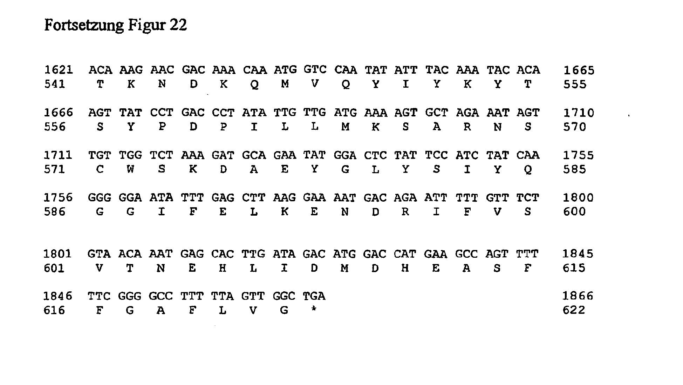

**Figur 22** zeigt die Nukleinsäuresequenz und die korrespondierende Aminosäuresequenz von scTRAIL (Konstrukt D)

Merkmale Konstrukt D

- Leitpeptidsequenz für Sekretion des Proteins in eukaryontischen Zellen: AA 1-15, NT 1-45

- Flag Tag Peptidsequenz zur Affinitätsreinigung des gebildeten Proteins: AA 19-26, NT 55-78

- Sequenz des humanen TRAIL Modul1 (extrazelluläre Domäne AA95-281 des natürlichen, humanen TRAIL Moleküls): AA 30-216, NT 88-648

- Sequenz des $(GGGS)_4$- Linker1: AA 217- 232, NT 649- 696

- Sequenz des humanen TRAIL Modul2 (extrazelluläre Domäne AA95-281 des natürlichen, humanen TRAIL Moleküls): AA 233-419, NT 697-1257

- Sequenz des $(GGGS)_4$- Linker2: AA 420- 435, NT 1258- 1305

- Sequenz des humanen TRAIL Modul3 (extrazelluläre Domäne AA95-281 des natürlichen, humanen TRAIL Moleküls): AA 436-622, NT 1306-1866

- Stopcodon: NT 1861-1863

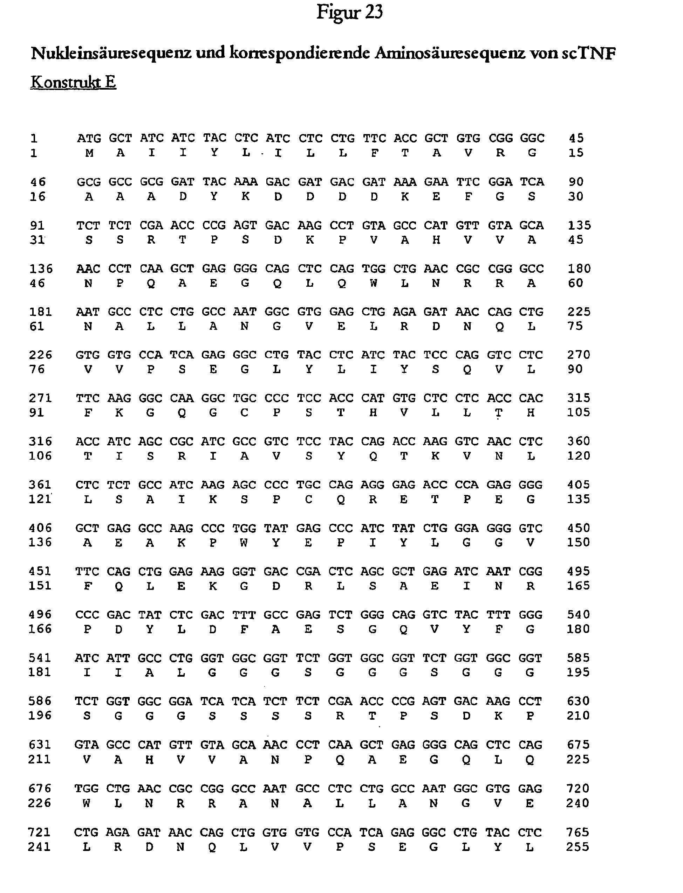

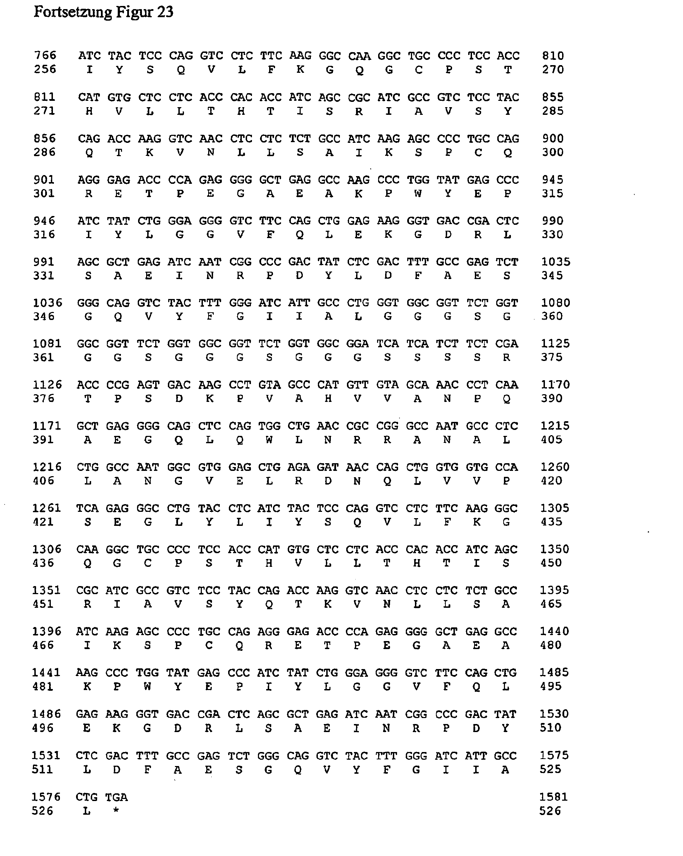

**Figur 23** zeigt die Nukleinsäuresequenz und die korrespondierende Aminosäuresequenz von scTNF (Konstrukt E)

Merkmale Konstrukt E

- Leitpeptidsequenz für Sekretion des Proteins in eukaryontischen Zellen: AA 1-15, NT 1-45

- Flag Tag Peptidsequenz zur Affinitätsreinigung des gebildeten Proteins:: AA 19-26, NT 55-78

- Sequenz des humanen TNF Modul1 (extrazelluläre Domäne des natürlichen, humanen TNF Moleküls): AA30-184 , NT 88-552

- Sequenz des $(GGGS)_4$- Linker1: AA 85- 200, NT 553- 600

- Sequenz des humanen TNF Modul2 (extrazelluläre Domäne des natürlichen, humanen TNF Moleküls): AA 201-355, NT 601-1065

- Sequenz des $(GGGS)_4$- Linker2: AA 356- 371, NT 1066- 1113

- Sequenz des humanen TNF Modul3 (extrazelluläre Domäne des natürlichen, humanen TNF Moleküls): AA 372-526, NT 1114-1581

- Stopcodon: NT 1799-1581

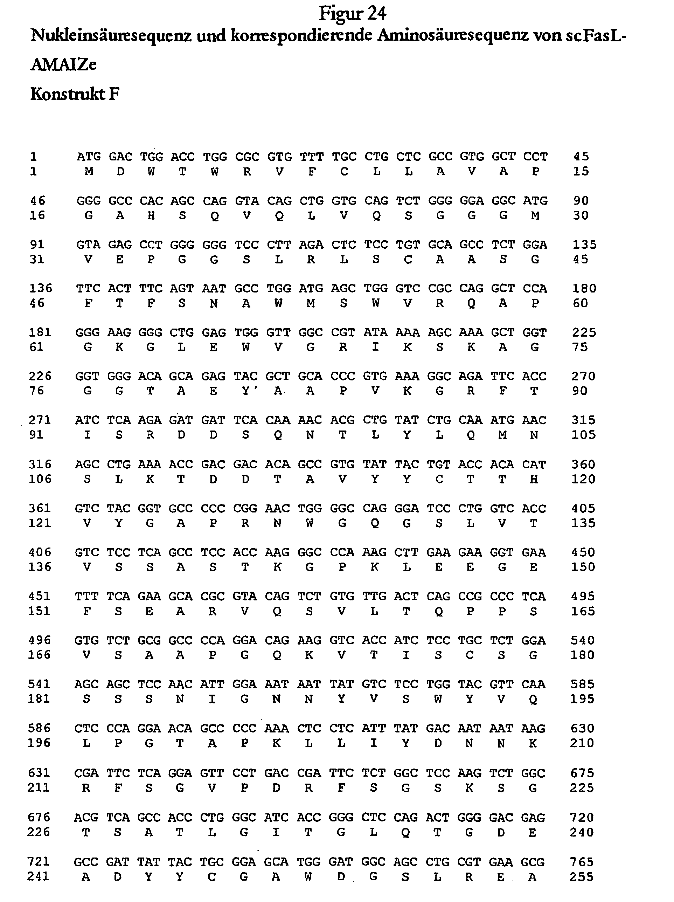

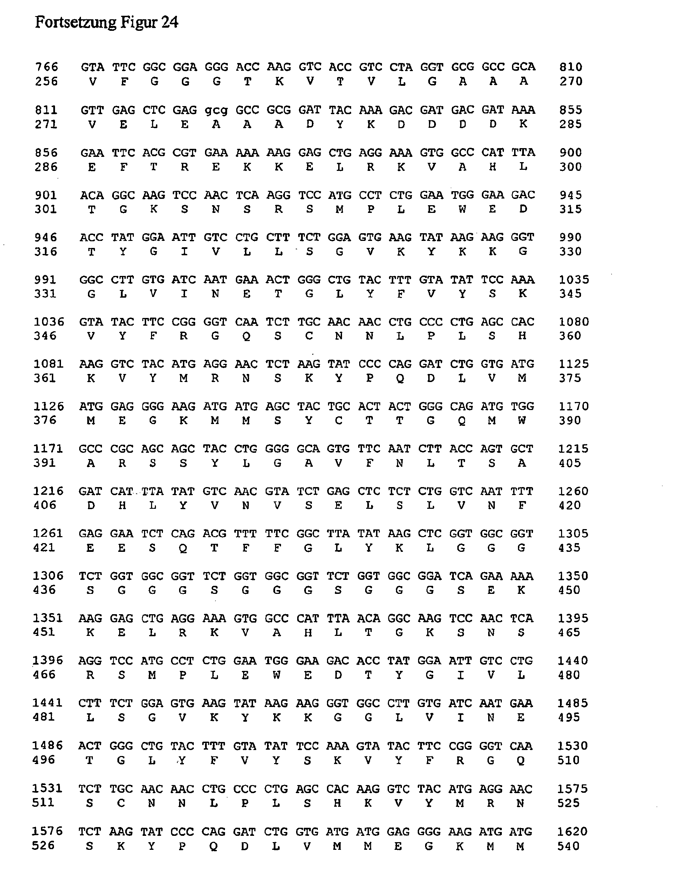

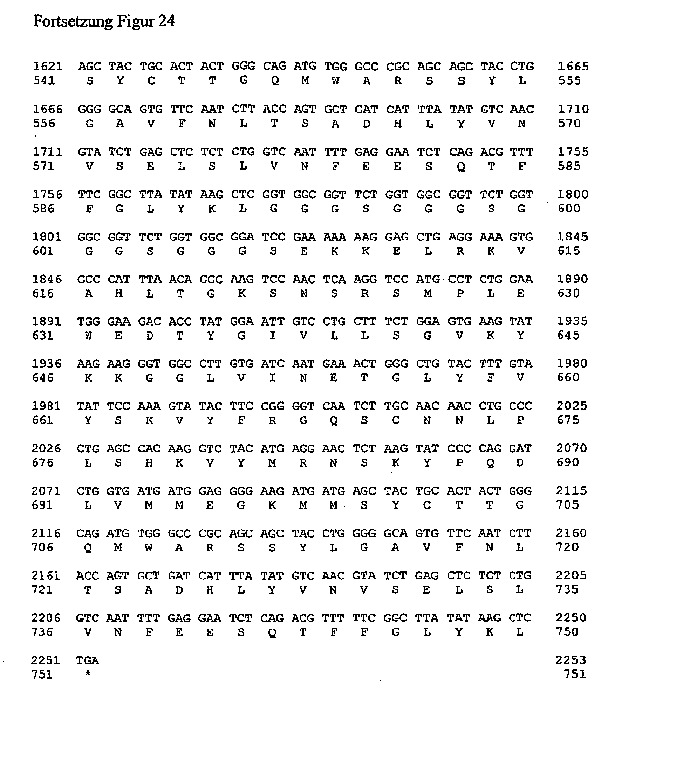

**Figur 24** zeigt die Nukleinsäuresequenz und die korrespondierende Aminosäuresequenz von scFasL-AMAIZe (Konstrukt F)

Merkmale Konstrukt F

- Leitpeptidsequenz für Sekretion des Proteins in eukaryontischen Zellen: AA 1-19, NT 1-57

- Sequenz des Einzelketten (scFv)- Antikörperfragmentes 40 spezifisch für das Tumorstroma- Antigen FAP: AA 20-267, NT 58- 801

- Flag Tag Peptidsequenz zur Affinitätsreinigung des gebildeten Proteins: AA278-285, NT 832-855

- Sequenz des humanen FasL Modul1 (extrazelluläre Domäne, AA 139 -281, des natürlichen, humanen FasL Moleküls): AA 290-432, NT 868-1296

- Sequenz des $(GGGS)_4$- Linker1: AA 433- 448, NT 1297- 1344

- Sequenz des humanen FasL Modul2 (extrazelluläre Domäne, AA 139-281, des natürlichen, humanen FasL Moleküls): AA 449-591, NT 1345-1773

- Sequenz des (GGGS) 4- Linker2: AA 592- 607, NT 1774- 1821

- Sequenz des humanen FasL Modul3 (extrazelluläre Domäne, AA 139-281, des natürlichen, humanen FasL Moleküls): AA 608-750, NT 1822-2250

- Stopcodon: NT 2251-2253

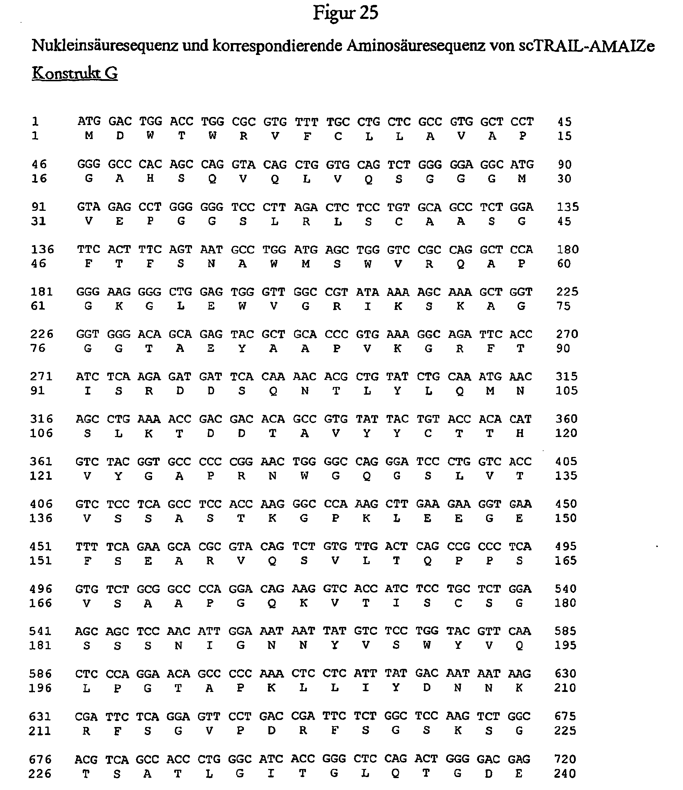

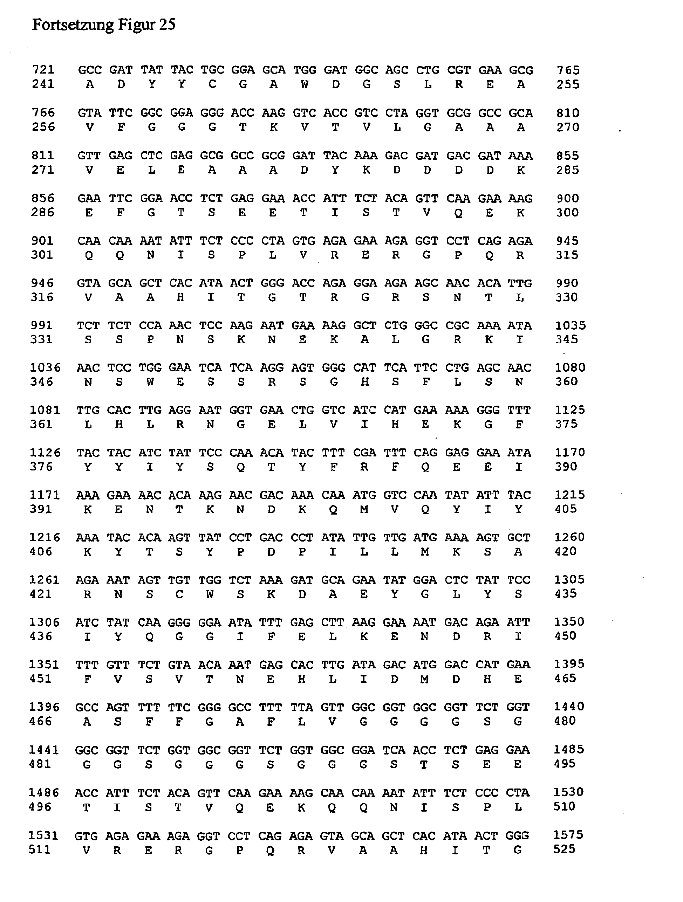

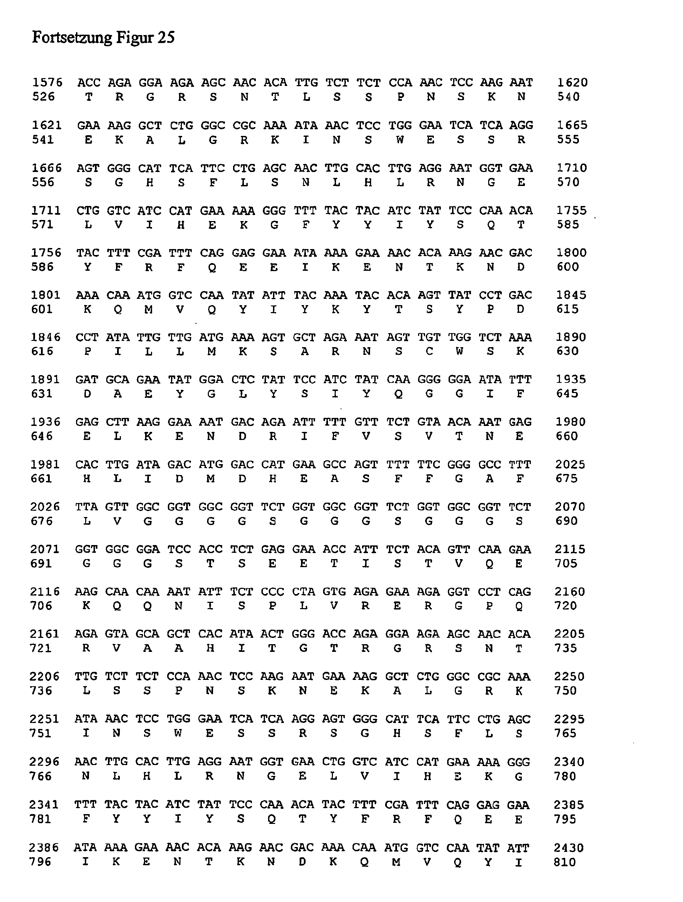

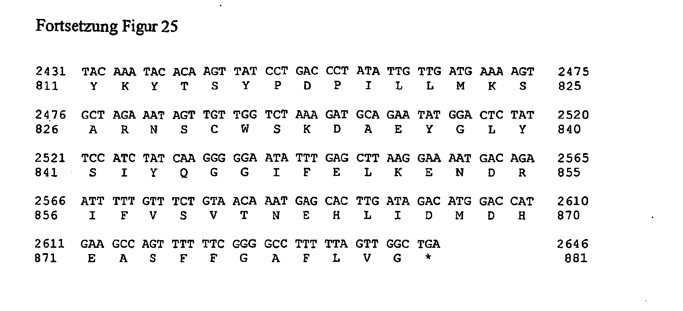

**Figur 25** zeigt die Nukleinsäuresequenz und die korrespondierende Aminosäuresequenz von scTRAIL-AMAIZe (Konstrukt G)

Merkmale Konstrukt G

- Leitpeptidsequenz für Sekretion des Proteins in eukaryontischen Zellen: AA 1-19, NT 1-57

- Sequenz des Einzelketten (scPv)- Antikörperfragmentes 40 spezifisch für das Tumorstroma- Antigen FAP: AA 20-267, NT 58- 801

- Flag Tag Peptidsequenz zur Affinitätsreinigung des gebildeten Proteins: AA278-285, NT 832-855

- Sequenz des humanen TRAIL Modul1 (extrazelluläre Domäne, AA95-281, des natürlichen, humanen TRAIL Moleküls): AA 289-475, NT 865-1426

- Sequenz des $(GGGS)_4$- Linker1: AA 476- 491, NT 1427- 1476

- Sequenz des humanen TRAIL Modul2 (extrazelluläre Domäne, AA95-281, des natürlichen, humanen TRAIL Moleküls): AA 492-678, NT 1477-2034

- Sequenz des (GGGS) 4- Linker2: AA 679- 694, NT 2035- 208

- Sequenz des humanen TRAIL Modul3 (extrazelluläre Domäne, AA95-281, des natürlichen, humanen TRAIL Moleküls): AA 695-, NT 2083-2643

- Stopcodon: NT 2644-2646

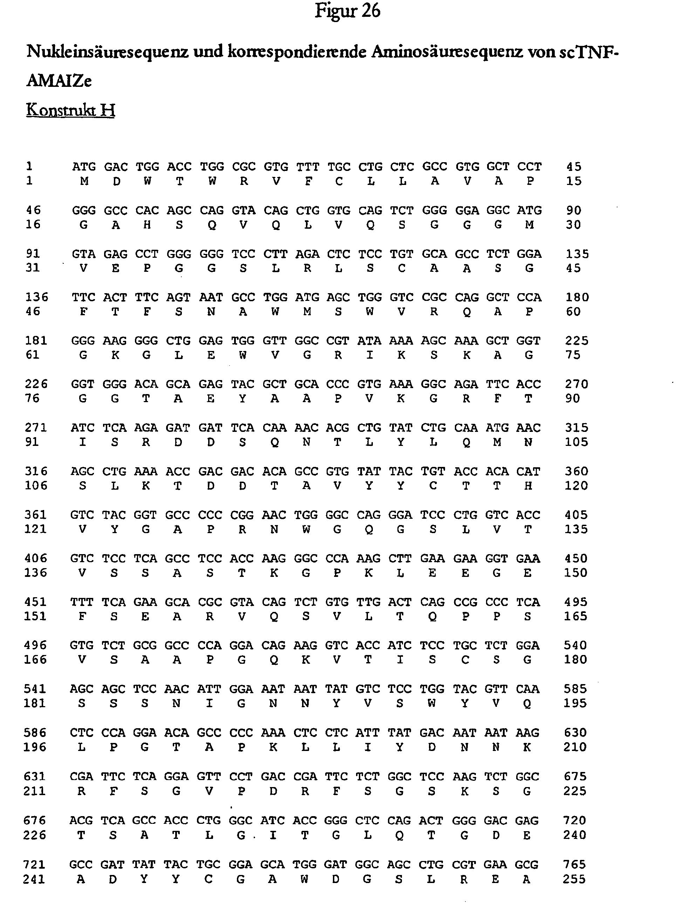

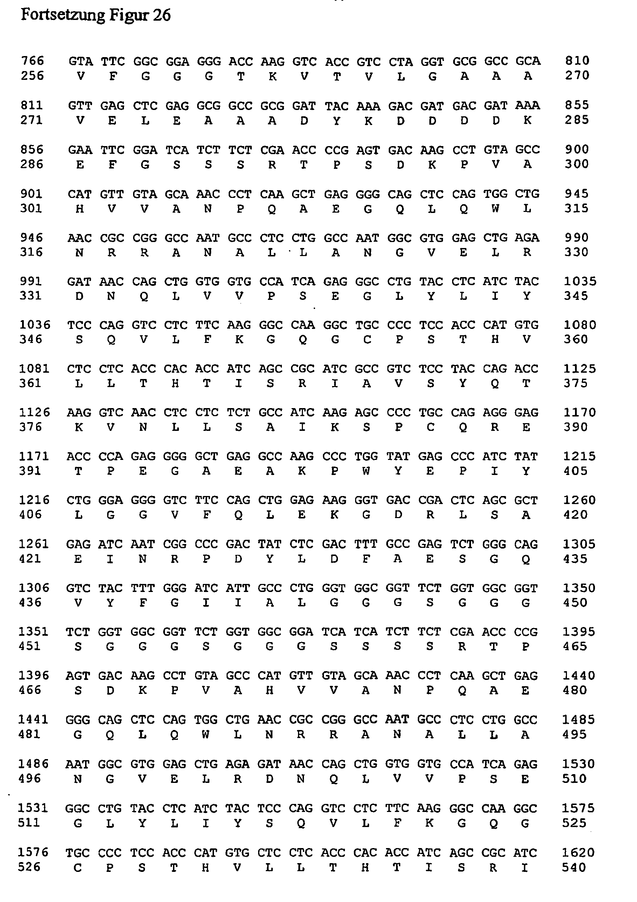

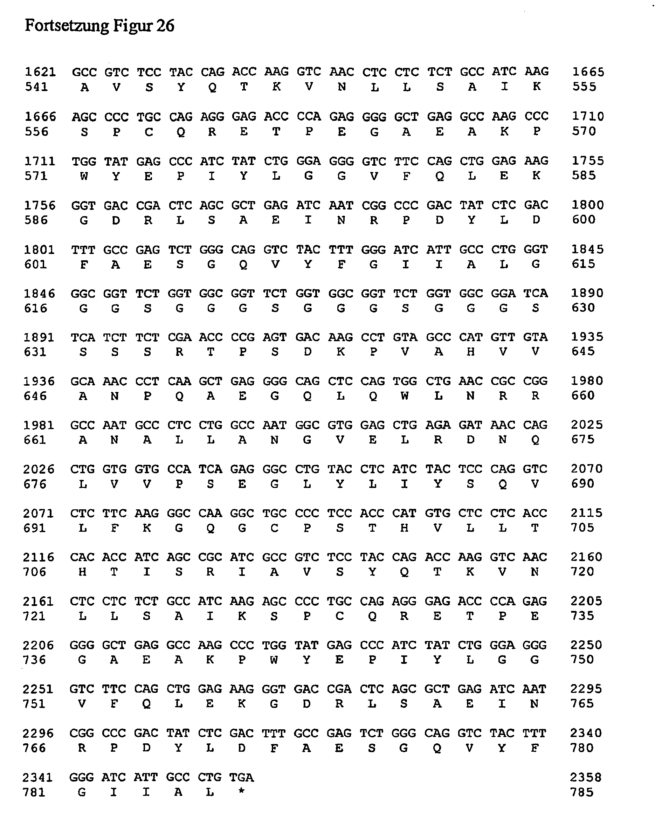

**Figur 26** zeigt die Nukleinsäuresequenz und die korrespondierende Aminosäuresequenz von scTNF-AMAIZe (Konstrukt H)

Merkmale Konstrukt H

- Leitpeptidsequenz für Sekretion des Proteins in eukaryontischen Zellen: AA 1-19, NT 1-57

- Sequenz des Einzelketten (scFv)- Antikörperfragmentes 40 spezifisch für das Tumorstroma- Antigen FAP: AA 20-267, NT 58- 801

- Flag Tag Peptidsequenz zur Affinitätsreinigung des gebildeten Proteins:: AA 278-285, NT 832-855

- Sequenz des humanen TNF Modul1 (extrazelluläre Domäne, AA 79-181, des natürlichen, humanen TNF Moleküls): AA 289-443, NT 865-1329

- Sequenz des $(GGGS)_4$- Linker1: AA 444-  , NT 1330- 1377

- Sequenz des humanen TNF Modul2 (extrazelluläre Domäne, AA 79-181, des natürlichen, humanen TNF Moleküls): AA 460-614, NT 1378-1842

- Sequenz des $(GGGS)_4$- Linker2: AA 615- 630, NT 1843- 1890

- Sequenz des humanen TNF Modul3 (extrazelluläre Domäne, AA 79-181, des natürlichen, humanen TNF Moleküls): AA 631-785, NT 1891-2353

- Stopcodon: NT 2356-2358

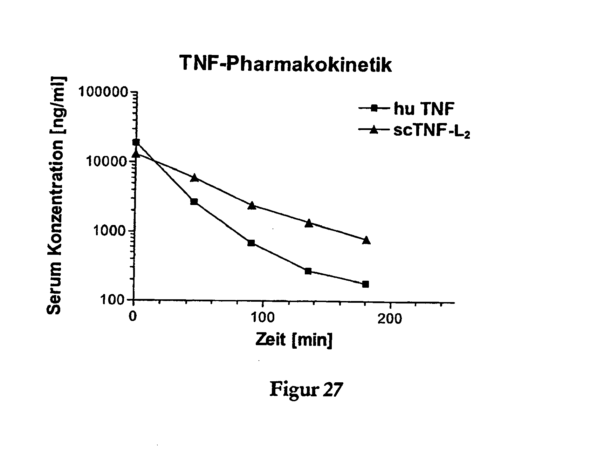

**Figur 27** zeigt die Pharmakokinetik von humanem Wildtyp-TNF und humanem scTNF (vgL Beispiel 2). Die Daten in Figur 27 zeigen einen klaren Anstieg der *in vivo* Halbwertszeit der scTNF Varianten. Es wird für scTNF damit gegenüber TNF eine deutlich erhöhte Wirkungsdauer  in vivo erwartet, was damit den Wert von scTNF, insbesondere von scTNF-$L_2$, als potentielles Therapeutikum unterstreicht.

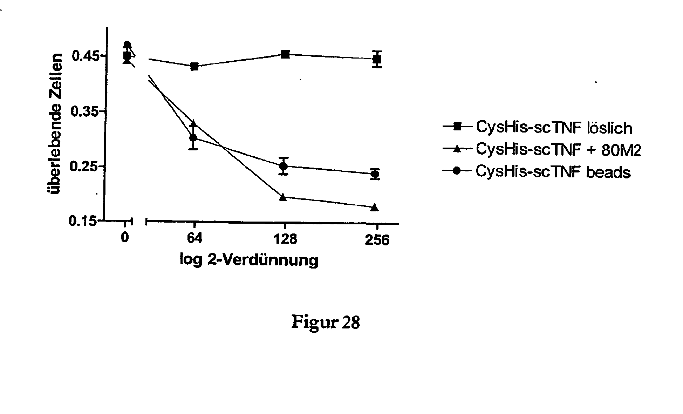

**Figur 28** zeigt kovalent an Partikel (Silica) gekoppeltes CysHis-scTNF. Dieses kovalent gekoppelte CysHis-scTNF ist bioaktiv und besitzt die besondere Aktivität von membrangebundenem TNF, i.e. es aktiviert TNFR2. Figur 28 zeigt , daß Zellen aus Mausfibroblasten, die mit dem Konstrukt TNFR2-Fas transfiziert sind und mit seriellen Verdünnungen der angegebenen Reagenzien behandelt wurden, völlig resistent gegen lösliches wtTNF sind. Nach kovalenter Kopplung von reduziertem CysHis-scTNF an Silica- Micropartikel (beads) nach etablierten Protokollen (DPA 2001, Nr. DE10144252) bewirken diese eine starke zytotoxische Antwort (Kreis), ähnlich wie eine Positivkontrolle bestehend aus CysHis-scTNF und einem TNFR2 vernetzenden Antikörper, mAk 80M2 (Dreieck). Nicht gekoppletes Cys-His scTNF zeigt erwartungsgemäß keine Aktivität auf TNFR2 positiven Zellen (Quadrate).

Die vorliegende Erfindung wird nachfolgend durch die Beispiele illustriert:

**Beispiele**

**Beispiel 1:** Herstellung verschiedener Polypeptid-Konstrukte

Alle Klonierungen wurden nach Standardprotokollen durchgeführt. Im Folgenden werden die Bedingungen dieser aufgeführt.

Standard-PCR;

60 ng Template, 0,5 $\mu$l 100 $\mu$M Primer, 1 $\mu$l 10 mM dNTPs sowie 5$\mu$l 10x Puffer und 2 U Taq-Polymerase wurden in einem Reaktionsvolumen von 50 $\mu$l mit folgendem PCR-Programm amplifiziert. Denaturierung: 94°C 3 min; 15 Zyklen: Denaturierung 94°C 30s, Annealing 55°C 30s, Elongation 72°C 90s; finale Elongation: 72°C 7 min.

Verdau von PCR Produkten:

Das PCR-Produkt wurde über ein Agarose Gel aufgereinigt und eluiert und nachfolgend mit den jeweiligen Restriktionsenzymen (s. genaue Angaben) in einem 40 $\mu$l Reationsansatz bei optimaler Spalttemperatur (wird vom Hersteller angeben) für 2 Stunden verdaut.

Verdau von Vektoren:

1$\mu$g Vektor wurde mit 5U der entsprechenden Restriktionsenzyme in einem 20 $\mu$l Reaktionvolumen für 2

Stunden bei optimaler Spalttemperatur (diese ist abhängig vom verwendeten Enzym und wird vom Hersteller angegeben) verdaut Zur Dephosphorylierung von Vektoren wurde 10 U Alkalische Phosphatase für 1 Stunde zu dem Reaktionsverdau gegeben.

"Fill in"-Reaktion:

Der Ansatz eines Vektorverdaus wurde mit 33 $\mu$M dNTPs und 1U/1$\mu$g DNA Klenow-Fragment der DNA-Polymaerase I versetzt und 15 min bei 25°C inkubiert. Diese Reaktion wurde mit 10 mM EDTA für 20 min bei 75°C gestoppt

Ligation:

Vektor und Insert wurden in einem molaren Verhältnis von 1:5 zusammen mit 400 U Ligase in einem 10 $\mu$l Volumen über Nacht bei 16°C ligiert

I. Herstellung von scTNF$_{human}$ (scTNF) mit vier- oder dreifach Linken:

Es wurden zwei scTNF Varianten hergestellt, die sich durch die Länge des Peptid- Linkers zwischen den einzelnen Modulen unterscheiden. Es wurden Primer mit 4fach bzw. 3fach Peptid- Linker- Sequenz eingesetzt Linker$_{long}$ mit (GGGS)$_4$- oder Linker$_{short}$ mit (GGGS)$_3$- Sequenz. Die erzeugten Konstrukte enthielten demnach entweder nur zwei 4fach Linker (L1 oder L2 mit (GGGS)$_4$- als "long" bezeichnet) oder nur zwei 3- fach Linker (L1 oder L2 mit (GGGS)$_3$- mit "short" bezeichnet) .

1. Mit den Primern V und I bzw. II (für Linker$_{long}$) und dem pQE- 9 Vektor mit einem TNF Modul (pQE9- HisTNF) als Template wurde eine Standard- PCR durchgerührt. Der Vektor trägt eine His Tag Sequenz zur späteren Affnitätsaufreinigung des produzierten Proteins.

2. Das erhaltene PCR-Produkt I wurde nachfolgend mit 20 U der jeweiligen Restriktionsenzyme StuI und HindIII bei 37°C verdaut Der gleiche Verdau sowie eine Dephosphorylierungsreaktion wurde mit dem pQE9-HisTNF Vektor durchgeführt und durch eine Ligation das PCR-Produkt I in den pQE9-HisTNF Vektor eingebracht. Ergebnis dieses Schrittes war ein His Tag - TNF Modull mit einem Linker1$_{short bzw. long}$. bzw. folgendes bzw. folgendes Konstrukt im pQE9-Vektor:
**EcoRI- His Tag - TNF Modul1-Linker1$_{short bzw. long}$ - BamHI**

3. Über eine weitere PCR mit den Primern III und I bzw. II (für Linker$_{long}$) und dem pQE9-HisTNF Vektor als Template wurde das PCR-Produkt II erzeugt. Das PCR-Produkt II wurde nachfolgend mit jeweils 20 U der Restriktionsenzyme EcoRI und HindIII geschnitten und in den pQE9-HisTNF Vektor, der mit den gleichen Enzymen geschnitten und dephosphoryliert wurde, eingebracht Ergebnis dieser Klonierung war ein pQE9-Vektor, der wie folgt aussah:
**TNF Modul2-Linker2$_{short bzw. long}$ - BamHI**
Dieses Konstrukt trägt keine Sequenz für einen His-Tag vor der TNF Sequenz.

4. Das klonierte PCR- Produkt II aus Schritt 3 wurde zunächst mit dem Restriktionsenzym Hind III und nachfolgend partiell mit BamHI (1 U/ $\mu$g DNA) aus dem Vektor herausgeschnitten Der pQE9- His Tag TNF Modull-Linker1$_{short bzw. long}$ Vektor wurde ebenfalls mit den Restriktionsenzymen BamHI und HindIII sequentiell geschnitten, dephosphoryliert und das PCR- Produkt II in diesen Vektor ligiert. Ergebnis war ein pQE9- Vektor mit folgendem Konstrukt:
**His Tag- TNF Modul1- Linker1$_{short bzw. long}$- TNF Modul2- Linker2$_{short bzw. long}$.**

5. Mit dem pQE9-HisTNF-Vektor als Template und den Primern III und IV wurde eine weitere PCR unter Standardbedingungen durchgeführt und das erhaltene PCR-Produkt III mit den Restriktionsenzymen BamHI (40U) und HindIII (40U) sequentiell verdaut Dieses Fragment wurde dann in einen pBluescript SKII Vektor, der ebenfalls mit den Re-striktionsenzymen BamHI und HindIII geschnitten wurde, ligiert. Ergebnis dieser Klo-nierung war ein pBluescript SKII Vektor, der das TNF Modul 3 ohne Linker enthält.

6. Der pQE9- Vektor mit dem **His Tag - TNF Modul1 -Linker1$_{short bzw. long}$ -TNF Mo-dul2 -Linker2$_{short bzw. long}$** Konstrukt wurde mit dem Restriktionsenzym EcoRI geschnitten, anschließend wurde dieser Vektor mit dem Klenow-Fragment der DNA-Polymerase I aus E. coli behandelt, um ein "fill in" vorzunehmen. Nach diesem Schritt wurde ein partieller Restriktionsverdau mit dem Enzym BamHI (1 U/$\mu$g DNA) durchgeführt.

7. Parallel zum Schritt 6. wurde der pBluescrpt SKII Vektor, der das TNF Modul 3 enthält, mit dem Restriktionsenzym XbaI geschnitten, anschließend wurde dieser Vektor mit dem Klenow-Fragment der DNA-Polymerase I aus E. coli behandelt, um ein "fill in" vorzunehmen. Nach diesem Schritt wurde ein zweiter Restriktionsverdau mit dem Enzym BamHI unter Standardbedingungenmit zusätzlicher Dephosphorylierung durchgeführt.

8. Das durch den Restriktionsverdau erhaltene Fragment aus Schritt 6. wurde nachfolgend in den im Schritt 7 generierten linearem Vektor ligiert Die Konstrukte lagen in reverser Anordnung wie folgt gezeigt vor:
HindIII- TNF Modul3- Linker2$_{short bzw. long}$- TNF Modul2- Linker$_{short bzw. long}$- TNF Modul1- His Tag- EcoRI

9. Das reverse liegende TNF- Konstrukt aus Schritt 8. wurde mit den Restriktionsenzymen EcoRI und HindIII aus dem pBluescript SKII Vektor herausgeschnitten und in dem mit den gleichen Enzymen behandelten sowie dephosphorylierten pQE9- HisTNF Vektor ligiert. Hierdurch entstand folgendes Konstrukt mit dem vollständigen scTNF in korrekter Orientierung:
**EcoRI- His Tag- TNF Modul1- Linker1$_{short bzw. long}$- TNF Modul2- Linker2$_{short bzw. long}$- TNF Modul3- HindIII**

10. Zur Herstellung eines scTNF mit einem N- terminalen Cystein wurden die Oligos cys- scTNF VI und VII annealed (je 20$\mu$l 100$\mu$M Oligo VI bzw VII wurden zusammen für 5 min bei 95°C erhitzt und langsam auf Raumtemperatur abkühlen lassen) und so das Oligo1 gebildet. Es wurde das Konstrukt aus Punkt 9 mit den Restriktionsenzymen EcoRI und BbsI verdaut und das Oligo1, welches über die gleichen Schnittstellen verfügt, in den Vektor ligiert . Alternativ wurde das Cystein über PCR- Mutagenese eingefügt.
Ergebnis dieser Klonierung war folgendes Konstrukt:
**EcoRI- Cystein- His Tag- TNF Modul1- Linker1$_{short bzw. long}$- TNF Modul2- Linker2$_{short bzw. long}$- TNF Modul3- HindIII**

Alle Konstrukte wurden mittels Sequenzierung verifiziert.

Die Expression erfolgte im *E.coli* Stamm XL-1 blue. Die Aufreinigung der exprimierten scTNF Varianten erfolgte mit Hilfe chromatographischer Methoden (His Tag Affinitäs- und Anionenaustauscherchromatographie).

Nachfolgend werden die Sequenzen der verwendeten Primer angegeben:

Peptid-Linker Sequenzen auf Proteinebene

3fach GGGS-Linker (short) = (GGGS)$_3$: GGGS GGGS GGGS
4fach GGGS-Linker (long) = (GGGS)$_4$: GGGS GGGS GGGS GGGS

Peptid-Linker Sequenzen auf Nukleotidebene

3fach GGGS-Linker (short): 5´GGT GGC GGT TCT GGT GGC GGT TCT GGT GGC GGA TCC3´

4fach GGGS-Linker (long): 5´GGT GGC GGT TCT GGT GGC GGT TCT GGT GGC GGT TCT GGT GGC GGA TCC3`

Primer für die Klonierungen

**scTNF Primer I**

5'- TCG ATT AAG CTT CCC GGG GGA TCC GCC ACC AGA ACC GCC ACC AGA ACC GCC ACC CAG AGC GAT GAT ACC GAA GTA AAC CTG ACC -3'

**scTNF Primer II**

5'- ATC GAT TAA GCT TCC CGG GGG ATC CGC CAC CAG AAC CGC CAC CAG AAC CGC CAC CAG AAC CGC CAC CCA GAG CGA TGA TAC CGA AGT AAA CCT GAC C -3'

**scTNF Primer III**
5'- CCC CGA ATT CGG ATC CTC TTC TCG TAC CCC GTC TGA CAA ACC G -3'

**scTNF Primer IV**

5'- GGG GGG GAA GCT TAT CGA TAG TTA GAT ATC ATC ACA GAG CGA TGA TAC CGA AG -3'

**scTNF Primer V**
5'- CCT GTA CCT GAT CTA CTC CCA GGT TCT GTT CAA AGG CCA GG -3'

Oligo für CysHis-Insertion :

cys-scTNF Primer VI

AAT TCA TTA AAG AGG AGA AAT TAA CTA TGG GAG AGC TCA TCG AAG GTC GCT GCG CCG GTG GAT CTG GTC ATC ATC ATC ACC ATC ACG GCT CAG ACG G

cys-scTNF Primer VII

CGC TCC GTC TGA GCC GTG ATG GTG ATG ATG ATG ACC AGA TCC ACC GGC GCA GCG ACC TTC GAT GAG CTC TCC CAT AGT TAA TTT CTC CTC TTT AAT G

II. Herstellung von scFasL in pcDNA3 und AMAIZe-Konstrukte:

Für nachfolgende Klonierungen wurden die unter Beispiel 1 angegebenen Standardbedingungen verwendet

A. Generierung vom HA-Signal in pcDNA3

1. Verdau des pcDNA3-Vektors mit KpnI und NotI.

2. Herstellung des HA- Oligo mit KpnI- HA- Signal- NotI- Sequenz über Annealing der Primer HA- IF und HA- IIR für Leitpetidsequenz:

3. Ligation des HA-Oligos (enthält KpnI und NotI- Schnittstelle) in den pcDNA3-Vektor

B. Herstellung des scFasL im pcDNA3(+)- Vektor

1. PCR mit Primer FasL#1F und FasL#2R auf Template FasL- AMAIZE- Vektor. Die Herstellung dieser Art von Konstrukten werden in der Patenanmeldung DE 10045591.3 beschrieben, die hiermit vollinhaltlich zum Offenbarungsgehalt der vorliegenden Erfindung gemacht wird. Produkt dieser PCR 1 war ein **NotI- Flag Tag- FasL Modul1- Linker1- BamHI- Xbal** Konstrukt.

2. Über die NotI und XbaI Restriktionsschnittstelle wurde dieses Konstrukt in den mit den gleichen Enzymen verdauten pcDNA3-HA Sequenz Vektor kloniert, so dass folgendes Konstrukt entsteht:

**HA Sequenz - Flag Tag - FasL Modul1 -Linker1 - BamHI - XbaI**

3. Mit Hilfe einer weiteren PCR auf dem Template FasL-AMAIZE Vektor und den Primern FasL#3 und FasL#1 wurde folgendes PCR-Produkt 2 generiert

**blunt end- FasL Modul2 - Linker2 - BamHI - XbaI**

4. Im nächsten Schritt wurde der pcDNA3 Vektor aus Punkt 2 mit BamHI verdaut, diese Schnittstelle mit dem Klenow Enzym aufgefüllt und nachfolgend mit XbaI geschnitten, so dass ein "blund end" und ein "sticky end" entstand. In diesen so modifizierten Vektor wurde anschließend das PCR-Produkt 2 kloniert, wodurch folgendes Konstrukt gebildet wurde:

**HA Sequenz - Flag Tag - FasL Modul1 - Linker1-FasL Modul2- Linker2- BamHI - XbaI**

5. Für das dritte Modul wurde eine weitere PCR mit dem Template FasL-AMAIZE Vektor und den Primern FasL#4 und FasL#5 durchgeführt. Das gebildete PCR-Produkt wurde anschließend mit den Restriktionsenzymen BamHI und XbaI verdaut und in den mit den gleichen Enzymen geschnitten Vektor aus Punkt 4 ligiert. Resultat dieser Klonierung war folgendes Konstruk im pcDNA3 Vektor:

**HA Sequenz - NotI - Flag Tag - FasL Modul1 - Linker1-FasL Modul2- Linker2-FasL Modul3 - stop -XbaI.**

Für die Herstellung der scFasL- bzw. scTNF-AMAIZe Konstrukte wurden die jeweiligen scFasL bzw. scTNF mit den Restriktionsenzymen NotI bzw. EcoRI und XbaI verdaut und die Inserts als Kassette in die entsprechenden AMAIZe-Vektoren (siehe Patentanmeldung DE 10045591.3) eingefügt, wobei diese Vektoren ebenfalls mit den Enzymen NotI bzw. E-coRI und XbaI geschnitten wurden. Hierüber wurden folgende Konstrukte hergestellt:

**Leitpeptid-scFv40-Flag Tag- FasL Modul1-Linker1-FasL Modul2-Linker2-FasL Mo-dul3**

**Leitpeptid-scFv40-Flag Tag- TNF Modul1-Linker1-TNF Modul2-Linker2-TNF Modul3**

Nachfolgend werden die Sequenzen der verwendeten Primer angegeben:

FasL#1R:

5´ATCGATTTCTAGACCCGGGGGGATCCGCCACCAGAACCGCCACCAGAACCGCC
ACCAGAACCGCCACCGAGCTTATATAAGCCGAAAAACGTCTGAGATTC3´

FasL#2F:
5'GGGGTAGCGGCCGCGCTGTCGACGATTACAAAGAC3'

FasL#3F:
5'AGAAAAAA-AGGAGCTGAGGAAAGTGG3'

FasL#4F:
5'GGGGCGGATCCGAAAAAAAGGAGCTGAGGAAAGTGG3'

FasL#5R:
5'GGGGCCTCTAGAATCGATGGTCAGAGCTTATATAAGCCGAAAAACGTCTG3'

HA-IF

5´CGCCAT GGCTATCATC TACCTCATCC TCCTGTTCAC CGCTGTGCGG
GGAGC3´

HA-IIR

5′GGC CGC TGC CCC GCA CAG CGG TGA ACA GGA GGA TGA GGT AGA TGA

TAG CCA TGG CGG TAC3′

III. scTRAIL.-Klonierung und Herstellung  von scTRAIL AMAIZe Konstrukten

Für nachfolgende Klonierungen wurden die unter Beispiel 1 angegebenen Standardbedingungen verwendet

1. PCR mit Primern TRAIL#1 und TRAIL#2 auf Template pcDNA3-sc40-TRAIL (siehe Patenanmeldung DE 10045591.3). PCR-Produkt 1 wurde mit EcoRI und XbaI geschnitten  und in den mit den gleichen Restriktionsenzymen verdauten pcDNA3-scFasL Vektor ligiert. Dieser Verdau deletierte die FasL Sequenz, wobei die HA- und die Flag Tag Sequenz erhalten blieb und nun folgendes Konstrukt entstanden war:
**HA-Sequenz - Flag Tag - TRAIL Modul1 - Linker1- BamHI - XbaI**

2. Mit Hilfe der Primer TRAIL#1R und TRAIL#2F wurde mit dem Template TRAIL-AMAIZe (siehe. Patentanmeldung DE 10045591.3) das PCR-Produkt 2 generiert. Dieses wurde nur mit XbaI geschnitten, wobei ein blund-end und ein sticky-end entsteht Das Konstrukt aus Punkt 1 wurde mit BamHI verdaut und nachfolgend mit dem Klenow-Enzym behandelt, so dass die Enden aufgefüllt wurden. Im Anschluß daran wurde ein XbaI Verdau durchgeführt und das PCR-Produkt 2 in diesen Vektor kloniert Ergebnis war folgendes Konstrukt:
**HA-Sequenz - Flag Tag - TRAIL Modul1 - Linker1 - TRAIL Modul2 - Linker2 - BamHI-XbaI**

3. Für die Klonierung des TRAIL Moduls 3 wurde eine PCR mit den Primern TRAIL#4 und TRAIL#5 auf dem Template TRAIL-AMAIze durchgeführt, das Produkt nachfolgend mit BamHI und XbaI verdaut und in das Konstrukt aus Punkt 2 - ebenfalls mit BamHI und XbaI verdaut- kloniert, wodurch folgendes Konstrukt entstanden ist:
**HA-Sequenz - Flag Tag - TRAIL Modul1 - Linker1 - TRAIL Modul2 - Linker2 - TRAIL Modul3 Stop - XbaI**

Für die Herstellung der scTRAIL-AMAIZe Konstrukte wurden die jeweiligen scTRAIL Vektoren mit den Restriktionsenzymen NotI bzw. EcoRI und XbaI verdaut und die Inserts als Kassette in die entsprechenden AMAIZe-Vektoren (siehe. Patentanmeldung DE 10045591.3), wobei diese Vektoren ebenfalls mit den Enzymen NotI bzw. EcoRI und XbaI geschnitten wurden. Hierüber wurden folgende Konstrukte hergestellt:
HA-svFv40-Flag Tag- TRAIL Modul1-Linker1-TRAIL Modul2-Linker2- TRAIL Modul3
Nachfolgend werden die Sequenzen der verwendeten Primer angegeben:

Primer für die scTRAIL Klonierung

TRAIL#1R:

5′ATCGATTTCTAGACCCGGGGGGATCCGCCACCAGAACCGCCACCAGAACCGCC

ACCAGAACCGCCACCGCCAACTAAAAAGGCCCCGAAAAAACTGGCTT-

CATGGTC3′

TRAIL#2F:
S'GGGGTAGAATTCGGAACCTCTGAGGAAACCATTTCTACAGTTCAAG3'

TRAIL#3F:
5'AACCTCTGAGGAAACCATTTCTACAG3'

TRAIL#4F:
5'GGGGCGGATCCACCTCTGAGGAAACCATTTCTACAG3'

TRAIL#5R:

5´GGGGCCTCTAGAATCGATGGTCAGCCAACTAAAAAGGCCCCGAAAAAACTGG

C3´

Beispiel 2: Pharmakokinetik von humanem Wildtyp-TNF und humanem scTNF.

6 Wochen alte Balb/c Mäusen wurden i.v. mit 12 μg TNF oder sc TNF (jeweils 3 Mäuse) injiziert. Alle 45 min. wurde Blut abgenommen, gesammelt und die Konzentration von TNF im Serum wurde mittels eines humanen TNF spezifischen ELISA Kits bestimmt. Die Daten in Figur 27 zeigen einen klaren Anstieg der *in vivo* Halbwertszeit der scTNF Varianten. Es wird daher für scTNF erwartet, daß es eine deutlich erhöhte biologische Wirkdauer in vivo besitzt, was damit den Wert von scTNF als potentielle Therapeutikum unterstreicht

**Beispiel 3:** Kovalent an Partikel (Silica) gekoppeltes CysHis-scTNF.

Mausfibroblasten, die mit dem Konstrukt TNFR2- Fas transfiziert sind, wurden mit seriellen Verdünnungen der angegebenen Reagenzien behandelt. Diese Zellen sind völlig resistent gegen lösliches wtTNF. Nach kovalenter Kopplung von reduziertem CysHis- scTNF an Silica- Micropartikel (beads) nach etablierten Protokollen (DPA 2001, Nr. DE10144252) bewirken diese eine starke zytotoxische Antwort (Kreis) , ähnlich wie eine Positivkontrolle bestehend aus CysHis- scTNF und einem TNFR2 vernetzenden Antikörper, mAk 80M2 (Dreieck) . Nicht gekoppeltes Cys- His scTNF zeigt erwartungsgemäß keine Aktivität auf TNFR2 positi- ven Zellen (Quadrate) . Kovalent gekoppeltes CysHis- scTNF ist bioaktiv und besitzt die besondere Aktivität von membrangebundenem TNF, i.e. es aktiviert TNFR2.

**Beispiel 4:** Vergleich von Standard rekombinantem humanen (rh)TNF und scTNF in *in vivo* Tumornekrose- Modellen und *in vitro* L929-Zytotoxizitäts-Aktivität

Mäuse: C3H/HeJ (weiblich), 17-19 g von Charles River

Tumor Zellen: CFS-1 Methylcholanthrene-induzierte Fibrosarkomazelllinie aus C3H/HeN abgeleitet aus Maus (Referenz: Hafner M., P. Orosz, A. Krüger, und D.N. Männel. 1996 TNF promotes metastasis by impairing natural killer cell activity. Internat. J. Cancer 66:388-392);

Tumornekrose Experiment: Die Mäuse erhielten $1.6 \times 10^7$ CFS-1 Zellen in 50 μl Medium (RPMI, 10% FCS) intradermal in den Rücken; Die Tumore ließ man 12 Tage wachsen, bis sie eine Größe von etwa 5-6 mm Durchmesser, vor der intraperitonealen Injektion mit TNF (10 μg pro Maus) in 200 μl PBS oder PBS alleine als Kontrolle, erreichten. Die Tumorgröße wurde täglich gemessen und makroskopisch untersucht. Die Mäuse wurden am 6. Tag nach der Behandlung getötet und die Tumore für die Histologie entfernt Die Tumore wurden herausgeschnitten, über Nacht in 4% PBS-gepuffertem Formalin fixiert und in Paraffin eingebettet Equatoriale Vertikalschnitte (4μm) wurden mit Hema-toxilin und Eosin gefärbt und mikroskopisch auf Nekrose untersucht (wie z.B. beschrieben in: Lucas R. et aL, 2001, Int J Cancer, 91:543-549).

TNF: rhTNF spezifische Aktivität $6.6 \times 10^6$U/mg (48 h L929 Test ohne Act D) scTNF

In vitro Experiment, LD50 Aktivität im L929 Zytotoxizitätsassay mit Act.D für:

$$\text{rhTNF} = 391 \text{ pg/ml}$$

$$\text{scTNF} = 39 \text{ pg/ml}$$

(getestet mit den gleichen TNF Proben, die für *in vivo* Experimente eingesetzt wurden) scTNF zeigt in diesem in vitro Experiment eine 10-fach erhöhte Aktivität.

In vivo Tumor Nekrose Experiment

| Gruppe | n | Tumordurchmesser | | Nekrose | | |
|--------|---|------|------|----------------|------|------|
| | | d0 | d4 | makroscopisch* | mikroskopisch** | |
| | | | | | <5% | >10% |
| PBS | 6 | 5.2+1 | 7.4+0.5 | 1 | 2 | 0 |
| rhTNF | 7 | 5.2+0.9 | 6.6+1.7 | 3 | 6 | 1 |
| scTNF | 7 | 5.9+0.5 | 7.3+0.3 | 5 | 0 | 7 |

\* = makroskopisch klar erkennbare oberflächliche Nekrose

\*\* = bei mikroskopischer Untersuchung zentrale hämorrhagische Nekrose, <5% oder > 10% des Tumorgewebes

Schlußfolgerung:

Nach 4 Tagen Behandlung mit Einzeldosen wurde kein Unterschied in der Tumorgröße festgestellt (einen Überblick über TNF als Tumortherapeutikum siehe z.B. Eggermont et al, Lancet Oncol. 4.429 (2003) ).
rhTNF induzierte kleine hämorrhagische Nekrosen (<5% des Tumorareals) , makroskopisch nur in 3/7 der Tiere zu sehen.
scTNF induziert größere hämorrhagische Nekrosen (> 10% des Tumorareals) in allen Tumoren (7/7) , 5/7 davon sind makroskopisch zu sehen.
scTNF >> rhTNF in Bezug auf Tumorzytotoxizität *in vitro* und Induktion von Nekrose.


**Patentansprüche**

1.  Verfahren zur extrakorporalen Depletion und/ oder Entfernung von Tumor- Nekrose- Faktor- Rezeptor (TNFR) aus Blut oder Blutfraktionen umfassend die folgenden Schritte:

    a) Gegebenenfalls Auftrennung des Blutes in eine oder mehrere Fraktionen mit festen und/ oder flüssigen Bestandteilen;
    b) Bindung des Bluts oder der Blutfraktionen an eine mit einem Polypeptid gekoppelte Oberfläche oder Partikel, wobei das Polypeptid mindestens drei Komponenten A und mindestens zwei Komponenten B umfasst, wobei jede Komponente A ein TNF-Monomer oder ein Fragment und/oder eine Variante hiervon, das/die Bindungs- und Trimerisierungseigenschaft aufweist, ist und jede Komponenten B ein Peptid-Linker ist, und
    c) Abtrennung des gebundenen TNFR.

2.  Verfahren gemäß Anspruch 1, wobei die Komponenten A identisch oder verschieden sind.

3.  Verfahren gemäß einem der vorangegangenen Ansprüche, wobei die Komponenten A aus dem gleichen Organismus oder verschieden Organismen stammt/stammen.

4.  Verfahren gemäß einem der vorangegangenen Ansprüche, wobei die Komponenten B jeweils zwei der mindestens drei Komponenten A miteinander verknüpfen.

5.  Verfahren gemäß einem der vorangegangenen Ansprüche, wobei mindestens eine der Komponenten B die Aminosäuresequenz $(GGGS)_3$ oder $(GGGS)_4$ aufweist.

6.  Verfahren gemäß einem der vorangegangenen Ansprüche, wobei die Komponenten A und die Komponenten B ein trimere Proteinstruktur ausbilden.

7.  Verfahren gemäß Anspruch 6, wobei die Komponenten A und die Komponenten B eine homotrimere Proteinstruktur ausbilden.

8.  Verfahren gemäß Anspruch 6, wobei die Komponenten A und die Komponenten B eine heterotrimere Proteinstruktur ausbilden.

9.  Verfahren gemäß einem der vorangegangenen Ansprüche, wobei die Komponenten B identisch oder verschieden sind.

**10.** Verfahren gemäß einem der vorangegangenen Ansprüche, wobei die Komponenten B aus dem gleichen Organismus oder verschieden Organismen stammt/stammen.

**11.** Verfahren gemäß einem der vorangegangenen Ansprüche, wobei das Polypeptid eine vorzugsweise N- terminale Tag- Sequenz, insbesondere eine His- Tag- Sequenz oder eine Flag- Tag- Sequenz, aufweist.

**12.** Verfahren gemäß einem der vorangegangenen Ansprüche, wobei das Polypeptid eine vorzugsweise N-terminale Leitpeptidsequenz aufweist.

**13.** Verfahren gemäß einem der vorangegangenen Ansprüche, wobei das Polypeptid mindestens eine weitere Komponente C enthält, die ein Antikörperfragment oder ein anders Protein oder Peptid ist, welches ein spezifisches Zielmolekül auf einer Zelloberfläche selektiv erkennt.

**14.** Verfahren gemäß Anspruch 13, wobei die Komponente C ein Antikörperfragment eines Säugetiers, insbesondere murinen oder humanen Ursprungs, oder ein humanisiertes Antikörperfragment ist.

**15.** Verfahren gemäß Anspruch 13 oder 14, wobei das Antikörperfragment in verschiedenen Antikörperformaten vorliegen kann, z.B. als scFv, insbesondere scFv40.

**16.** Verfahren gemäß Anspruch 13, wobei die Komponente C ein Protein oder Peptid mit Spezifität für ein Zelloberflächenmolekül, insbesondere einen Zytokinrezeptor, ein Wachstumsfaktorrezeptor, ein Integrin oder Zelladhäsionsmolekül ist.

**17.** Verfahren gemäß Anspruch 16, wobei der Zytokinrezeptor ausgewählt ist aus der Gruppe der TNFR Genfamilie.

**Claims**

**1.** Method for extracorporeal depletion and/or removal of tumor necrosis factor receptor (TNFR) from blood or blood fractions comprising the following steps:

(a) optionally separation of the blood in one or more fractions including solid and/or liquid components;
(b) binding of the blood or blood fractions to a surface or particle coupled to a polypeptide, wherein the polypeptide comprises at least three components A and at least two components B, wherein each component A is a TNF monomer or a fragment and/or a variant thereof exhibiting binding and trimerization property, and each component B is a peptide linker, and
(c) separation of the bound TNFR.

**2.** Method according to claim 1, wherein the components A are identical or different.

**3.** Method according to any of the preceding claims, wherein the components A are from the same organism or from different organisms.

**4.** Method according to any of the preceding claims, wherein each of the components B links two of the at least three components A.

**5.** Method according to any of the preceding claims, wherein at least one of the components B comprises the amino acid sequence $(GGGS)_3$ or $(GGGS)_4$.

**6.** Method according to any of the preceding claims, wherein the components A and the components B form a trimeric protein structure.

**7.** Method according to claim 6, wherein the components A and the components B form a homotrimeric protein structure.

**8.** Method according to claim 6, wherein the components A and the components B form a heterotrimeric protein structure.

**9.** Method according to any of the preceding claims, wherein the components B are identical or different.

10. Method according to any of the preceding claims, wherein the components B are from the same organism or from different organisms.

11. Method according to any of the preceding claims, wherein the polypeptide comprises a preferably N-terminal tag sequence, in particular a His tag sequence or a Flag tag sequence.

12. Method according to any of the preceding claims, wherein the polypeptide comprises a preferably N-terminal leader peptide sequence.

13. Method according to any of the preceding claims, wherein the polypeptide contains at least one further component C, which is an antibody fragment or another protein or peptide, which selectively recognises a specific target molecule on a cell surface.

14. Method according to claim 13, wherein the component C is an antibody fragment of a mammal, in particular of murine or human origin, or is a humanized antibody fragment.

15. Method according to claim 13 or 14, wherein the antibody fragment may be present in different antibody formats, such as scFv, in particular scFv40.

16. Method according to claim 13, wherein the component C is a protein or a peptide having specificity for a cell surface molecule, in particular a cytokine receptor, a growth factor receptor, an integrin or a cell adhesion molecule.

17. Method according to claim 16, wherein the cytokine receptor is selected from the group consisting of the TNFR gene family.


**Revendications**

1. Procédé d'élimination et/ou de déplétion extracorporelle du récepteur du facteur de nécrose tumorale (TNFR) du sang ou de fractions sanguines, comprenant les étapes suivantes:

   a) le cas échéant séparer le sang en une ou plusieurs fractions avec des composants solides et/ou liquides ;
   b) lier le sang ou les fractions sanguines à une surface ou une particule couplée à un polypeptide, le polypeptide ayant au moins trois composants A et au moins deux composants B, chaque composant A étant un monomère de TNF ou un fragment et/ou une variante de celui-ci, présentant une propriété de liaison et de trimérisation, et chaque composant B étant un lieur peptidique, et
   c) séparer le TNFR lié.

2. Procédé selon la revendication 1, dans lequel les composants A sont identiques ou différents.

3. Procédé selon l'une des revendications précédentes, dans lequel les composants A proviennent du même organisme ou d'organismes différents.

4. Procédé selon l'une des revendications précédentes, dans lequel les composants B relient respectivement deux des au moins trois composants A.

5. Procédé selon l'une des revendications précédentes, dans lequel au moins un des composants B présente la séquence d'acides aminés $(GGGS)_3$ ou $(GGGS)_4$.

6. Procédé selon l'une des revendications précédentes, dans lequel les composants A et les composants B forment une structure de protéine trimère.

7. Procédé selon la revendication 6, dans lequel les composants A et les composants B forment une structure de protéine homotrimère.

8. Procédé selon la revendication 6, dans lequel les composants A et les composants B forment une structure de protéine hétérotrimère.

9. Procédé selon l'une des revendications précédentes, dans lequel les composants B sont identiques ou différents.

10. Procédé selon l'une des revendications précédentes, dans lequel les composants B proviennent du même organisme ou d'organismes différents.

11. Procédé selon l'une des revendications précédentes, dans lequel le polypeptide présente une séquence Tag de préférence N-terminale, en particulier une séquence His-Tag ou une séquence Flag-Tag.

12. Procédé selon l'une des revendications précédentes, dans lequel le polypeptide présente une séquence de peptide de contrôle de préférence N-terminale.

13. Procédé selon l'une des revendications précédentes, dans lequel le polypeptide comprend au moins un autre composant C, qui est un fragment d'anticorps ou une autre protéine ou un autre peptide, qui identifie sélectivement une molécule cible spécifique sur une surface cellulaire.

14. Procédé selon la revendication 13, dans lequel le composant C est un fragment d'anticorps d'un mammifère, en particulier d'origine murine ou humaine, ou un fragment d'anticorps humanisé.

15. Procédé selon la revendication 13 ou 14, dans lequel le fragment d'anticorps peut se présenter sous différents formats d'anticorps, par exemple sous forme scFv, en particulier scFv40.

16. Procédé selon la revendication 13, dans lequel le composant C est une protéine ou un peptide ayant une spécificité pour une molécule de surface cellulaire, en particulier un récepteur de cytokine, un récepteur de facteur de croissance, une intégrine ou une molécule d'adhésion cellulaire.

17. Procédé selon la revendication 16, dans lequel le récepteur de cytokine est choisi dans le groupe de la famille génique TNFR.

Zytotoxitätstest
sTNF und scTNFs mit neuralisierendem AK

Figur 1

## Zytotoxitätstest
## sTNF und scTNFs auf MF-TNFR2

Figur 2

Zytotoxitätstest
sTNF und scTNFs auf MF-TNFR2 + 80M2

Figur 3

## Stabilitätstest mit MF – frisch titriert

Legend:
- löslichesTNF$_{human}$
- singlechainTNF 3x Linker
- singlechainTNF 4x Linker
- Negativkontr.: Bakterienlysat

Y-axis: relative Zellzahl (OD$_{550nm}$), values 0.2, 0.5, 1.0, 1.5, 2.0

X-axis: ng/ml lösl. bzw. singlechain TNF, values 0.01, 0,05 0.1, 0,5 1, 5

**Figur 4**

## Stabilitätstest mit MF – 8 Tage inkubiert

Figur 5

Figur 6

## Stabilitätstest mit Kym1 – frisch titriert

Figur 7

## Stabilitätstest mit Kym1 – 16 Tage inkubiert

Figur 8

## Stabilitätstest mit Kym1 – 22 Tage inkubiert

Figur 9

## Stabilitätstest mit Kym1 – aus Stammlösung titriert

Figur 10

Stabilitätstest mit humanem Serum
Serumstabilität – frisch titriert

lösl. bzw. singlechainTNF ng/ml

**Figur 11**

**Stabilitätstest mit humanem Serum**
**Serumstabilität – 8 Tage**

**Figur 12**

Silbergel

Figur 13

# Western Blot

**Figur 14**

IkB-Degradations-Assay

Figur 15

**JNK-Assay**

| lösliches TNF | scTNF 3xGGGS-Linker | scTNF 4xGGGS-Linker | Kontroll Lysat |

←c-jun

0  30 60   0 30 60   0  30 60   0  30  60   min.

**Figur 16**

## Electrophoretic Mobility Shift Assay (EMSA)

Figur 17

**Figur 18**

## Figur 19

**Nukleinsäuresequenz und korrespondierende Aminosäuresequenz von scTNF-L short Konstrukt A-II**

```
1    ATG AGA GGA TCG CAT CAC CAT CAC CAT CAC GGA TCA GCG TCG TCT   45
1     M   R   G   S   H   H   H   H   H   H   G   S   A   S   S    15

46   TCT TCT CGT ACC CCG TCT GAC AAA CCG GTT GCT CAC GTT GTT GCA   90
16    S   S   R   T   P   S   D   K   P   V   A   H   V   V   A    30

91   AAC CCG CAG GCT GAA GGT CAA CTG CAA TGG CTG AAC CGT CGT GCT   135
31    N   P   Q   A   E   G   Q   L   Q   W   L   N   R   R   A    45

136  AAC GCT CTG CTG GCT AAC GGT GTT GAA CTG CGT GAC AAC CAG CTG   180
46    N   A   L   L   A   N   G   V   E   L   R   D   N   Q   L    60

181  GTT GTT CCG TCT GAA GGC CTG TAC CTG ATC TAC TCC CAG GTT CTG   225
61    V   V   P   S   E   G   L   Y   L   I   Y   S   Q   V   L    75

226  TTC AAA GGC CAG GGC TGC CCG TCC ACC CAC GTT CTG CTG ACC CAC   270
76    F   K   G   Q   G   C   P   S   T   H   V   L   L   T   H    90

271  ACC ATC TCT CGT ATC GCT GTT TCC TAC CAG ACC AAA GTA AAC CTG   315
91    T   I   S   R   I   A   V   S   Y   Q   T   K   V   N   L   105

316  CTG TCT GCA ATC AAA TCT CCG TGC CAG CGT GAA ACC CCG GAA GGT   360
106   L   S   A   I   K   S   P   C   Q   R   E   T   P   E   G   120

361  GCT GAA GCT AAA CCG TGG TAC GAA CCG ATC TAC CTG GGT GGC GTT   405
121   A   E   A   K   P   W   Y   E   P   I   Y   L   G   G   V   135

406  TTT CAA CTG GAG AAA GGT GAC CGT CTG TCT GCA GAA ATT AAC CGT   450
136   F   Q   L   E   K   G   D   R   L   S   A   E   I   N   R   150

451  CCG GAC TAC CTG GAC TTC GCA GAA TCT GGT CAG GTT TAC TTC GGT   495
151   P   D   Y   L   D   F   A   E   S   G   Q   V   Y   F   G   165

496  ATC ATC GCT CTG GGT GGC GGT TCT GGT GGC GGT TCT GGT GGC GGA   540
166   I   I   A   L   G   G   G   S   G   G   G   S   G   G   G   180

541  TCC TCT TCT CGT ACC CCG TCT GAC AAA CCG GTT GCT CAC GTT GTT   585
181   S   S   S   R   T   P   S   D   K   P   V   A   H   V   V   195

586  GCA AAC CCG CAG GCT GAA GGT CAA CTG CAA TGG CTG AAC CGT CGT   630
196   A   N   P   Q   A   E   G   Q   L   Q   W   L   N   R   R   210

631  GCT AAC GCT CTG CTG GCT AAC GGT GTT GAA CTG CGT GAC AAC CAG   675
211   A   N   A   L   L   A   N   G   V   E   L   R   D   N   Q   225

676  CTG GTT GTT CCG TCT GAA GGC CTG TAC CTG ATC TAC TCC CAG GTT   720
226   L   V   V   P   S   E   G   L   Y   L   I   Y   S   Q   V   240

721  CTG TTC AAA GGC CAG GGC TGC CCG TCC ACC CAC GTT CTG CTG ACC   765
241   L   F   K   G   Q   G   C   P   S   T   H   V   L   L   T   255

766  CAC ACC ATC TCT CGT ATC GCT GTT TCC TAC CAG ACC AAA GTA AAC   810
256   H   T   I   S   R   I   A   V   S   Y   Q   T   K   V   N   270

811  CTG CTG TCT GCA ATC AAA TCT CCG TGC CAG CGT GAA ACC CCG GAA   855
271   L   L   S   A   I   K   S   P   C   Q   R   E   T   P   E   285
```

Fortsetzung Figur 19

```
856    GGT GCT GAA GCT AAA CCG TGG TAC GAA CCG ATC TAC CTG GGT GGC    900
286    G   A   E   A   K   P   W   Y   E   P   I   Y   L   G   G      300

901    GTT TTT CAA CTG GAG AAA GGT GAC CGT CTG TCT GCA GAA ATT AAC    945
301    V   F   Q   L   E   K   G   D   R   L   S   A   E   I   N      315

946    CGT CCG GAC TAC CTG GAC TTC GCA GAA TCT GGT CAG GTT TAC TTC    990
316    R   P   D   Y   L   D   F   A   E   S   G   Q   V   Y   F      330

991    GGT ATC ATC GCT CTG GGT GGC GGT TCT GGT GGC GGT TCT GGT GGC    1035
331    G   I   I   A   L   G   G   G   S   G   G   G   S   G   G      345

1036   GGA TCC TCT TCT CGT ACC CCG TCT GAC AAA CCG GTT GCT CAC GTT    1080
346    G   S   S   S   R   T   P   S   D   K   P   V   A   H   V      360

1081   GTT GCA AAC CCG CAG GCT GAA GGT CAA CTG CAA TGG CTG AAC CGT    1125
361    V   A   N   P   Q   A   E   G   Q   L   Q   W   L   N   R      375

1126   CGT GCT AAC GCT CTG CTG GCT AAC GGT GTT GAA CTG CGT GAC AAC    1170
376    R   A   N   A   L   L   A   N   G   V   E   L   R   D   N      390

1171   CAG CTG GTT GTT CCG TCT GAA GGC CTG TAC CTG ATC TAC TCC CAG    1215
391    Q   L   V   V   P   S   E   G   L   Y   L   I   Y   S   Q      405

1216   GTT CTG TTC AAA GGC CAG GGC TGC CCG TCC ACC CAC GTT CTG CTG    1260
406    V   L   F   K   G   Q   G   C   P   S   T   H   V   L   L      420

1261   ACC CAC ACC ATC TCT CGT ATC GCT GTT TCC TAC CAG ACC AAA GTA    1305
421    T   H   T   I   S   R   I   A   V   S   Y   Q   T   K   V      435

1306   AAC CTG CTG TCT GCA ATC AAA TCT CCG TGC CAG CGT GAA ACC CCG    1350
436    N   L   L   S   A   I   K   S   P   C   Q   R   E   T   P      450

1351   GAA GGT GCT GAA GCT AAA CCG TGG TAC GAA CCG ATC TAC CTG GGT    1395
451    E   G   A   E   A   K   P   W   Y   E   P   I   Y   L   G      465

1396   GGC GTT TTT CAA CTG GAG AAA GGT GAC CGT CTG TCT GCA GAA ATT    1440
466    G   V   F   Q   L   E   K   G   D   R   L   S   A   E   I      480

1441   AAC CGT CCG GAC TAC CTG GAC TTC GCA GAA TCT GGT CAG GTT TAC    1485
481    N   R   P   D   Y   L   D   F   A   E   S   G   Q   V   Y      495

1486   TTC GGT ATC ATC GCT CTG TGA                                    1506
496    F   G   I   I   A   L   *                                      501
```

## Figur 20

Nukleinsäuresequenz und korrespondierende Aminosäuresequenz von
cys- scTNF-L short
Konstrukt B-II

```
1    ATG GGA GAG CTC ATC GAA GGT CGC TGC GCC GGT GGA TCT GGT CAT   45
1    M   G   E   L   I   E   G   R   C   A   G   G   S   G   H     15

46   CAT CAT CAC CAT CAC GGC TCA GAC GGA GCG TCG TCT TCT TCT CGT   90
16   H   H   H   H   H   G   S   D   G   A   S   S   S   S   R     30

91   ACC CCG TCT GAC AAA CCG GTT GCT CAC GTT GTT GCA AAC CCG CAG   135
31   T   P   S   D   K   P   V   A   H   V   V   A   N   P   Q     45

136  GCT GAA GGT CAA CTG CAA TGG CTG AAC CGT CGT GCT AAC GCT CTG   180
46   A   E   G   Q   L   Q   W   L   N   R   R   A   N   A   L     60

181  CTG GCT AAC GGT GTT GAA CTG CGT GAC AAC CAG CTG GTT GTT CCG   225
61   L   A   N   G   V   E   L   R   D   N   Q   L   V   V   P     75

226  TCT GAA GGC CTG TAC CTG ATC TAC TCC CAG GTT CTG TTC AAA GGC   270
76   S   E   G   L   Y   L   I   Y   S   Q   V   L   F   K   G     90

271  CAG GGC TGC CCG TCC ACC CAC GTT CTG CTG ACC CAC ACC ATC TCT   315
91   Q   G   C   P   S   T   H   V   L   L   T   H   T   I   S     105

316  CGT ATC GCT GTT TCC TAC CAG ACC AAA GTA AAC CTG CTG TCT GCA   360
106  R   I   A   V   S   Y   Q   T   K   V   N   L   L   S   A     120

361  ATC AAA TCT CCG TGC CAG CGT GAA ACC CCG GAA GGT GCT GAA GCT   405
121  I   K   S   P   C   Q   R   E   T   P   E   G   A   E   A     135

406  AAA CCG TGG TAC GAA CCG ATC TAC CTG GGT GGC GTT TTT CAA CTG   450
136  K   P   W   Y   E   P   I   Y   L   G   G   V   F   Q   L     150

451  GAG AAA GGT GAC CGT CTG TCT GCA GAA ATT AAC CGT CCG GAC TAC   495
151  E   K   G   D   R   L   S   A   E   I   N   R   P   D   Y     165

496  CTG GAC TTC GCA GAA TCT GGT CaG GTT TAC TTC GGT ATC ATC GCT   540
166  L   D   F   A   E   S   G   Q   V   Y   F   G   I   I   A     180

541  CTG GGT GGC GGT TCT GGT GGC GGT TCT GGT GGC GGA TCC TCT TCT   585
181  L   G   G   G   S   G   G   G   S   G   G   G   S   S   S     195

586  CGT ACC CCG TCT GAC AAA CCG GTT GCT CAC GTT GTT GCA AAC CCG   630
196  R   T   P   S   D   K   P   V   A   H   V   V   A   N   P     210

631  CAG GCT GAA GGT CAA CTG CAA TGG CTG AAC CGT CGT GCT AAC GCT   675
211  Q   A   E   G   Q   L   Q   W   L   N   R   R   A   N   A     225

676  CTG CTG GCT AAC GGT GTT GAA CTG CGT GAC AAC CAG CTG GTT GTT   720
226  L   L   A   N   G   V   E   L   R   D   N   Q   L   V   V     240

721  CCG TCT GAA GGC CTG TAC CTG ATC TAC TCC CAG GTT CTG TTC AAA   765
241  P   S   E   G   L   Y   L   I   Y   S   Q   V   L   F   K     255

766  GGC CAG GGC TGC CCG TCC ACC CAC GTT CTG CTG ACC CAC ACC ATC   810
256  G   Q   G   C   P   S   T   H   V   L   L   T   H   T   I     270
```

## Fortsetzung Figur 20

```
811   TCT CGT ATC GCT GTT TCC TAC CAG ACC AAA GTA AAC CTG CTG TCT   855
271    S   R   I   A   V   S   Y   Q   T   K   V   N   L   L   S    285

856   GCA ATC AAA TCT CCG TGC CAG CGT GAA ACC CCG GAA GGT GCT GAA   900
286    A   I   K   S   P   C   Q   R   E   T   P   E   G   A   E    300

901   GCT AAA CCG TGG TAC GAA CCG ATC TAC CTG GGT GGC GTT TTT CAA   945
301    A   K   P   W   Y   E   P   I   Y   L   G   G   V   F   Q    315

946   CTG GAG AAA GGT GAC CGT CTG TCT GCA GAA ATT AAC CGT CCG GAC   990
316    L   E   K   G   D   R   L   S   A   E   I   N   R   P   D    330

991   TAC CTG GAC TTC GCA GAA TCT GGT CAG GTT TAC TTC GGT ATC ATC   1035
331    Y   L   D   F   A   E   S   G   Q   V   Y   F   G   I   I    345

1036  GCT CTG GGT GGC GGT TCT GGT GGC GGT TCT GGT GGC GGA TCC TCT   1080
346    A   L   G   G   G   S   G   G   G   S   G   G   G   S   S    360

1081  TCT CGT ACC CCG TCT GAC AAA CCG GTT GCT CAC GTT GTT GCA AAC   1125
361    S   R   T   P   S   D   K   P   V   A   H   V   V   A   N    375

1126  CCG CAG GCT GAA GGT CAA CTG CAA TGG CTG AAC CGT CGT GCT AAC   1170
376    P   Q   A   E   G   Q   L   Q   W   L   N   R   R   A   N    390

1171  GCT CTG CTG GCT AAC GGT GTT GAA CTG CGT GAC AAC CAG CTG GTT   1215
391    A   L   L   A   N   G   V   E   L   R   D   N   Q   L   V    405

1216  GTT CCG TCT GAA GGC CTG TAC CTG ATC TAC TCC CAG GTT CTG TTC   1260
406    V   P   S   E   G   L   Y   L   I   Y   S   Q   V   L   F    420

1261  AAA GGC CAG GGC TGC CCG TCC ACC CAC GTT CTG CTG ACC CAC ACC   1305
421    K   G   Q   G   C   P   S   T   H   V   L   L   T   H   T    435

1306  ATC TCT CGT ATC GCT GTT TCC TAC CAG ACC AAA GTA AAC CTG CTG   1350
436    I   S   R   I   A   V   S   Y   Q   T   K   V   N   L   L    450

1351  TCT GCA ATC AAA TCT CCG TGC CAG CGT GAA ACC CCG GAA GGT GCT   1395
451    S   A   I   K   S   P   C   Q   R   E   T   P   E   G   A    465

1396  GAA GCT AAA CCG TGG TAC GAA CCG ATC TAC CTG GGT GGC GTT TTT   1440
466    E   A   K   P   W   Y   E   P   I   Y   L   G   G   V   F    480

1441  CAA CTG GAG AAA GGT GAC CGT CTG TCT GCA GAA ATT AAC CGT CCG   1485
481    Q   L   E   K   G   D   R   L   S   A   E   I   N   R   P    495

1486  GAC TAC CTG GAC TTC GCA GAA TCT GGT CAG GTT TAC TTC GGT ATC   1530
496    D   Y   L   D   F   A   E   S   G   Q   V   Y   F   G   I    510

1531  ATC GCT CTG TGA                                               1542
511    I   A   L   *                                                513
```

# Figur 21

Nukleinsäuresequenz und korrespondierende Aminosäuresequenz von scFasL
Konstrukt C

```
1      ATG GCT ATC ATC TAC CTC ATC CTC CTG TTC ACC GCT GTG CGG GGC    45
1       M   A   I   I   Y   L   I   L   L   F   T   A   V   R   G     15

46     GCG GCC GCG GAT TAC AAA GAC GAT GAC GAT AAA GAA TTC ACG CGT    90
16      A   A   A   D   Y   K   D   D   D   D   K   E   F   T   R     30

91     GAA AAA AAG GAG CTG AGG AAA GTG GCC CAT TTA ACA GGC AAG TCC   135
31      E   K   K   E   L   R   K   V   A   H   L   T   G   K   S     45

136    AAC TCA AGG TCC ATG CCT CTG GAA TGG GAA GAC ACC TAT GGA ATT   180
46      N   S   R   S   M   P   L   E   W   E   D   T   Y   G   I     60

181    GTC CTG CTT TCT GGA GTG AAG TAT AAG AAG GGT GGC CTT GTG ATC   225
61      V   L   L   S   G   V   K   Y   K   K   G   G   L   V   I     75

226    AAT GAA ACT GGG CTG TAC TTT GTA TAT TCC AAA GTA TAC TTC CGG   270
76      N   E   T   G   L   Y   F   V   Y   S   K   V   Y   F   R     90

271    GGT CAA TCT TGC AAC AAC CTG CCC CTG AGC CAC AAG GTC TAC ATG   315
91      G   Q   S   C   N   N   L   P   L   S   H   K   V   Y   M    105

316    AGG AAC TCT AAG TAT CCC CAG GAT CTG GTG ATG ATG GAG GGG AAG   360
106     R   N   S   K   Y   P   Q   D   L   V   M   M   E   G   K    120

361    ATG ATG AGC TAC TGC ACT ACT GGG CAG ATG TGG GCC CGC AGC AGC   405
121     M   M   S   Y   C   T   T   G   Q   M   W   A   R   S   S    135

406    TAC CTG GGG GCA GTG TTC AAT CTT ACC AGT GCT GAT CAT TTA TAT   450
136     Y   L   G   A   V   F   N   L   T   S   A   D   H   L   Y    150

451    GTC AAC GTA TCT GAG CTC TCT CTG GTC AAT TTT GAG GAA TCT CAG   495
151     V   N   V   S   E   L   S   L   V   N   F   E   E   S   Q    165

496    ACG TTT TTC GGC TTA TAT AAG CTC GGT GGC GGT TCT GGT GGC GGT   540
166     T   F   F   G   L   Y   K   L   G   G   G   S   G   G   G    180

541    TCT GGT GGC GGT TCT GGT GGC GGA TCA GAA AAA AAG GAG CTG AGG   585
181     S   G   G   G   S   G   G   G   S   E   K   K   E   L   R    195

586    AAA GTG GCC CAT TTA ACA GGC AAG TCC AAC TCA AGG TCC ATG CCT   630
196     K   V   A   H   L   T   G   K   S   N   S   R   S   M   P    210

631    CTG GAA TGG GAA GAC ACC TAT GGA ATT GTC CTG CTT TCT GGA GTG   675
211     L   E   W   E   D   T   Y   G   I   V   L   L   S   G   V    225

676    AAG TAT AAG AAG GGT GGC CTT GTG ATC AAT GAA ACT GGG CTG TAC   720
226     K   Y   K   K   G   G   L   V   I   N   E   T   G   L   Y    240

721    TTT GTA TAT TCC AAA GTA TAC TTC CGG GGT CAA TCT TGC AAC AAC   765
241     F   V   Y   S   K   V   Y   F   R   G   Q   S   C   N   N    255
```

Fortsetzung Figur 21

```
766   CTG CCC CTG AGC CAC AAG GTC TAC ATG AGG AAC TCT AAG TAT CCC   810
256    L   P   L   S   H   K   V   Y   M   R   N   S   K   Y   P    270

811   CAG GAT CTG GTG ATG ATG GAG GGG AAG ATG ATG AGC TAC TGC ACT   855
271    Q   D   L   V   M   M   E   G   K   M   M   S   Y   C   T    285

856   ACT GGG CAG ATG TGG GCC CGC AGC AGC TAC CTG GGG GCA GTG TTC   900
286    T   G   Q   M   W   A   R   S   S   Y   L   G   A   V   F    300

901   AAT CTT ACC AGT GCT GAT CAT TTA TAT GTC AAC GTA TCT GAG CTC   945
301    N   L   T   S   A   D   H   L   Y   V   N   V   S   E   L    315

946   TCT CTG GTC AAT TTT GAG GAA TCT CAG ACG TTT TTC GGC TTA TAT   990
316    S   L   V   N   F   E   E  ·S   Q   T   F   F   G   L   Y    330

991   AAG CTC GGT GGC GGT TCT GGT GGC GGT TCT GGT GGC GGT TCT GGT   1035
331    K   L   G   G   G   S   G   G   G   S   G   G   G   S   G    345

1036  GGC GGA TCC GAA AAA AAG GAG CTG AGG AAA GTG GCC CAT TTA ACA   1080
346    G   G   S   E   K   K   E   L   R   K   V   A   H   L   T    360

1081  GGC AAG TCC AAC TCA AGG TCC ATG CCT CTG GAA TGG GAA GAC ACC   1125
361    G   K   S   N   S   R   S   M   P   L   E   W   E   D   T    375

1126  TAT GGA ATT GTC CTG CTT TCT GGA GTG AAG TAT AAG AAG GGT GGC   1170
376    Y   G   I   V   L   L   S   G   V   K   Y   K   K   G   G    390

1171  CTT GTG ATC AAT GAA ACT GGG CTG TAC TTT GTA TAT TCC AAA GTA   1215
391    L   V   I   N   E   T   G   L   Y   F   V   Y   S   K   V    405

1216  TAC TTC CGG GGT CAA TCT TGC AAC AAC CTG CCC CTG AGC CAC AAG   1260
406    Y   F   R   G   Q   S   C   N   N   L   P   L   S   H   K    420

1261  GTC TAC ATG AGG AAC TCT AAG TAT CCC CAG GAT CTG GTG ATG ATG   1305
421    V   Y   M   R   N   S   K   Y   P   Q   D   L   V   M   M    435

1306  GAG GGG AAG ATG ATG AGC TAC TGC ACT ACT GGG CAG ATG TGG GCC·· 1350
436    E   G   K   M   M   S   Y   C   T   T   G   Q   M   W   A    450

1351  CGC AGC AGC TAC CTG GGG GCA GTG TTC AAT CTT ACC AGT GCT GAT   1395
451    R   S   S   Y   L   G   A   V   F   N   L   T   S   A   D    465

1396  CAT TTA TAT GTC AAC GTA TCT GAG CTC TCT CTG GTC AAT TTT GAG   1440
466    H   L   Y   V   N   V   S   E   L   S   L   V   N   F   E    480

1441  GAA TCT CAG ACG TTT TTC GGC TTA TAT AAG CTC TGA               1476
481    E   S   Q   T   F   F   G   L   Y   K   L   *                491
```

## Figur 22

Nukleinsäuresequenz und korrespondierende Aminosäuresequenz von scTRAIL
Konstrukt D

```
1     ATG GCT ATC ATC TAC CTC ATC CTC CTG TTC ACC GCT GTG CGG GGC   45
1      M   A   I   I   Y   L   I   L   L   F   T   A   V   R   G    15

46    GCG GCC GCG GAT TAC AAA GAC GAT GAC GAT AAA GAA TTC GGA ACC   90
16     A   A   A   D   Y   K   D   D   D   D   K   E   F   G   T    30

91    TCT GAG GAA ACC ATT TCT ACA GTT CAA GAA AAG CAA CAA AAT ATT  135
31     S   E   E   T   I   S   T   V   Q   E   K   Q   Q   N   I    45

136   TCT CCC CTA GTG AGA GAA AGA GGT CCT CAG AGA GTA GCA GCT CAC  180
46     S   P   L   V   R   E   R   G   P   Q   R   V   A   A   H    60

181   ATA ACT GGG ACC AGA GGA AGA AGC AAC ACA TTG TCT TCT CCA AAC  225
61     I   T   G   T   R   G   R   S   N   T   L   S   S   P   N    75

226   TCC AAG AAT GAA AAG GCT CTG GGC CGC AAA ATA AAC TCC TGG GAA  270
76     S   K   N   E   K   A   L   G   R   K   I   N   S   W   E    90

271   TCA TCA AGG AGT GGG CAT TCA TTC CTG AGC AAC TTG CAC TTG AGG  315
91     S   S   R   S   G   H   S   F   L   S   N   L   H   L   R   105

316   AAT GGT GAA CTG GTC ATC CAT GAA AAA GGG TTT TAC TAC ATC TAT  360
106    N   G   E   L   V   I   H   E   K   G   F   Y   Y   I   Y   120

361   TCC CAA ACA TAC TTT CGA TTT CAG GAG GAA ATA AAA GAA AAC ACA  405
121    S   Q   T   Y   F   R   F   Q   E   E   I   K   E   N   T   135

406   AAG AAC GAC AAA CAA ATG GTC CAA TAT ATT TAC AAA TAC ACA AGT  450
136    K   N   D   K   Q   M   V   Q   Y   I   Y   K   Y   T   S   150

451   TAT CCT GAC CCT ATA TTG TTG ATG AAA AGT GCT AGA AAT AGT TGT  495
151    Y   P   D   P   I   L   L   M   K   S   A   R   N   S   C   165

496   TGG TCT AAA GAT GCA GAA TAT GGA CTC TAT TCC ATC TAT CAA GGG  540
166    W   S   K   D   A   E   Y   G   L   Y   S   I   Y   Q   G   180

541   GGA ATA TTT GAG CTT AAG GAA AAT GAC AGA ATT TTT GTT TCT GTA  585
181    G   I   F   E   L   K   E   N   D   R   I   F   V   S   V   195

586   ACA AAT GAG CAC TTG ATA GAC ATG GAC CAT GAA GCC AGT TTT TTC  630
196    T   N   E   H   L   I   D   M   D   H   E   A   S   F   F   210

631   GGG GCC TTT TTA GTT GGC GGT GGC GGT TCT GGT GGC GGT TCT GGT  675
211    G   A   F   L   V   G   G   G   G   S   G   G   G   S   G   225

676   GGC GGT TCT GGT GGC GGA TCA ACC TCT GAG GAA ACC ATT TCT ACA  720
226    G   G   S   G   G   G   S   T   S   E   E   T   I   S   T   240

721   GTT CAA GAA AAG CAA CAA AAT ATT TCT CCC CTA GTG AGA GAA AGA  765
241    V   Q   E   K   Q   Q   N   I   S   P   L   V   R   E   R   255
```

Fortsetzung Figur 22

```
766    GGT CCT CAG AGA GTA GCA GCT CAC ATA ACT GGG ACC AGA GGA AGA    810
256     G   P   Q   R   V   A   A   H   I   T   G   T   R   G   R     270

811    AGC AAC ACA TTG TCT TCT CCA AAC TCC AAG AAT GAA AAG GCT CTG    855
271     S   N   T   L   S   S   P   N   S   K   N   E   K   A   L     285

856    GGC CGC AAA ATA AAC TCC TGG GAA TCA TCA AGG AGT GGG CAT TCA    900
286     G   R   K   I   N   S   W   E   S   S   R   S   G   H   S     300

901    TTC CTG AGC AAC TTG CAC TTG AGG AAT GGT GAA CTG GTC ATC CAT    945
301     F   L   S   N   L   H   L   R   N   G   E   L   V   I   H     315

946    GAA AAA GGG TTT TAC TAC ATC TAT TCC CAA ACA TAC TTT CGA TTT    990
316     E   K   G   F   Y   Y   I   Y   S   Q   T   Y   F   R   F     330

991    CAG GAG GAA ATA AAA GAA AAC ACA AAG AAC GAC AAA CAA ATG GTC    1035
331     Q   E   E   I   K   E   N   T   K   N   D   K   Q   M   V     345

1036   CAA TAT ATT TAC AAA TAC ACA AGT TAT CCT GAC CCT ATA TTG TTG    1080
346     Q   Y   I   Y   K   Y   T   S   Y   P   D   P   I   L   L     360

1081   ATG AAA AGT GCT AGA AAT AGT TGT TGG TCT AAA GAT GCA GAA TAT    1125
361     M   K   S   A   R   N   S   C   W   S   K   D   A   E   Y     375

1126   GGA CTC TAT TCC ATC TAT CAA GGG GGA ATA TTT GAG CTT AAG GAA    1170
376     G   L   Y   S   I   Y   Q   G   G   I   F   E   L   K   E     390

1171   AAT GAC AGA ATT TTT GTT TCT GTA ACA AAT GAG CAC TTG ATA GAC    1215
391     N   D   R   I   F   V   S   V   T   N   E   H   L   I   D     405

1216   ATG GAC CAT GAA GCC AGT TTT TTC GGG GCC TTT TTA GTT GGC GGT    1260
406     M   D   H   E   A   S   F   F   G   A   F   L   V   G   G     420

1261   GGC GGT TCT GGT GGC GGT TCT GGT GGC GGT TCT GGT GGC GGA TCC    1305
421     G   G   S   G   G   G   S   G   G   G   S   G   G   G   S     435

1306   ACC TCT GAG GAA ACC ATT TCT ACA GTT CAA GAA AAG CAA CAA AAT    1350
436     T   S   E   E   T   I   S   T   V   Q   E   K   Q   Q   N     450

1351   ATT TCT CCC CTA GTG AGA GAA AGA GGT CCT CAG AGA GTA GCA GCT    1395
451     I   S   P   L   V   R   E   R   G   P   Q   R   V   A   A     465

1396   CAC ATA ACT GGG ACC AGA GGA AGA AGC AAC ACA TTG TCT TCT CCA    1440
466     H   I   T   G   T   R   G   R   S   N   T   L   S   S   P     480

1441   AAC TCC AAG AAT GAA AAG GCT CTG GGC CGC AAA ATA AAC TCC TGG    1485
481     N   S   K   N   E   K   A   L   G   R   K   I   N   S   W     495

1486   GAA TCA TCA AGG AGT GGG CAT TCA TTC CTG AGC AAC TTG CAC TTG    1530
496     E   S   S   R   S   G   H   S   F   L   S   N   L   H   L     510

1531   AGG AAT GGT GAA CTG GTC ATC CAT GAA AAA GGG TTT TAC TAC ATC    1575
511     R   N   G   E   L   V   I   H   E   K   G   F   Y   Y   I     525

1576   TAT TCC CAA ACA TAC TTT CGA TTT CAG GAG GAA ATA AAA GAA AAC    1620
526     Y   S   Q   T   Y   F   R   F   Q   E   E   I   K   E   N     540
```

Fortsetzung Figur 22

```
1621  ACA AAG AAC GAC AAA CAA ATG GTC CAA TAT ATT TAC AAA TAC ACA  1665
541    T   K   N   D   K   Q   M   V   Q   Y   I   Y   K   Y   T   555

1666  AGT TAT CCT GAC CCT ATA TTG TTG ATG AAA AGT GCT AGA AAT AGT  1710
556    S   Y   P   D   P   I   L   L   M   K   S   A   R   N   S   570

1711  TGT TGG TCT AAA GAT GCA GAA TAT GGA CTC TAT TCC ATC TAT CAA  1755
571    C   W   S   K   D   A   E   Y   G   L   Y   S   I   Y   Q   585

1756  GGG GGA ATA TTT GAG CTT AAG GAA AAT GAC AGA ATT TTT GTT TCT  1800
586    G   G   I   F   E   L   K   E   N   D   R   I   F   V   S   600

1801  GTA ACA AAT GAG CAC TTG ATA GAC ATG GAC CAT GAA GCC AGT TTT  1845
601    V   T   N   E   H   L   I   D   M   D   H   E   A   S   F   615

1846  TTC GGG GCC TTT TTA GTT GGC TGA                              1866
616    F   G   A   F   L   V   G   *                               622
```

# Figur 23

Nukleinsäuresequenz und korrespondierende Aminosäuresequenz von scTNF

<u>Konstrukt E</u>

```
1     ATG GCT ATC ATC TAC CTC ATC CTC CTG TTC ACC GCT GTG CGG GGC    45
1     M   A   I   I   Y   L · I   L   L   F   T   A   V   R   G       15

46    GCG GCC GCG GAT TAC AAA GAC GAT GAC GAT AAA GAA TTC GGA TCA    90
16    A   A   A   D   Y   K   D   D   D   D   K   E   F   G   S       30

91    TCT TCT CGA ACC CCG AGT GAC AAG CCT GTA GCC CAT GTT GTA GCA   135
31    S   S   R   T   P   S   D   K   P   V   A   H   V   V   A       45

136   AAC CCT CAA GCT GAG GGG CAG CTC CAG TGG CTG AAC CGC CGG GCC   180
46    N   P   Q   A   E   G   Q   L   Q   W   L   N   R   R   A       60

181   AAT GCC CTC CTG GCC AAT GGC GTG GAG CTG AGA GAT AAC CAG CTG   225
61    N   A   L   L   A   N   G   V   E   L   R   D   N   Q   L       75

226   GTG GTG CCA TCA GAG GGC CTG TAC CTC ATC TAC TCC CAG GTC CTC   270
76    V   V   P   S   E   G   L   Y   L   I   Y   S   Q   V   L       90

271   TTC AAG GGC CAA GGC TGC CCC TCC ACC CAT GTG CTC CTC ACC CAC   315
91    F   K   G   Q   G   C   P   S   T   H   V   L   L   T   H      105

316   ACC ATC AGC CGC ATC GCC GTC TCC TAC CAG ACC AAG GTC AAC CTC   360
106   T   I   S   R   I   A   V   S   Y   Q   T   K   V   N   L      120

361   CTC TCT GCC ATC AAG AGC CCC TGC CAG AGG GAG ACC CCA GAG GGG   405
121   L   S   A   I   K   S   P   C   Q   R   E   T   P   E   G      135

406   GCT GAG GCC AAG CCC TGG TAT GAG CCC ATC TAT CTG GGA GGG GTC   450
136   A   E   A   K   P   W   Y   E   P   I   Y   L   G   G   V      150

451   TTC CAG CTG GAG AAG GGT GAC CGA CTC AGC GCT GAG ATC AAT CGG   495
151   F   Q   L   E   K   G   D   R   L   S   A   E   I   N   R      165

496   CCC GAC TAT CTC GAC TTT GCC GAG TCT GGG CAG GTC TAC TTT GGG   540
166   P   D   Y   L   D   F   A   E   S   G   Q   V   Y   F   G      180

541   ATC ATT GCC CTG GGT GGC GGT TCT GGT GGC GGT TCT GGT GGC GGT   585
181   I   I   A   L   G   G   G   S   G   G   G   S   G   G   G      195

586   TCT GGT GGC GGA TCA TCA TCT TCT CGA ACC CCG AGT GAC AAG CCT   630
196   S   G   G   G   S   S   S   S   R   T   P   S   D   K   P      210

631   GTA GCC CAT GTT GTA GCA AAC CCT CAA GCT GAG GGG CAG CTC CAG   675
211   V   A   H   V   V   A   N   P   Q   A   E   G   Q   L   Q      225

676   TGG CTG AAC CGC CGG GCC AAT GCC CTC CTG GCC AAT GGC GTG GAG   720
226   W   L   N   R   R   A   N   A   L   L   A   N   G   V   E      240

721   CTG AGA GAT AAC CAG CTG GTG GTG CCA TCA GAG GGC CTG TAC CTC   765
241   L   R   D   N   Q   L   V   V   P   S   E   G   L   Y   L      255
```

EP 1 727 833 B1

Fortsetzung Figur 23

```
766    ATC TAC TCC CAG GTC CTC TTC AAG GGC CAA GGC TGC CCC TCC ACC    810
256     I   Y   S   Q   V   L   F   K   G   Q   G   C   P   S   T     270

811    CAT GTG CTC CTC ACC CAC ACC ATC AGC CGC ATC GCC GTC TCC TAC    855
271     H   V   L   L   T   H   T   I   S   R   I   A   V   S   Y     285

856    CAG ACC AAG GTC AAC CTC CTC TCT GCC ATC AAG AGC CCC TGC CAG    900
286     Q   T   K   V   N   L   L   S   A   I   K   S   P   C   Q     300

901    AGG GAG ACC CCA GAG GGG GCT GAG GCC AAG CCC TGG TAT GAG CCC    945
301     R   E   T   P   E   G   A   E   A   K   P   W   Y   E   P     315

946    ATC TAT CTG GGA GGG GTC TTC CAG CTG GAG AAG GGT GAC CGA CTC    990
316     I   Y   L   G   G   V   F   Q   L   E   K   G   D   R   L     330

991    AGC GCT GAG ATC AAT CGG CCC GAC TAT CTC GAC TTT GCC GAG TCT    1035
331     S   A   E   I   N   R   P   D   Y   L   D   F   A   E   S     345

1036   GGG CAG GTC TAC TTT GGG ATC ATT GCC CTG GGT GGC GGT TCT GGT    1080
346     G   Q   V   Y   F   G   I   I   A   L   G   G   G   S   G     360

1081   GGC GGT TCT GGT GGC GGT TCT GGT GGC GGA TCA TCA TCT TCT CGA    1125
361     G   G   S   G   G   G   S   G   G   G   S   S   S   S   R     375

1126   ACC CCG AGT GAC AAG CCT GTA GCC CAT GTT GTA GCA AAC CCT CAA    1170
376     T   P   S   D   K   P   V   A   H   V   V   A   N   P   Q     390

1171   GCT GAG GGG CAG CTC CAG TGG CTG AAC CGC CGG GCC AAT GCC CTC    1215
391     A   E   G   Q   L   Q   W   L   N   R   R   A   N   A   L     405

1216   CTG GCC AAT GGC GTG GAG CTG AGA GAT AAC CAG CTG GTG GTG CCA    1260
406     L   A   N   G   V   E   L   R   D   N   Q   L   V   V   P     420

1261   TCA GAG GGC CTG TAC CTC ATC TAC TCC CAG GTC CTC TTC AAG GGC    1305
421     S   E   G   L   Y   L   I   Y   S   Q   V   L   F   K   G     435

1306   CAA GGC TGC CCC TCC ACC CAT GTG CTC CTC ACC CAC ACC ATC AGC    1350
436     Q   G   C   P   S   T   H   V   L   L   T   H   T   I   S     450

1351   CGC ATC GCC GTC TCC TAC CAG ACC AAG GTC AAC CTC CTC TCT GCC    1395
451     R   I   A   V   S   Y   Q   T   K   V   N   L   L   S   A     465

1396   ATC AAG AGC CCC TGC CAG AGG GAG ACC CCA GAG GGG GCT GAG GCC    1440
466     I   K   S   P   C   Q   R   E   T   P   E   G   A   E   A     480

1441   AAG CCC TGG TAT GAG CCC ATC TAT CTG GGA GGG GTC TTC CAG CTG    1485
481     K   P   W   Y   E   P   I   Y   L   G   G   V   F   Q   L     495

1486   GAG AAG GGT GAC CGA CTC AGC GCT GAG ATC AAT CGG CCC GAC TAT    1530
496     E   K   G   D   R   L   S   A   E   I   N   R   P   D   Y     510

1531   CTC GAC TTT GCC GAG TCT GGG CAG GTC TAC TTT GGG ATC ATT GCC    1575
511     L   D   F   A   E   S   G   Q   V   Y   F   G   I   I   A     525

1576   CTG TGA                                                         1581
526     L   *                                                         526
```

59

## Figur 24

**Nukleinsäuresequenz und korrespondierende Aminosäuresequenz von scFasL-
AMAIZe**

**Konstrukt F**

```
1     ATG GAC TGG ACC TGG CGC GTG TTT TGC CTG CTC GCC GTG GCT CCT   45
1      M   D   W   T   W   R   V   F   C   L   L   A   V   A   P    15

46    GGG GCC CAC AGC CAG GTA CAG CTG GTG CAG TCT GGG GGA GGC ATG   90
16     G   A   H   S   Q   V   Q   L   V   Q   S   G   G   G   M    30

91    GTA GAG CCT GGG GGG TCC CTT AGA CTC TCC TGT GCA GCC TCT GGA  135
31     V   E   P   G   G   S   L   R   L   S   C   A   A   S   G    45

136   TTC ACT TTC AGT AAT GCC TGG ATG AGC TGG GTC CGC CAG GCT CCA  180
46     F   T   F   S   N   A   W   M   S   W   V   R   Q   A   P    60

181   GGG AAG GGG CTG GAG TGG GTT GGC CGT ATA AAA AGC AAA GCT GGT  225
61     G   K   G   L   E   W   V   G   R   I   K   S   K   A   G    75

226   GGT GGG ACA GCA GAG TAC GCT GCA CCC GTG AAA GGC AGA TTC ACC  270
76     G   G   T   A   E   Y'  A   A   P   V   K   G   R   F   T    90

271   ATC TCA AGA GAT GAT TCA CAA AAC ACG CTG TAT CTG CAA ATG AAC  315
91     I   S   R   D   D   S   Q   N   T   L   Y   L   Q   M   N   105

316   AGC CTG AAA ACC GAC GAC ACA GCC GTG TAT TAC TGT ACC ACA CAT  360
106    S   L   K   T   D   D   T   A   V   Y   Y   C   T   T   H   120

361   GTC TAC GGT GCC CCC CGG AAC TGG GGC CAG GGA TCC CTG GTC ACC  405
121    V   Y   G   A   P   R   N   W   G   Q   G   S   L   V   T   135

406   GTC TCC TCA GCC TCC ACC AAG GGC CCA AAG CTT GAA GAA GGT GAA  450
136    V   S   S   A   S   T   K   G   P   K   L   E   E   G   E   150

451   TTT TCA GAA GCA CGC GTA CAG TCT GTG TTG ACT CAG CCG CCC TCA  495
151    F   S   E   A   R   V   Q   S   V   L   T   Q   P   P   S   165

496   GTG TCT GCG GCC CCA GGA CAG AAG GTC ACC ATC TCC TGC TCT GGA  540
166    V   S   A   A   P   G   Q   K   V   T   I   S   C   S   G   180

541   AGC AGC TCC AAC ATT GGA AAT AAT TAT GTC TCC TGG TAC GTT CAA  585
181    S   S   S   N   I   G   N   N   Y   V   S   W   Y   V   Q   195

586   CTC CCA GGA ACA GCC CCC AAA CTC CTC ATT TAT GAC AAT AAT AAG  630
196    L   P   G   T   A   P   K   L   L   I   Y   D   N   N   K   210

631   CGA TTC TCA GGA GTT CCT GAC CGA TTC TCT GGC TCC AAG TCT GGC  675
211    R   F   S   G   V   P   D   R   F   S   G   S   K   S   G   225

676   ACG TCA GCC ACC CTG GGC ATC ACC GGG CTC CAG ACT GGG GAC GAG  720
226    T   S   A   T   L   G   I   T   G   L   Q   T   G   D   E   240

721   GCC GAT TAT TAC TGC GGA GCA TGG GAT GGC AGC CTG CGT GAA GCG  765
241    A   D   Y   Y   C   G   A   W   D   G   S   L   R   E   A   255
```

Fortsetzung Figur 24

```
766   GTA TTC GGC GGA GGG ACC AAG GTC ACC GTC CTA GGT GCG GCC GCA   810
256    V   F   G   G   G   T   K   V   T   V   L   G   A   A   A    270

811   GTT GAG CTC GAG gcg GCC GCG GAT TAC AAA GAC GAT GAC GAT AAA   855
271    V   E   L   E   A   A   A   D   Y   K   D   D   D   D   K    285

856   GAA TTC ACG CGT GAA AAA AAG GAG CTG AGG AAA GTG GCC CAT TTA   900
286    E   F   T   R   E   K   K   E   L   R   K   V   A   H   L    300

901   ACA GGC AAG TCC AAC TCA AGG TCC ATG CCT CTG GAA TGG GAA GAC   945
301    T   G   K   S   N   S   R   S   M   P   L   E   W   E   D    315

946   ACC TAT GGA ATT GTC CTG CTT TCT GGA GTG AAG TAT AAG AAG GGT   990
316    T   Y   G   I   V   L   L   S   G   V   K   Y   K   K   G    330

991   GGC CTT GTG ATC AAT GAA ACT GGG CTG TAC TTT GTA TAT TCC AAA  1035
331    G   L   V   I   N   E   T   G   L   Y   F   V   Y   S   K    345

1036  GTA TAC TTC CGG GGT CAA TCT TGC AAC AAC CTG CCC CTG AGC CAC  1080
346    V   Y   F   R   G   Q   S   C   N   N   L   P   L   S   H    360

1081  AAG GTC TAC ATG AGG AAC TCT AAG TAT CCC CAG GAT CTG GTG ATG  1125
361    K   V   Y   M   R   N   S   K   Y   P   Q   D   L   V   M    375

1126  ATG GAG GGG AAG ATG ATG AGC TAC TGC ACT ACT GGG CAG ATG TGG  1170
376    M   E   G   K   M   M   S   Y   C   T   T   G   Q   M   W    390

1171  GCC CGC AGC AGC TAC CTG GGG GCA GTG TTC AAT CTT ACC AGT GCT  1215
391    A   R   S   S   Y   L   G   A   V   F   N   L   T   S   A    405

1216  GAT CAT TTA TAT GTC AAC GTA TCT GAG CTC TCT CTG GTC AAT TTT  1260
406    D   H   L   Y   V   N   V   S   E   L   S   L   V   N   F    420

1261  GAG GAA TCT CAG ACG TTT TTC GGC TTA TAT AAG CTC GGT GGC GGT  1305
421    E   E   S   Q   T   F   F   G   L   Y   K   L   G   G   G    435

1306  TCT GGT GGC GGT TCT GGT GGC GGT TCT GGT GGC GGA TCA GAA AAA  1350
436    S   G   G   G   S   G   G   G   S   G   G   G   S   E   K    450

1351  AAG GAG CTG AGG AAA GTG GCC CAT TTA ACA GGC AAG TCC AAC TCA  1395
451    K   E   L   R   K   V   A   H   L   T   G   K   S   N   S    465

1396  AGG TCC ATG CCT CTG GAA TGG GAA GAC ACC TAT GGA ATT GTC CTG  1440
466    R   S   M   P   L   E   W   E   D   T   Y   G   I   V   L    480

1441  CTT TCT GGA GTG AAG TAT AAG AAG GGT GGC CTT GTG ATC AAT GAA  1485
481    L   S   G   V   K   Y   K   K   G   G   L   V   I   N   E    495

1486  ACT GGG CTG TAC TTT GTA TAT TCC AAA GTA TAC TTC CGG GGT CAA  1530
496    T   G   L   Y   F   V   Y   S   K   V   Y   F   R   G   Q    510

1531  TCT TGC AAC AAC CTG CCC CTG AGC CAC AAG GTC TAC ATG AGG AAC  1575
511    S   C   N   N   L   P   L   S   H   K   V   Y   M   R   N    525

1576  TCT AAG TAT CCC CAG GAT CTG GTG ATG ATG GAG GGG AAG ATG ATG  1620
526    S   K   Y   P   Q   D   L   V   M   M   E   G   K   M   M    540
```

Fortsetzung Figur 24

```
1621  AGC TAC TGC ACT ACT GGG CAG ATG TGG GCC CGC AGC AGC TAC CTG   1665
541    S   Y   C   T   T   G   Q   M   W   A   R   S   S   Y   L    555

1666  GGG GCA GTG TTC AAT CTT ACC AGT GCT GAT CAT TTA TAT GTC AAC   1710
556    G   A   V   F   N   L   T   S   A   D   H   L   Y   V   N    570

1711  GTA TCT GAG CTC TCT CTG GTC AAT TTT GAG GAA TCT CAG ACG TTT   1755
571    V   S   E   L   S   L   V   N   F   E   E   S   Q   T   F    585

1756  TTC GGC TTA TAT AAG CTC GGT GGC GGT TCT GGT GGC GGT TCT GGT   1800
586    F   G   L   Y   K   L   G   G   G   S   G   G   G   S   G    600

1801  GGC GGT TCT GGT GGC GGA TCC GAA AAA AAG GAG CTG AGG AAA GTG   1845
601    G   G   S   G   G   G   S   E   K   K   E   L   R   K   V    615

1846  GCC CAT TTA ACA GGC AAG TCC AAC TCA AGG TCC ATG·CCT CTG GAA   1890
616    A   H   L   T   G   K   S   N   S   R   S   M   P   L   E    630

1891  TGG GAA GAC ACC TAT GGA ATT GTC CTG CTT TCT GGA GTG AAG TAT   1935
631    W   E   D   T   Y   G   I   V   L   L   S   G   V   K   Y    645

1936  AAG AAG GGT GGC CTT GTG ATC AAT GAA ACT GGG CTG TAC TTT GTA   1980
646    K   K   G   G   L   V   I   N   E   T   G   L   Y   F   V    660

1981  TAT TCC AAA GTA TAC TTC CGG GGT CAA TCT TGC AAC AAC CTG CCC   2025
661    Y   S   K   V   Y   F   R   G   Q   S   C   N   N   L   P    675

2026  CTG AGC CAC AAG GTC TAC ATG AGG AAC TCT AAG TAT CCC CAG GAT   2070
676    L   S   H   K   V   Y   M   R   N   S   K   Y   P   Q   D    690

2071  CTG GTG ATG ATG GAG GGG AAG ATG ATG AGC TAC TGC ACT ACT GGG   2115
691    L   V   M   M   E   G   K   M   M   S   Y   C   T   T   G    705

2116  CAG ATG TGG GCC CGC AGC AGC TAC CTG GGG GCA GTG TTC AAT CTT   2160
706    Q   M   W   A   R   S   S   Y   L   G   A   V   F   N   L    720

2161  ACC AGT GCT GAT CAT TTA TAT GTC AAC GTA TCT GAG CTC TCT CTG   2205
721    T   S   A   D   H   L   Y   V   N   V   S   E   L   S   L    735

2206  GTC AAT TTT GAG GAA TCT CAG ACG TTT TTC GGC TTA TAT AAG CTC   2250
736    V   N   F   E   E   S   Q   T   F   F   G   L   Y   K   L    750

2251  TGA                                                           2253
751    *                                                            751
```

# Figur 25

Nukleinsäuresequenz und korrespondierende Aminosäuresequenz von scTRAIL-AMAIZe

## Konstrukt G

```
1     ATG GAC TGG ACC TGG CGC GTG TTT TGC CTG CTC GCC GTG GCT CCT    45
1     M   D   W   T   W   R   V   F   C   L   L   A   V   A   P       15

46    GGG GCC CAC AGC CAG GTA CAG CTG GTG CAG TCT GGG GGA GGC ATG    90
16    G   A   H   S   Q   V   Q   L   V   Q   S   G   G   G   M       30

91    GTA GAG CCT GGG GGG TCC CTT AGA CTC TCC TGT GCA GCC TCT GGA    135
31    V   E   P   G   G   S   L   R   L   S   C   A   A   S   G       45

136   TTC ACT TTC AGT AAT GCC TGG ATG AGC TGG GTC CGC CAG GCT CCA    180
46    F   T   F   S   N   A   W   M   S   W   V   R   Q   A   P       60

181   GGG AAG GGG CTG GAG TGG GTT GGC CGT ATA AAA AGC AAA GCT GGT    225
61    G   K   G   L   E   W   V   G   R   I   K   S   K   A   G       75

226   GGT GGG ACA GCA GAG TAC GCT GCA CCC GTG AAA GGC AGA TTC ACC    270
76    G   G   T   A   E   Y   A   A   P   V   K   G   R   F   T       90

271   ATC TCA AGA GAT GAT TCA CAA AAC ACG CTG TAT CTG CAA ATG AAC    315
91    I   S   R   D   D   S   Q   N   T   L   Y   L   Q   M   N       105

316   AGC CTG AAA ACC GAC GAC ACA GCC GTG TAT TAC TGT ACC ACA CAT    360
106   S   L   K   T   D   D   T   A   V   Y   Y   C   T   T   H       120

361   GTC TAC GGT GCC CCC CGG AAC TGG GGC CAG GGA TCC CTG GTC ACC    405
121   V   Y   G   A   P   R   N   W   G   Q   G   S   L   V   T       135

406   GTC TCC TCA GCC TCC ACC AAG GGC CCA AAG CTT GAA GAA GGT GAA    450
136   V   S   S   A   S   T   K   G   P   K   L   E   E   G   E       150

451   TTT TCA GAA GCA CGC GTA CAG TCT GTG TTG ACT CAG CCG CCC TCA    495
151   F   S   E   A   R   V   Q   S   V   L   T   Q   P   P   S       165

496   GTG TCT GCG GCC CCA GGA CAG AAG GTC ACC ATC TCC TGC TCT GGA    540
166   V   S   A   A   P   G   Q   K   V   T   I   S   C   S   G       180

541   AGC AGC TCC AAC ATT GGA AAT AAT TAT GTC TCC TGG TAC GTT CAA    585
181   S   S   S   N   I   G   N   N   Y   V   S   W   Y   V   Q       195

586   CTC CCA GGA ACA GCC CCC AAA CTC CTC ATT TAT GAC AAT AAT AAG    630
196   L   P   G   T   A   P   K   L   L   I   Y   D   N   N   K       210

631   CGA TTC TCA GGA GTT CCT GAC CGA TTC TCT GGC TCC AAG TCT GGC    675
211   R   F   S   G   V   P   D   R   F   S   G   S   K   S   G       225

676   ACG TCA GCC ACC CTG GGC ATC ACC GGG CTC CAG ACT GGG GAC GAG    720
226   T   S   A   T   L   G   I   T   G   L   Q   T   G   D   E       240
```

## Fortsetzung Figur 25

```
721    GCC GAT TAT TAC TGC GGA GCA TGG GAT GGC AGC CTG CGT GAA GCG    765
241     A   D   Y   Y   C   G   A   W   D   G   S   L   R   E   A     255

766    GTA TTC GGC GGA GGG ACC AAG GTC ACC GTC CTA GGT GCG GCC GCA    810
256     V   F   G   G   G   T   K   V   T   V   L   G   A   A   A     270

811    GTT GAG CTC GAG GCG GCC GCG GAT TAC AAA GAC GAT GAC GAT AAA    855
271     V   E   L   E   A   A   A   D   Y   K   D   D   D   D   K     285

856    GAA TTC GGA ACC TCT GAG GAA ACC ATT TCT ACA GTT CAA GAA AAG    900
286     E   F   G   T   S   E   E   T   I   S   T   V   Q   E   K     300

901    CAA CAA AAT ATT TCT CCC CTA GTG AGA GAA AGA GGT CCT CAG AGA    945
301     Q   Q   N   I   S   P   L   V   R   E   R   G   P   Q   R     315

946    GTA GCA GCT CAC ATA ACT GGG ACC AGA GGA AGA AGC AAC ACA TTG    990
316     V   A   A   H   I   T   G   T   R   G   R   S   N   T   L     330

991    TCT TCT CCA AAC TCC AAG AAT GAA AAG GCT CTG GGC CGC AAA ATA    1035
331     S   S   P   N   S   K   N   E   K   A   L   G   R   K   I     345

1036   AAC TCC TGG GAA TCA TCA AGG AGT GGG CAT TCA TTC CTG AGC AAC    1080
346     N   S   W   E   S   S   R   S   G   H   S   F   L   S   N     360

1081   TTG CAC TTG AGG AAT GGT GAA CTG GTC ATC CAT GAA AAA GGG TTT    1125
361     L   H   L   R   N   G   E   L   V   I   H   E   K   G   F     375

1126   TAC TAC ATC TAT TCC CAA ACA TAC TTT CGA TTT CAG GAG GAA ATA    1170
376     Y   Y   I   Y   S   Q   T   Y   F   R   F   Q   E   E   I     390

1171   AAA GAA AAC ACA AAG AAC GAC AAA CAA ATG GTC CAA TAT ATT TAC    1215
391     K   E   N   T   K   N   D   K   Q   M   V   Q   Y   I   Y     405

1216   AAA TAC ACA AGT TAT CCT GAC CCT ATA TTG TTG ATG AAA AGT GCT    1260
406     K   Y   T   S   Y   P   D   P   I   L   L   M   K   S   A     420

1261   AGA AAT AGT TGT TGG TCT AAA GAT GCA GAA TAT GGA CTC TAT TCC    1305
421     R   N   S   C   W   S   K   D   A   E   Y   G   L   Y   S     435

1306   ATC TAT CAA GGG GGA ATA TTT GAG CTT AAG GAA AAT GAC AGA ATT    1350
436     I   Y   Q   G   G   I   F   E   L   K   E   N   D   R   I     450

1351   TTT GTT TCT GTA ACA AAT GAG CAC TTG ATA GAC ATG GAC CAT GAA    1395
451     F   V   S   V   T   N   E   H   L   I   D   M   D   H   E     465

1396   GCC AGT TTT TTC GGG GCC TTT TTA GTT GGC GGT GGC GGT TCT GGT    1440
466     A   S   F   F   G   A   F   L   V   G   G   G   G   S   G     480

1441   GGC GGT TCT GGT GGC GGT TCT GGT GGC GGA TCA ACC TCT GAG GAA    1485
481     G   G   S   G   G   G   S   G   G   G   S   T   S   E   E     495

1486   ACC ATT TCT ACA GTT CAA GAA AAG CAA CAA AAT ATT TCT CCC CTA    1530
496     T   I   S   T   V   Q   E   K   Q   Q   N   I   S   P   L     510

1531   GTG AGA GAA AGA GGT CCT CAG AGA GTA GCA GCT CAC ATA ACT GGG    1575
511     V   R   E   R   G   P   Q   R   V   A   A   H   I   T   G     525
```

Fortsetzung Figur 25

```
1576  ACC AGA GGA AGA AGC AAC ACA TTG TCT TCT CCA AAC TCC AAG AAT  1620
526    T   R   G   R   S   N   T   L   S   S   P   N   S   K   N   540

1621  GAA AAG GCT CTG GGC CGC AAA ATA AAC TCC TGG GAA TCA TCA AGG  1665
541    E   K   A   L   G   R   K   I   N   S   W   E   S   S   R   555

1666  AGT GGG CAT TCA TTC CTG AGC AAC TTG CAC TTG AGG AAT GGT GAA  1710
556    S   G   H   S   F   L   S   N   L   H   L   R   N   G   E   570

1711  CTG GTC ATC CAT GAA AAA GGG TTT TAC TAC ATC TAT TCC CAA ACA  1755
571    L   V   I   H   E   K   G   F   Y   Y   I   Y   S   Q   T   585

1756  TAC TTT CGA TTT CAG GAG GAA ATA AAA GAA AAC ACA AAG AAC GAC  1800
586    Y   F   R   F   Q   E   E   I   K   E   N   T   K   N   D   600

1801  AAA CAA ATG GTC CAA TAT ATT TAC AAA TAC ACA AGT TAT CCT GAC  1845
601    K   Q   M   V   Q   Y   I   Y   K   Y   T   S   Y   P   D   615

1846  CCT ATA TTG TTG ATG AAA AGT GCT AGA AAT AGT TGT TGG TCT AAA  1890
616    P   I   L   L   M   K   S   A   R   N   S   C   W   S   K   630

1891  GAT GCA GAA TAT GGA CTC TAT TCC ATC TAT CAA GGG GGA ATA TTT  1935
631    D   A   E   Y   G   L   Y   S   I   Y   Q   G   G   I   F   645

1936  GAG CTT AAG GAA AAT GAC AGA ATT TTT GTT TCT GTA ACA AAT GAG  1980
646    E   L   K   E   N   D   R   I   F   V   S   V   T   N   E   660

1981  CAC TTG ATA GAC ATG GAC CAT GAA GCC AGT TTT TTC GGG GCC TTT  2025
661    H   L   I   D   M   D   H   E   A   S   F   F   G   A   F   675

2026  TTA GTT GGC GGT GGC GGT TCT GGT GGC GGT TCT GGT GGC GGT TCT  2070
676    L   V   G   G   G   G   S   G   G   G   S   G   G   G   S   690

2071  GGT GGC GGA TCC ACC TCT GAG GAA ACC ATT TCT ACA GTT CAA GAA  2115
691    G   G   G   S   T   S   E   E   T   I   S   T   V   Q   E   705

2116  AAG CAA CAA AAT ATT TCT CCC CTA GTG AGA GAA AGA GGT CCT CAG  2160
706    K   Q   Q   N   I   S   P   L   V   R   E   R   G   P   Q   720

2161  AGA GTA GCA GCT CAC ATA ACT GGG ACC AGA GGA AGA AGC AAC ACA  2205
721    R   V   A   A   H   I   T   G   T   R   G   R   S   N   T   735

2206  TTG TCT TCT CCA AAC TCC AAG AAT GAA AAG GCT CTG GGC CGC AAA  2250
736    L   S   S   P   N   S   K   N   E   K   A   L   G   R   K   750

2251  ATA AAC TCC TGG GAA TCA TCA AGG AGT GGG CAT TCA TTC CTG AGC  2295
751    I   N   S   W   E   S   S   R   S   G   H   S   F   L   S   765

2296  AAC TTG CAC TTG AGG AAT GGT GAA CTG GTC ATC CAT GAA AAA GGG  2340
766    N   L   H   L   R   N   G   E   L   V   I   H   E   K   G   780

2341  TTT TAC TAC ATC TAT TCC CAA ACA TAC TTT CGA TTT CAG GAG GAA  2385
781    F   Y   Y   I   Y   S   Q   T   Y   F   R   F   Q   E   E   795

2386  ATA AAA GAA AAC ACA AAG AAC GAC AAA CAA ATG GTC CAA TAT ATT  2430
796    I   K   E   N   T   K   N   D   K   Q   M   V   Q   Y   I   810
```

Fortsetzung Figur 25

```
2431  TAC AAA TAC ACA AGT TAT CCT GAC CCT ATA TTG TTG ATG AAA AGT    2475
811    Y   K   Y   T   S   Y   P   D   P   I   L   L   M   K   S     825

2476  GCT AGA AAT AGT TGT TGG TCT AAA GAT GCA GAA TAT GGA CTC TAT    2520
826    A   R   N   S   C   W   S   K   D   A   E   Y   G   L   Y     840

2521  TCC ATC TAT CAA GGG GGA ATA TTT GAG CTT AAG GAA AAT GAC AGA    2565
841    S   I   Y   Q   G   G   I   F   E   L   K   E   N   D   R     855

2566  ATT TTT GTT TCT GTA ACA AAT GAG CAC TTG ATA GAC ATG GAC CAT    2610
856    I   F   V   S   V   T   N   E   H   L   I   D   M   D   H     870

2611  GAA GCC AGT TTT TTC GGG GCC TTT TTA GTT GGC TGA                2646
871    E   A   S   F   F   G   A   F   L   V   G   *                 881
```

# Figur 26

Nukleinsäuresequenz und korrespondierende Aminosäuresequenz von scTNF-AMAIZe

Konstrukt H

```
1     ATG GAC TGG ACC TGG CGC GTG TTT TGC CTG CTC GCC GTG GCT CCT   45
1      M   D   W   T   W   R   V   F   C   L   L   A   V   A   P    15

46    GGG GCC CAC AGC CAG GTA CAG CTG GTG CAG TCT GGG GGA GGC ATG   90
16     G   A   H   S   Q   V   Q   L   V   Q   S   G   G   G   M    30

91    GTA GAG CCT GGG GGG TCC CTT AGA CTC TCC TGT GCA GCC TCT GGA   135
31     V   E   P   G   G   S   L   R   L   S   C   A   A   S   G    45

136   TTC ACT TTC AGT AAT GCC TGG ATG AGC TGG GTC CGC CAG GCT CCA   180
46     F   T   F   S   N   A   W   M   S   W   V   R   Q   A   P    60

181   GGG AAG GGG CTG GAG TGG GTT GGC CGT ATA AAA AGC AAA GCT GGT   225
61     G   K   G   L   E   W   V   G   R   I   K   S   K   A   G    75

226   GGT GGG ACA GCA GAG TAC GCT GCA CCC GTG AAA GGC AGA TTC ACC   270
76     G   G   T   A   E   Y   A   A   P   V   K   G   R   F   T    90

271   ATC TCA AGA GAT GAT TCA CAA AAC ACG CTG TAT CTG CAA ATG AAC   315
91     I   S   R   D   D   S   Q   N   T   L   Y   L   Q   M   N    105

316   AGC CTG AAA ACC GAC GAC ACA GCC GTG TAT TAC TGT ACC ACA CAT   360
106    S   L   K   T   D   D   T   A   V   Y   Y   C   T   T   H    120

361   GTC TAC GGT GCC CCC CGG AAC TGG GGC CAG GGA TCC CTG GTC ACC   405
121    V   Y   G   A   P   R   N   W   G   Q   G   S   L   V   T    135

406   GTC TCC TCA GCC TCC ACC AAG GGC CCA AAG CTT GAA GAA GGT GAA   450
136    V   S   S   A   S   T   K   G   P   K   L   E   E   G   E    150

451   TTT TCA GAA GCA CGC GTA CAG TCT GTG TTG ACT CAG CCG CCC TCA   495
151    F   S   E   A   R   V   Q   S   V   L   T   Q   P   P   S    165

496   GTG TCT GCG GCC CCA GGA CAG AAG GTC ACC ATC TCC TGC TCT GGA   540
166    V   S   A   A   P   G   Q   K   V   T   I   S   C   S   G    180

541   AGC AGC TCC AAC ATT GGA AAT AAT TAT GTC TCC TGG TAC GTT CAA   585
181    S   S   S   N   I   G   N   N   Y   V   S   W   Y   V   Q    195

586   CTC CCA GGA ACA GCC CCC AAA CTC CTC ATT TAT GAC AAT AAT AAG   630
196    L   P   G   T   A   P   K   L   L   I   Y   D   N   N   K    210

631   CGA TTC TCA GGA GTT CCT GAC CGA TTC TCT GGC TCC AAG TCT GGC   675
211    R   F   S   G   V   P   D   R   F   S   G   S   K   S   G    225

676   ACG TCA GCC ACC CTG GGC ATC ACC GGG CTC CAG ACT GGG GAC GAG   720
226    T   S   A   T   L   G . I   T   G   L   Q   T   G   D   E    240

721   GCC GAT TAT TAC TGC GGA GCA TGG GAT GGC AGC CTG CGT GAA GCG   765
241    A   D   Y   Y   C   G   A   W   D   G   S   L   R   E   A    255
```

## Fortsetzung Figur 26

```
766    GTA TTC GGC GGA GGG ACC AAG GTC ACC GTC CTA GGT GCG GCC GCA    810
256     V   F   G   G   G   T   K   V   T   V   L   G   A   A   A     270

811    GTT GAG CTC GAG GCG GCC GCG GAT TAC AAA GAC GAT GAC GAT AAA    855
271     V   E   L   E   A   A   A   D   Y   K   D   D   D   D   K     285

856    GAA TTC GGA TCA TCT TCT CGA ACC CCG AGT GAC AAG CCT GTA GCC    900
286     E   F   G   S   S   S   R   T   P   S   D   K   P   V   A     300

901    CAT GTT GTA GCA AAC CCT CAA GCT GAG GGG CAG CTC CAG TGG CTG    945
301     H   V   V   A   N   P   Q   A   E   G   Q   L   Q   W   L     315

946    AAC CGC CGG GCC AAT GCC CTC CTG GCC AAT GGC GTG GAG CTG AGA    990
316     N   R   R   A   N   A   L   L   A   N   G   V   E   L   R     330

991    GAT AAC CAG CTG GTG GTG CCA TCA GAG GGC CTG TAC CTC ATC TAC    1035
331     D   N   Q   L   V   V   P   S   E   G   L   Y   L   I   Y     345

1036   TCC CAG GTC CTC TTC AAG GGC CAA GGC TGC CCC TCC ACC CAT GTG    1080
346     S   Q   V   L   F   K   G   Q   G   C   P   S   T   H   V     360

1081   CTC CTC ACC CAC ACC ATC AGC CGC ATC GCC GTC TCC TAC CAG ACC    1125
361     L   L   T   H   T   I   S   R   I   A   V   S   Y   Q   T     375

1126   AAG GTC AAC CTC CTC TCT GCC ATC AAG AGC CCC TGC CAG AGG GAG    1170
376     K   V   N   L   L   S   A   I   K   S   P   C   Q   R   E     390

1171   ACC CCA GAG GGG GCT GAG GCC AAG CCC TGG TAT GAG CCC ATC TAT    1215
391     T   P   E   G   A   E   A   K   P   W   Y   E   P   I   Y     405

1216   CTG GGA GGG GTC TTC CAG CTG GAG AAG GGT GAC CGA CTC AGC GCT    1260
406     L   G   G   V   F   Q   L   E   K   G   D   R   L   S   A     420

1261   GAG ATC AAT CGG CCC GAC TAT CTC GAC TTT GCC GAG TCT GGG CAG    1305
421     E   I   N   R   P   D   Y   L   D   F   A   E   S   G   Q     435

1306   GTC TAC TTT GGG ATC ATT GCC CTG GGT GGC GGT TCT GGT GGC GGT    1350
436     V   Y   F   G   I   I   A   L   G   G   G   S   G   G   G     450

1351   TCT GGT GGC GGT TCT GGT GGC GGA TCA TCA TCT TCT CGA ACC CCG    1395
451     S   G   G   G   S   G   G   G   S   S   S   S   R   T   P     465

1396   AGT GAC AAG CCT GTA GCC CAT GTT GTA GCA AAC CCT CAA GCT GAG    1440
466     S   D   K   P   V   A   H   V   V   A   N   P   Q   A   E     480

1441   GGG CAG CTC CAG TGG CTG AAC CGC CGG GCC AAT GCC CTC CTG GCC    1485
481     G   Q   L   Q   W   L   N   R   R   A   N   A   L   L   A     495

1486   AAT GGC GTG GAG CTG AGA GAT AAC CAG CTG GTG GTG CCA TCA GAG    1530
496     N   G   V   E   L   R   D   N   Q   L   V   V   P   S   E     510

1531   GGC CTG TAC CTC ATC TAC TCC CAG GTC CTC TTC AAG GGC CAA GGC    1575
511     G   L   Y   L   I   Y   S   Q   V   L   F   K   G   Q   G     525

1576   TGC CCC TCC ACC CAT GTG CTC CTC ACC CAC ACC ATC AGC CGC ATC    1620
526     C   P   S   T   H   V   L   L   T   H   T   I   S   R   I     540
```

## Fortsetzung Figur 26

```
1621  GCC GTC TCC TAC CAG ACC AAG GTC AAC CTC CTC TCT GCC ATC AAG  1665
541    A   V   S   Y   Q   T   K   V   N   L   L   S   A   I   K   555

1666  AGC CCC TGC CAG AGG GAG ACC CCA GAG GGG GCT GAG GCC AAG CCC  1710
556    S   P   C   Q   R   E   T   P   E   G   A   E   A   K   P   570

1711  TGG TAT GAG CCC ATC TAT CTG GGA GGG GTC TTC CAG CTG GAG AAG  1755
571    W   Y   E   P   I   Y   L   G   G   V   F   Q   L   E   K   585

1756  GGT GAC CGA CTC AGC GCT GAG ATC AAT CGG CCC GAC TAT CTC GAC  1800
586    G   D   R   L   S   A   E   I   N   R   P   D   Y   L   D   600

1801  TTT GCC GAG TCT GGG CAG GTC TAC TTT GGG ATC ATT GCC CTG GGT  1845
601    F   A   E   S   G   Q   V   Y   F   G   I   I   A   L   G   615

1846  GGC GGT TCT GGT GGC GGT TCT GGT GGC GGT TCT GGT GGC GGA TCA  1890
616    G   G   S   G   G   G   S   G   G   G   S   G   G   G   S   630

1891  TCA TCT TCT CGA ACC CCG AGT GAC AAG CCT GTA GCC CAT GTT GTA  1935
631    S   S   S   R   T   P   S   D   K   P   V   A   H   V   V   645

1936  GCA AAC CCT CAA GCT GAG GGG CAG CTC CAG TGG CTG AAC CGC CGG  1980
646    A   N   P   Q   A   E   G   Q   L   Q   W   L   N   R   R   660

1981  GCC AAT GCC CTC CTG GCC AAT GGC GTG GAG CTG AGA GAT AAC CAG  2025
661    A   N   A   L   L   A   N   G   V   E   L   R   D   N   Q   675

2026  CTG GTG GTG CCA TCA GAG GGC CTG TAC CTC ATC TAC TCC CAG GTC  2070
676    L   V   V   P   S   E   G   L   Y   L   I   Y   S   Q   V   690

2071  CTC TTC AAG GGC CAA GGC TGC CCC TCC ACC CAT GTG CTC CTC ACC  2115
691    L   F   K   G   Q   G   C   P   S   T   H   V   L   L   T   705

2116  CAC ACC ATC AGC CGC ATC GCC GTC TCC TAC CAG ACC AAG GTC AAC  2160
706    H   T   I   S   R   I   A   V   S   Y   Q   T   K   V   N   720

2161  CTC CTC TCT GCC ATC AAG AGC CCC TGC CAG AGG GAG ACC CCA GAG  2205
721    L   L   S   A   I   K   S   P   C   Q   R   E   T   P   E   735

2206  GGG GCT GAG GCC AAG CCC TGG TAT GAG CCC ATC TAT CTG GGA GGG  2250
736    G   A   E   A   K   P   W   Y   E   P   I   Y   L   G   G   750

2251  GTC TTC CAG CTG GAG AAG GGT GAC CGA CTC AGC GCT GAG ATC AAT  2295
751    V   F   Q   L   E   K   G   D   R   L   S   A   E   I   N   765

2296  CGG CCC GAC TAT CTC GAC TTT GCC GAG TCT GGG CAG GTC TAC TTT  2340
766    R   P   D   Y   L   D   F   A   E   S   G   Q   V   Y   F   780

2341  GGG ATC ATT GCC CTG TGA                                      2358
781    G   I   I   A   L   *                                        785
```

TNF-Pharmakokinetik

Figur 27

Figur 28

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 0222680 A
- DE 10247755
- WO 0137873 A
- WO 0143770 A
- WO 9961085 A
- US 5877 A
- US 4737462 A
- US 4588585 A
- US 4959314 A
- US 5116943 A
- US 4879111 A
- US 5017691 A
- US 4619639 A
- DE 10144252 A1
- DE 10144252
- DE 10045591

### In der Beschreibung aufgeführte Nicht-Patentliteratur

- **AG-GARWAL, B.B.** *Nat. Rev. Immunol.,* 2003, vol. 3, 745-756
- **BANNER, D.W. et al.** *Cell,* 1993, vol. 73, 431-445
- **BECK et al.** *J Mol Biol,* 1996, vol. 256, 909-923
- **KISHORE ; REID.** *Immunopharmacol.,* 1999, vol. 42, 15-21
- **EPSTEIN et al.** *Current Opinion in Immunology,* 1996, vol. 8 (1), 29-35
- **WAJANT, H. et al.** *Cell Death, Differ,* 2003, vol. 10, 45-65
- **EGGERMONT, A.M. ; TEN HAGEN, T.L.** *Curr. Oncol. Rep.,* 2003, vol. 5, 79-80
- **WELEY et al.** *Immunity,* 1995, vol. 6, 673-682
- **PETTI et al.** *J Biol Chem,* 1996, vol. 271, 12687-12689
- **JO et al.** *Nat Med,* 2000, vol. 6, 564-567
- **ICHIKAWA et al.** *Nat Med,* 2001, vol. 7, 954-960
- **OGASAWARA et al.** *Nature,* 1993, vol. 364, 806-809
- **EGGERMONT, A.M. ; TEN HAGEN, T.L.** *Curr. OncoL Rep.,* 2003, vol. 5, 79-80
- **SMITH, R.A. ; BAGLIONI, C.** *J. Biol. Chem.,* 1987, vol. 262, 6951-6954
- **NARHI, LO. ; ARAKAWA, T.** *Biochem. Biophys. Res. Commun,* 1987, vol. 147, 740-746
- **CHA, S.S. et al.** *Immunity,* 1999, vol. 11, 253-261
- **HYMOWITZ, S.G. et al.** *Biochemistry,* 2000, vol. 39, 633-640
- **MANIATIS et al.** Molecular Cloning: A Laboratory Manual. Cold Spring Harbour Laboratory Press, 2001
- **MANIATIS et al.** Molecular cloning: Laboratory Manual. Cold Spring Harbour Laboratory Press, 2001
- **KARLIN et al.** *PNAS USA,* 1993, vol. 90, 5873
- **ALTSCHUL et al.** *Nucleic Acids Res,* 1997, vol. 25, 3389-3402
- Absorption, circular Dichroism and ORD of Polypeptides. **URRY et al.** Modem Physical Methods in Biochemistry. Elsevier, 1985
- **MANIATIS et al.** Molecular Cloning: A Laboratory Manual. Cold Spring Harbor Laboratory Press, 2001
- **COMACK B. ; AUSUBEL F. et al.** Current Protocols in Molecular Biology. 1991, 8.01-8.5.9
- **BROCKS et al.** *Immuaotechnology,* 1997, vol. 3, 173-184
- **LENTZ M R.** *Therapeutics Apheresis,* 1999, vol. 3 (1
- **HAFNER M. ; P. OROSZ ; A. KRÜGER ; D.N. MÄNNEL.** TNF promotes metastasis by impairing natural killer cell activity. *Internat. J. Cancer,* 1996, vol. 66, 388-392
- **LUCAS R. et al.** *Int J Cancer,* 2001, vol. 91, 543-549
- **EGGERMONT et al.** *Lancet Oncol.,* 2003, vol. 4, 429